# EUROPEAN PATENT SPECIFICATION

(11) **EP 3 215 513 B1**
(45) Date of publication and mention of the grant of the patent: **08.05.2019**
(21) Application number: 15788447.9
(22) Date of filing: 04.11.2015
(51) Int. Cl.: C07D 498/14, A61K 31/5383, A61P 35/00

(54) **FUNCTIONALIZED MORPHOLINYL ANTHRACYCLINE DERIVATIVES**
FUNKTIONALISIERTE MORPHOLINYL-ANTHRACYCLIN-DERIVATE
DÉRIVÉS DE MORPHOLINYLANTHRACYCLINE FONCTIONNALISÉS

(30) Priority: 05.11.2014 EP 14191952
(43) Date of publication of application: 13.09.2017
(73) Proprietor: Nerviano Medical Sciences S.r.l., 20014 Nerviano (MI) (IT)
(72) Inventor: BERIA, Italo, I-20014 Nerviano (MI) (IT); CARUSO, Michele, I-20131 Milan (IT); LUPI, Vittoria, I-20094 (IT)
(86) International application number: PCT/EP2015/075749
(87) International publication number: WO 2016/071418

(56) References cited:
- WO-A1-2009/099741
- WO-A1-2014/177441
- WO-A2-2010/009124

## Description

The present invention relates to new functionalized morpholinyl anthracycline derivatives, to a process for their preparation, to pharmaceutical compositions containing them and use thereof in the treatment of abnormal cell proliferation diseases. As an example, the compounds of the invention may be used to treat tumors. The invention also relates to their use in the preparation of conjugates.

Anthracyclines are antibiotic compounds that exhibit cytotoxic activity. Several studies have indicated that anthracyclines may operate to kill cells by a number of different mechanisms including: 1) intercalation with the DNA of a cell thereby inhibiting DNA-dependent nucleic acid synthesis; 2) production of free radicals which then react with cellular macromolecules to cause damage to the cells or 3) interactions with the cell membrane [see, e.g., C. Peterson et al., "Transport and storage of Anthracycline in experimental systems and human leukemia" in Anthracycline Antibiotics In Cancer Therapy (1982), pp.132-146; and N.R. Bachur, "Free Radical Damage" id. pp.97-102]. Because of their cytotoxic activity, anthracyclines have been used in the treatment of numerous cancers such as leukemia, breast carcinoma, lung carcinoma, ovarian adenocarcinoma and sarcomas [see e.g., P.H-Wiernik, in Anthracycline: Current Status And New Developments (1980), p. 11]. Commonly used anthracyclines include doxorubicin, epirubicin, idarubicin and daunomycin.

In the recent years many new highly cytotoxic anthracycline derivatives have been synthesized.

Anthracycline derivatives bearing a substituted morpholino ring linked at the C-3' position of the sugar moiety have shown promising antitumor activity on experimental murine tumors [see: J. W. Lown, Bioactive Molecules, vol 6, (1988), pp.55-101] and in clinical trials in the treatment of hepatocellular carcinoma [see: C. Sessa, O. Valota, C. Geroni, Cardiovascular Toxicology, vol. 7(2), (2007), pp. 75-79].

New morpholinyl anthracycline derivatives in which the morpholino ring is bridged with an oxygen atom to position C-4' of the sugar residue have been disclosed as antitumor agents in the International Pat. App. WO9802446 in the name of Pharmacia & Upjohn SPA.

4-Amino and 4-fluoro anthracycline derivatives have been also disclosed as antitumor agents in Pat. App. EP 288268 and EP 381989 in the name of Farmitalia Carlo Erba Srl.

Despite the efforts in anticancer research, cancer remains a looming and elusive target, therefore there is still a need for new anticancer agents.

Although these compounds may be useful in the treatment of neoplasms and other disease states wherein a selected cell population is sought to be eliminated, their therapeutic efficacy is often limited by the dose-dependent toxicity associated with their administration.

Limitations in the use of anthracycline derivatives in clinical include inadequate accumulation in the tumor, cardiotoxicity, prolonged time of infusions, resistance of cancer cells overexpressing membrane transporters and dose-limiting side effects such as diarrhea and bone marrow suppression (Int. J. Nanomedicine, 2007; 2(4): 567-83; Med. Sci. Monit. 2000; 6(2); Adriamycin Review, pp 123-131, Mediokon, Belgium: European Press, 1975; Ann Intern Med. 1982; 96(2): 133-139).

Drug conjugation of cytotoxic drugs to molecules able to vehicle the drug thus improving tumor targeting or able to modify its pharmacokinetic properties is one of the strategies that has been undertaken to solve the above mentioned issues.

Anthracycline derivative conjugates have been disclosed, for instance, in Int. Pat. App. WO2009/099741 and in WO2010/009124.

Different examples of conjugation of cytotoxic drugs with proteins, peptides, aptamers, polymers or nanoparticles allowing better target delivery, improving solubility and in some cases other pharmacokinetic properties, such as increasing half life or local concentration of the drug, and improving drug performances have been reported. As a matter of facts, the resultant conjugates have improved characteristics in terms of solubility, permeability into the cell, *in vivo* therapeutic window, controlled release, ability to reach the target according to the nature of the specific molecule conjugated with the cytotoxic agent, etc.

For this reason, there is an increasing demand for the development of functionalized cytotoxic agents suitable to be conjugated with different types of molecules.

The first object of the present invention is to provide functionalized anthracycline derivatives which, further to show cytotoxic activity, have high solubility so that to improve formulation and/or pharmacokinetic/pharmacodynamic properties. Furthermore these functionalized anthracycline derivatives are also suitable to be conjugated.

The present invention relates to morpholinyl anthracycline derivatives of formula (I)

Ant-L-W-Z-RM (I)

wherein
**RM** is a radical selected among the groups: 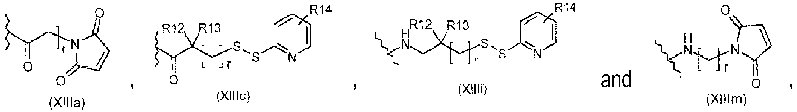 wherein
   **r** is an integer from 0 to 7;
   **R12** and **R13** are, each independently, hydrogen or methyl, and
   **R14** is hydrogen;
   the wavy line indicates the attachment point in the derivative of formula (I);
**Z** is null or a peptidic, non peptidic or hybrid - peptidic and non peptidic - linker selected among: 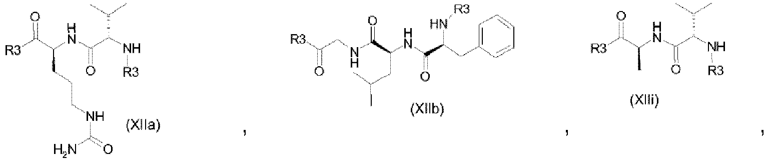 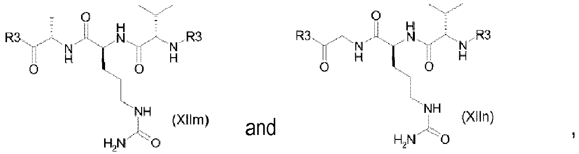 wherein
   one of the two **R3** substituents is a tether to **Ant,** to **W** or to **L,** and the other is null;
**W** is null or a group selected among: 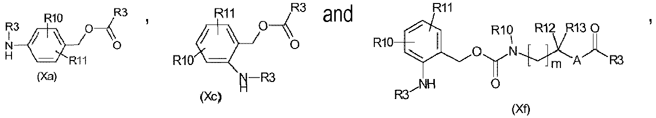 wherein
   one of the two **R3** substituents is a tether to **L,** or to **Ant** when **L** is null, and the other is null;
   **R10** and **R11** are hydrogen;
   **m** is an integer from 0 to 3
   **A** is N-CH₃; and
   **R12** and **R13** are hydrogen;
**L** is null or a moiety selected among: 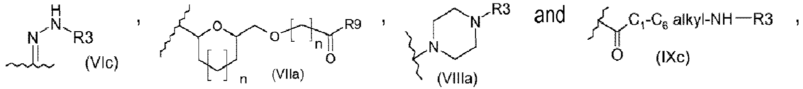 wherein
   C₁-C₆ alkyl is a straight or branched chain;
   **R3** is null or hydrogen, and the wavy line indicates the attachment point to **Ant;**
   **R9** is null;
   **n** is an integer from 0 to 2;
**Ant** is an anthracycline moiety selected from the formulas (IIa), (IIIa), (IVa) and (Va): 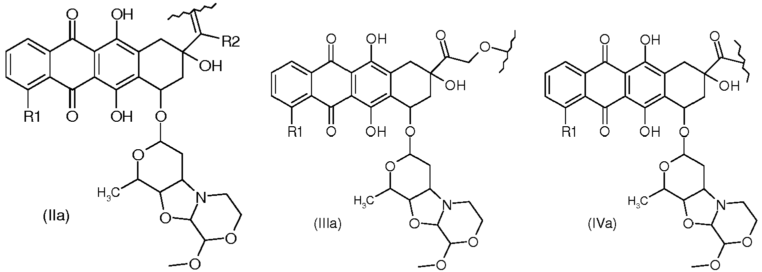 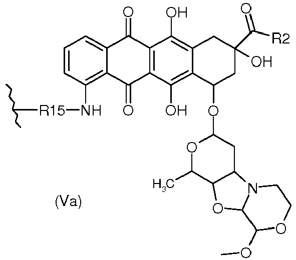 wherein the wavy line indicates the attachment
   to the moiety **L**, or
   to the group **W**, or
   to the linker **Z**, or
   to the radical **RM;**
   **R1** is NH₂;
   **R2** is methyl or hydroxymethyl;
   **R15** is null;
   or a pharmaceutically acceptable salt thereof.

The present invention also provides methods of synthesizing the morpholinyl anthracycline derivatives of formula (I), prepared through a process consisting of standard synthetic transformations, and their isomers, tautomers, hydrates, solvates, complexes, metabolites, prodrugs, carriers, N-oxides.

Moreover, the present invention provides a pharmaceutical composition comprising a therapeutically effective amount of a derivative of formula (I) or a pharmaceutically acceptable salt thereof as defined above and at least one pharmaceutically acceptable excipient, carrier or diluent.

The present invention further provides a pharmaceutical composition comprising a therapeutically effective amount of a derivative of formula (I) and one or more chemotherapeutic agents.

The present invention also provides a pharmaceutical composition comprising a therapeutically effective amount of a derivative of formula (I) in combination with known anticancer treatments such as radiation therapy or chemotherapy regimen, in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER2 agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

Additionally, the invention provides a product comprising a derivative of formula (I) or a pharmaceutically acceptable salt thereof, as defined above, and one or more chemotherapeutic agents, as a combined preparation for simultaneous, separate or sequential use in anticancer therapy.

In yet another aspect the invention provides a derivative of formula (I) or a pharmaceutically acceptable salt thereof, as defined above, for use as a medicament.

The present invention also provides a derivative of formula (I) as defined above, for use in a method of treating cancer, cellular proliferation disorders and viral infections.

Preferably, a derivative of formula (I) as defined above, is for use in a method of treating specific types of cancers, such as but not limited to: carcinomas, including bladder, breast, colon, kidney, liver, lung, comprising small cell lung cancer, esophagus, gall-bladder, ovary, pancreas, stomach, cervix, thyroid, prostate, and skin carcinoma, comprising squamous cell carcinoma; hematopoietic tumors of lymphoid lineage, including leukemia, acute lymphocitic leukemia, acute lymphoblastic leukemia, B-cell lymphoma, T-cell lymphoma, Hodgkin's lymphoma, non-Hodgkin's lymphoma, hairy cell lymphoma and Burkitt's lymphoma; hematopoietic tumors of myeloid lineage, including acute and chronic myelogenous leukemia, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannoma; and other tumors, including melanoma, seminoma, teratocarcinoma, osteosarcoma, xeroderma pigmentosum, keratoxanthoma, thyroid follicular cancer, Kaposi's sarcoma and mesothelioma.

Furthermore, a derivative of formula (I) as defined above is for use in a method of treating specific cellular proliferation disorders such as, for example, benign prostate hyperplasia, familial adenomatosis polyposis (FAP), neurofibromatosis, psoriasis, vascular smooth cell proliferation associated with atherosclerosis, pulmonary fibrosis, arthritis, glomerulonephritis and post-surgical stenosis and restenosis.

In addition, a derivative of formula (I) as defined above is for use in a method of inhibiting tumor angiogenesis and metastasis, as well as in a method of treating organ transplant rejection and host versus graft disease.

The present invention also provides a method for treating cancer, which comprises administering to a mammal in need thereof an effective amount of a derivative of formula (I) as defined above. The mammal in need thereof may be for example a human.

Moreover the invention provides the use of a derivative of formula (I) or a pharmaceutically acceptable salt thereof, as defined above, in the manufacture of a medicament for treating cancer.

Finally, the invention provides the use of a derivative of formula (I) or a pharmaceutically acceptable salt thereof, as defined above, in the preparation of conjugates.

Unless stated otherwise, the following terms and phrases as used herein are intended to have the following meanings.

With the term "straight or branched C₁-C₆ alkyl" we intend any of the groups such as, for instance, methyl, ethyl, n-propyl, n-butyl, n-pentyl, isopropyl, n-butyl, isobutyl, sec-butyl, tert-butyl.

With the term "halogen" we intend a fluorine, chlorine, bromine or iodine.

The term "aryl" as used herein refers to carbocyclic hydrocarbons with from 1 to 2 ring moieties, either fused or linked to each other by single bonds, wherein at least one of the rings is aromatic. Examples of aryl groups according to the invention are, for instance, phenyl, biphenyl, α- or β-naphthyl, dihydronaphthyl, and the like.

The term "leaving group" refers to a group that can be substituted by another group in a substitution reaction. Such leaving groups are well-known in the art and examples include, but are not limited to, halides (fluoride, chloride, bromide and iodide), azides, sulfonates (e.g., an optionally substituted C₁-C₆ alkanesulfonate, such as methanesulfonate and trifluoromethanesulfonate, or an optionally substituted C₇-C₁₂ alkylbenzenesulfonate, such as p-toluenesulfonate), succinimide-N-oxide, *p*-nitrophenoxide, pentafluorophenoxide, tetrafluorophenoxide, carboxylates, aminocarboxylates (carbamates) and alkoxycarboxylates (carbonates). For substitutions at saturated carbon, halides and sulfonates are preferred leaving groups. For substitutions at a carbonyl carbon a halide, succinimide-N-oxide, *p*-nitrophenoxide, pentafluorophenoxide, tetrafluorophenoxide, a carboxylate, or an alkoxycarboxylate (carbonate) may for example be used as a leaving group. The term "leaving group" also refers to a group that is eliminated as a consequence of an elimination reaction, e.g., an electronic cascade reaction or a spirocyclization reaction. In this instance, an halide, a sulfonate, an azide, an aminocarboxylate (carbamate) or an alkoxycarboxylate (carbonate) may for example be used as a leaving group.

It is known to the person skilled in the art that transformation of a chemical functional group into another may require that one or more reactive centers in the compound containing such functional group have to be protected in order to avoid undesired side reactions. Protection of such reactive centers, and subsequent deprotection at the end of the synthetic transformations, can be accomplished following standard procedures described in the literature (see, for instance, Green, Theodora W. and Wuts, Peter G.M. - Protective Groups in Organic Synthesis, Third Edition, John Wiley & Sons Inc., New York (NY), 1999).

Therefore, the term "protecting group" refers to a group used to protect such reactive centers in a chemical synthesis, for example, a hydroxyl group (-OH), an amino group (-NH), a thiol group (-SH), a carbonyl group (-C=O), a carboxylic group (-COOH). Examples of protecting groups are those reported in the literature (see, for instance, ibidem).

The term "nitrogen protecting group" refers to a group that with the nitrogen atom form carbamates, amides, cyclic imides, *N*-alkyl and *N*-aryl amines. Such protecting groups are well-known in the art (see e.g. ibidem). Non limiting examples of carbamate protecting groups are, for instance, methyl and ethyl carbamate, 9-fluorenylmethyl carbamate (Fmoc), 2,2,2-trichloroethylcarbamate (Troc), *t*-butyl carbamate (BOC), vinyl carbamate (Voc), allyl carbamate (Alloc), benzyl carbamate (Cbz), *p*-nitrobenzyl and the like. Non limiting examples of amides are, for instance *N*-trichloroacetamide, *N*-trifluoroacetamide (TFA) and the like. Non limiting examples of cyclic imide protecting groups are, for instance, *N*-phthalimide, *N*-dithiasuccinoylimide (Dts) and the like. Non limiting examples of *N-*alkyl and *N*-aryl protecting groups are, for instance, *N*-allylamine, N-benzylamine and the like.

The term "hydroxyl protecting group" refers to a group that with the oxygen atom form ethers, esters, cyclic acetals or ketals. Such protecting groups are well-known in the art (see e.g. ibidem). Non limiting examples of ethers protecting groups are, for instance, alkyl ethers and benzyl ethers, such as methoxymethyl ether (MOM-OR), tetrahydropyranyl ether (THP-OR), allyl ether (Allyl-OR), benzyl ether (Bn-OR), triphenylmethyl ether (Tr-OR) and the like, or silyl ethers, such as trimethylsilyl ether (TMS-OR), *t*-butyldimethylsilyl ether (TBS-OR or TBDMS-OR), *t*-butyldiphenylsilyl ether (TBDPS-OR) diphenylmethylsilyl ether (DPMS-OR) and the like. Non limiting examples of esters protecting groups are, for instance, trifluoroacetate, benzoate (Bz-OR) and carbonates, such as ethylcarbonate and the like. Non limiting examples of cyclic acetals or ketals protecting groups are, for instance, methylene acetal, ethylidene acetal, methoxymethylene acetal and the like.

The term "active ester" refers to a functional group in which the alkoxy group of the ester moiety is a good leaving group. Examples of such alkoxy groups include, but are not limited to, succinimide-*N*-oxide (NHS esters), *p-*nitrophenoxide, pentafluorophenoxide, tetrafluorophenoxide, 1-hydroxybenzotriazole and 1-hydroxy-7-azabenzotriazole, and groups with comparable leaving capability. Unsubstituted alkyl-based alkoxy groups such as methoxy, ethoxy, isopropoxy, and t-butoxy do not qualify as good leaving groups and methyl, ethyl, isopropyl, and t-butyl esters are therefore not considered to be active esters.

The term "electron withdrawing group" refers to an atom group that draws electron density from neighboring atoms towards itself, usually by resonance or inductive effects. Non limiting examples are halogens, trifluoromethyl group, nitro (NO₂) group and nitrile (CN).

The term "nucleophiles" refers to molecules that bear a nucleophilic group. The term "nucleophilic group" refers to a species that donates an electron-pair to an electrophilic group to form a chemical bond in a chemical reaction. Examples of such nucleophilic groups include, but are not limited to halogens, amines, nitrites, azides, hydroxyls, alkoxyde anions, carboxylate anions, thiols, thiolates, etc.

The term "electrophilic group" refers to a species that accepts an electron-pair from a nucleophilic group to form a chemical bond in a chemical reaction. Examples of such electrophilic groups include, but are not limited to esters, aldehydes, amides, ketons, etc.

The term "unnatural aminoacid" refers to the D-stereoisomer of the naturally occurring aminoacid (L-stereoisomer). The anthracycline moieties (**Ant**) of the invention are compounds of formula (IIa), (IIIa), (IVa) and (Va) depicted below: 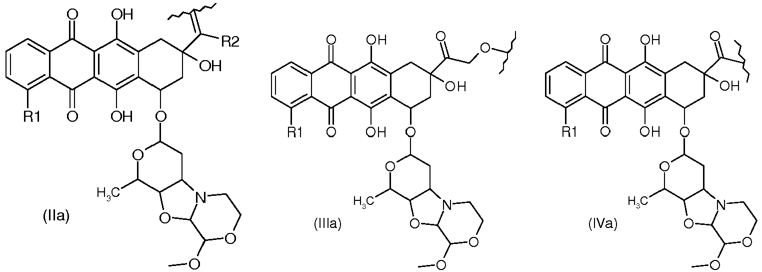 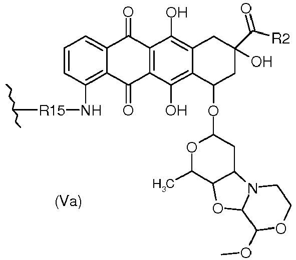 wherein **R1, R2** and **R15** are as defined above.

Pharmaceutically acceptable salts of the compounds of formula (I) also include the salts with inorganic or organic bases, e.g., alkali or alkaline-earth metals, especially sodium, potassium, calcium, ammonium or magnesium hydroxides, carbonates or bicarbonates, acyclic or cyclic amines.

Pharmaceutically acceptable salts of the compounds of formula (I) include the salts with inorganic or organic acids, e.g., nitric, hydrochloric, hydrobromic, sulfuric, perchloric, phosphoric, acetic, trifluoroacetic, propionic, glycolic, fumaric, lactic, oxalic, malonic, malic, maleic, tartaric, citric, benzoic, cinnamic, mandelic, methanesulfonic, isethionic and salicylic acid.

If a stereogenic center or another form of an isomeric center is present in a compound of the present invention, all forms of such isomer or isomers, including enantiomers and diastereomers, are intended to be covered herein. Compounds containing a stereogenic center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention.

In cases when compounds can exist in tautomeric forms, each form is contemplated as being included within this invention whether existing in equilibrium or predominantly in one form.

### The moiety L

The **L** moiety, if present, is a group that can be cleaved by a chemical, photochemical, physical, biological or enzymatic process upon being brought in or under certain conditions. One of these conditions may be, for example, bringing a compound of the invention in an aqueous environment which leads to hydrolysis of **L,** or bringing a compound of the invention in an environment that contains an enzyme that recognizes and cleaves **L,** or bringing a compound of the invention under reducing conditions, which leads to reduction and/or removal of **L,** or bringing a compound of the invention under oxidizing conditions, which leads to oxidation and removal of **L,** or bringing a compound of the invention in contact with radiation, e.g., **UV** light, which leads to cleavage of **L,** or bringing a compound of the invention in contact with heat, which leads to cleavage of **L.** This condition may be met directly after administering a compound of this invention to an animal, e.g. a mammal, for example a human, due to the presence of ubiquitous enzymes in the circulatory system. Alternatively, said condition may be met when the compound localizes to a specific organ, tissue, cell, subcellular target, or bacterial, viral, or microbial target, for example by the presence of internal factors (e.g. target-specific enzymes or hypoxia) or application of external factors (e.g. radiation, magnetic fields).

In one embodiment, **L** can be a moiety that is cleaved by an enzyme or hydrolytic conditions present in the vicinity of or inside the target cells as compared to other parts of the body, or by an enzyme or hydrolytic conditions present only in the vicinity of or inside the target cells. It is important to recognize that if target site specificity is achieved solely based upon the selective transformation and/or cleavage of said **L** at the target site, the condition causing the cleavage should preferably, at least to a certain degree, be target site-specific.

In one embodiment, cleavage of **L** occurs intracellularly.

In another embodiment, cleavage of **L** occurs extracellularly.

In another embodiment, cleavage of **L** occurs by an ubiquitous intracellular enzyme.

Cleavage of the **Ant-L** bond results in the release of anthracyclines of formula (II)', (III)', (IV)' or (V)', as reported below: 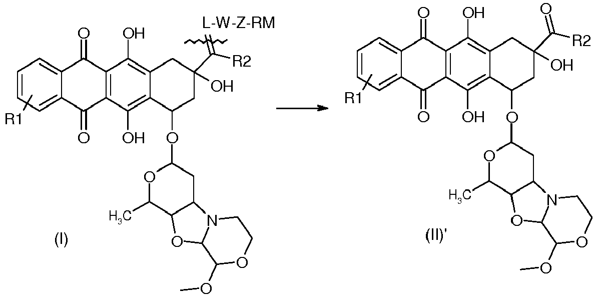 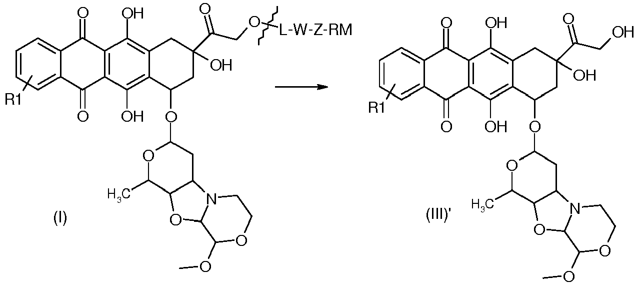 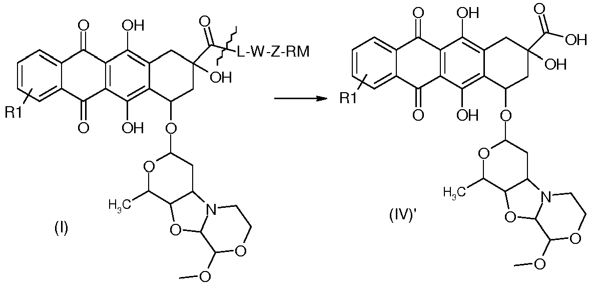 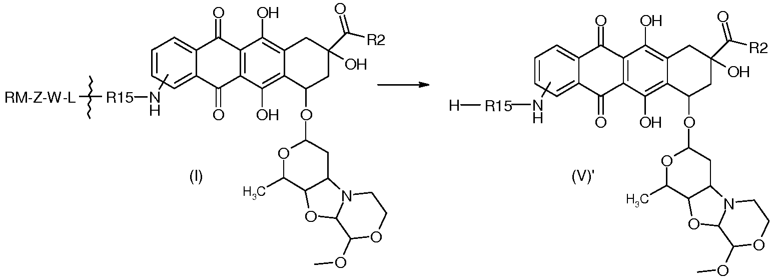 wherein the substituents R1 or H-R15-NH- are in position 4 of the anthracycline moiety and R2 is as defined above.

In one preferred embodiment, **L** may be a moiety that can be cleaved by ubiquitous enzymes, e.g., esterases that are present in the circulatory system or intracellular enzymes, such as for example proteases and phosphatases, or by pH-controlled hydrolysis. **L** may therefore form, optionally together with the connecting atom, an amide, amine, ester, ether, or hydrazone linkage that can be cleaved *in vivo.*

In a more preferred embodiment when **Ant** has formula (IIa), **L** is null or a group (VIc): 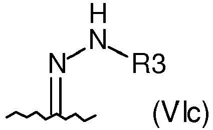 wherein:
**R3** is null or hydrogen, and the wavy line indicates the attachment point to **Ant.**

In another more preferred embodiment when **Ant** is a compound of formula (IIIa), **L** is null or a group (VIIa): 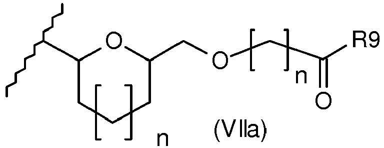 wherein:
**R9** is null;
**n** is an integer from 0 to 2, and
the wavy line indicates the attachment point to **Ant.**

In another more preferred embodiment when **Ant** has formula (IVa), **L** is null or a group of formula (VIIIa): 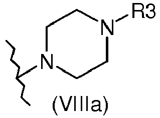 wherein
**R3** is as defined above and the wavy line indicates the attachment point to **Ant.**

In another more preferred embodiment when **Ant** has formula (Va), **L** is null or a group (IXc): 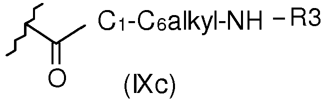 wherein:
C₁-C₆ alkyl is a straight or branched chain, **R3** is null or hydrogen and the wavy line indicates the attachment point to **Ant.**

### The group W

The **W** group, if present, is a group that in a compound of formula (I) tethers in a stable way the moiety **L** to the other part of derivative of formula (I) (**Z-RM**). The bond between **L** and **W,** or between **W** and **Ant** when **L** is null, can become labile upon activation by a chemical, photochemical, physical, biological or enzymatic process, upon being brought in or under certain conditions, as described above, leading optionally to the release of the corresponding moieties:

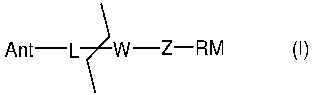

A group **W** may be incorporated in a compound of formula (I) for example to improve solubility or to improve space between the anthracycline moiety and the radical **RM;** in addition, said group **W** can modulate the reactivity of **RM** versus nucleophiles.

Moieties suitable as group **W** are known to the person skilled in the art (see for example those described in WO2002/083180 and WO2004/043493); or those described in Tranoy-Opalinsi, A. et al., Anticancer Agents in Medicinal Chemistry, 2008, 8, 618-637, Ahmed Alouane et al. Angew. Chem. Int. Ed. 2015, 54, 7492-7509. Other examples of these groups include, but are not limited to, optionally substituted 4-aminobutyric acid amides, appropriately substituted bicyclo[2.2.1] and bicyclo[2.2.2] ring systems or 2-aminophenylpropionic acid amides [see WO 2005/079398, WO 2005/105154 and WO 2006/012527; Greenwald, R.B., et al., Adv. Drug Delivery Rev. 2003, 55, 217-250; Kingsbury, W.D., et al., J. Med. Chem. 1984, 27, 1447-1451].

In one preferred embodiment, **W** may form, together with the connecting atom(s), a carbonate, carbamate, urea, ester, or amide linkage that can be optionally cleaved upon activation.

**W** is null or a group selected from the formulas (Xa), (Xc) and (Xf): 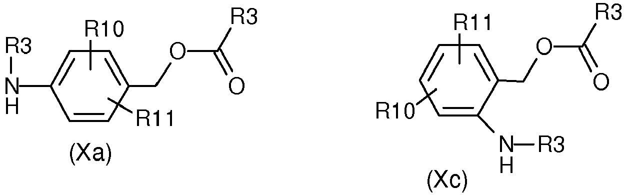 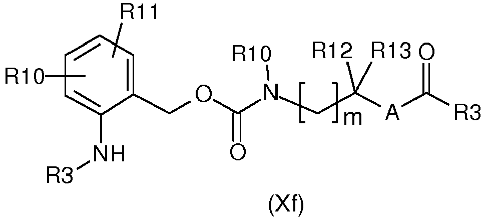 wherein
one of the two **R3** substituents is a tether to **L,** or to **Ant** when **L** is null, and the other is null;
**R10** and **R11** are hydrogen;
**m** is an integer from 0 to 3;
**A** is N-CH₃; and
**R12** and **R13** are hydrogen.

### The linker Z

The linker **Z**, if present, can be peptidic (Z1), non-peptidic (Z2) or hybrid (Z3), wherein said hybrid linker is peptidic and non-peptidic; in a compound of formula (I) said linker **Z** can be cleaved by a chemical, photochemical, physical, biological or enzymatic process, upon being brought in or under certain conditions, as described above, leading optionally to the release of the corresponding moieties (see e.g. Dubowchik G. M. et a/., Bioorg. Med. Chem. Lett. 8, 1998, 3347-3352; Burke P. J. et al., Bioorg. Med. Chem. Lett. 19, 2009, 2650-2653):

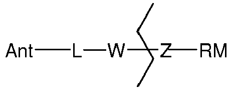

The bond between **Z** and the moiety **Ant-L-W, Ant-L** (when **W** is null) or **Ant** (when **L** and **W** are both null), can become labile upon activation by a chemical, photochemical, physical, biological or enzymatic process, upon being brought in or under certain conditions, as described above, leading optionally to the release of the corresponding moieties.

The linkage between **Z** and the connecting atom(s) may form a carbonate, carbamate, urea, ester, amide, thioamide or disulfide group.

The linker **Z** may be straight or branched.

In one embodiment **Z** is null.

In another embodiment **Z** is a peptidic linker **Z1** that can be cleaved by a proteolytic enzyme, plasmin, a cathepsin, β-glucuronidase, a galactosidase, prostate-specific antigen (PSA), urokinase-type plasminogen activator (u-PA) or a member of the family of matrix metalloproteinases.

In another embodiment **Z** is a non-peptidic linker **Z2** that may contain one or more non-peptidic water-soluble moieties. In this case the linker contributes to the water solubility of a compound of formula (I). In another embodiment **Z2** is a non-peptidic linker that may contain one or more non-peptidic moieties that reduce(s) aggregation of a compound of formula (I) which may or may not be a moiety/moieties that also increase(s) the water solubility of a compound of formula (I).

For example, non-peptidic water-soluble **Z2** linkers may contain an oligoethylene glycol or polyethylene glycol moiety or a derivative thereof.

In another embodiment, **Z** is a hybrid linker **Z3** that can contain both peptidic and non peptidic residues of general formula **Z1-Z2,** wherein **Z1** and **Z2** are independently a peptidic linker or a non-peptidic linker. Hybrid linkers may contribute to solubility of the compound of formula (I) and/or may be a substrate that can be cleaved by proteolytic enzymes, for example by a member of the family of matrix metalloproteinases.

In a preferred embodiment, **Z1** is a single aminoacid, a dipeptide, a tripeptide, a tetrapeptide, or an oligopeptide moiety, comprising natural L-aminoacids, unnatural D-aminoacids, synthetic aminoacids, or any combination thereof, wherein one of the C-terminal or the N-terminal aminoacid residue is linked to **Ant,** to **W** or to **L,** and the other terminal aminoacid residue is optionally linked to **RM.**

In a more preferred embodiment **Z1** is a dipeptide or a tripeptide, linked via its C-terminus to **W,** or to L when **W** is null, or to **Ant** when **W** and **L** are both null.

In another more preferred embodiment, the C-terminal aminoacid residue of the dipeptide or of the tripeptide is selected from glycine, leucine, alanine, arginine and citrulline; and the N-terminal aminoacid residue is selected from any natural or unnatural aminoacid; preferably, in case of the tripeptide, the middle aminoacid residue is selected from alanine, valine, leucine, isoleucine, methionine, phenylalanine, citrulline and proline.

In another more preferred embodiment **Z1** comprises a pentapeptide, wherein the C-terminal aminoacid is selected from any natural or unnatural aminoacid and the N-terminal aminoacid residue is 6-aminohexanoic acid.

In a preferred embodiment, **Z2** may contain an oligoethylene glycol or polyethylene glycol moiety or a derivative thereof.

In a preferred embodiment **Z3** is a hybrid moiety comprising a peptidic moiety **Z1,** wherein **Z1** is a single aminoacid, a dipeptide, a tripeptide or a tetrapeptide, comprising natural L-aminoacids and unnatural D-aminoacids; and a non-peptidic moiety **Z2,** wherein **Z2** is an oligoethylene glycol or polyethylene glycol moiety or a derivative thereof.

In another preferred embodiment **Z** is selected from the following formulas: 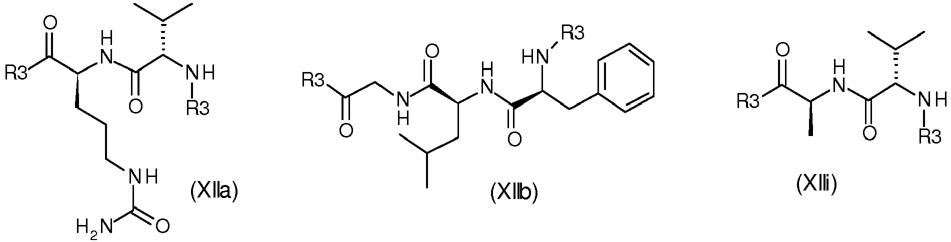 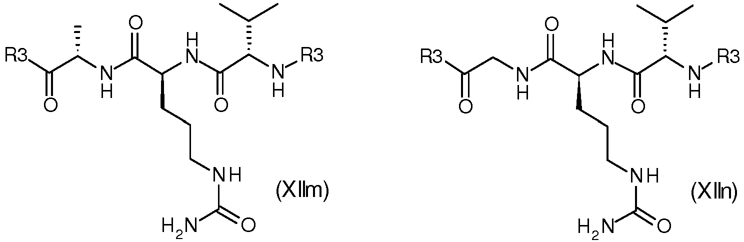 wherein
one of the two **R3** substituents is a tether to **Ant,** to **W** or to **L,** and the other is null.

### The radical RM

The **RM** moiety, if present, is an electrophilic group that can react with nucleophiles, i.e. molecules that bear a nucleophilic group, under relatively mild conditions and without the need of prior functionalization of the radical **RM,** said reaction between said radical **RM** and said nucleophile requiring only the application of one or more of the agents selected from the group comprising heat, pressure, a catalyst, an acid and a base.

The linkage between **RM** and **Z**, or between **RM** and **W** (when **Z** is null), or between **RM** and **L** (when **W** and Z are both null), or between **RM** and **Ant** (when **L, W** and **Z** are all null), can be cleaved by a chemical, photochemical, physical, biological or enzymatic process, upon being brought in or under certain conditions, as described above, leading optionally to the release of the corresponding moieties.

Therefore, when the **RM** moiety is present, a compound of formula (I) conjugates with different types of nucleophiles. When **RM** is null, a compound of formula (I) conjugates with different types of electrophiles, i.e. molecules that bear an electrophilic group, through one or more of the nucleophilic groups that are present on the **L, W,** and/or **Z** moiety(ies) e.g. amine, thiol and alcohol groups.

Examples of reactive moieties include, but are not limited to, carbamoyl halide, acyl halide, activated ester, anhydride, α-haloacetyl, α-haloacetamide, maleimide, isocyanate, isothiocyanate, disulfide, thiol, hydrazine, hydrazide, sulfonyl chloride, aldehyde, methyl ketone, vinyl sulfone, halomethyl, and methyl sulfonate.

In one preferred embodiment of the invention, when the nucleophilic group of the nucleophile, which **RM** can react with, is NH, NH₂, SH or OH, **RM** is null or selected from the following formulas: 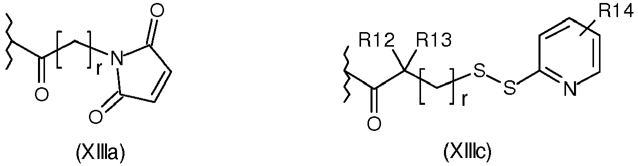 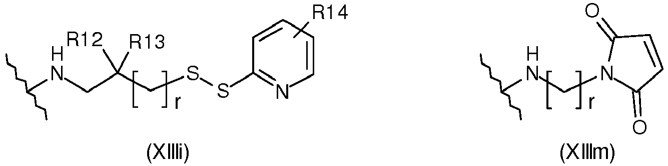
wherein **R14** is hydrogen;
**r** is an integer from 0 to 7; **R12** and **R13** are, each independently, hydrogen or methyl, and the wavy line indicates the attachment point in the derivative of formula (I).

From all of the above, it is to be noted that a morpholinyl anthracycline derivative of formula (I) can be cleaved at all bonds linking moieties **L, W, Z** and **RM,** releasing the said anthracyclines of formula (II)', (III)', (IV)' or (V)'.

Specific, not limiting, preferred compounds (comp.) of formula (I) of the present invention, optionally in the form of a pharmaceutically acceptable salt, are selected from the group consisting of the following compounds **(1)-(20), (23)-(27), (75)-(78):** 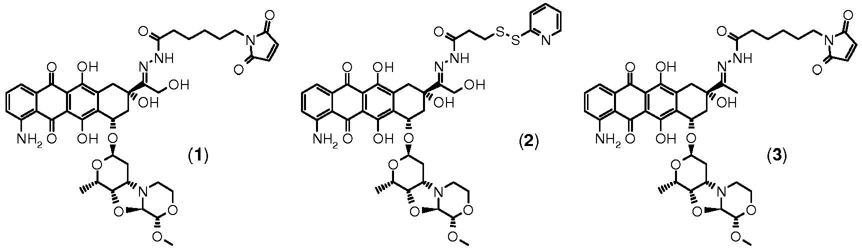 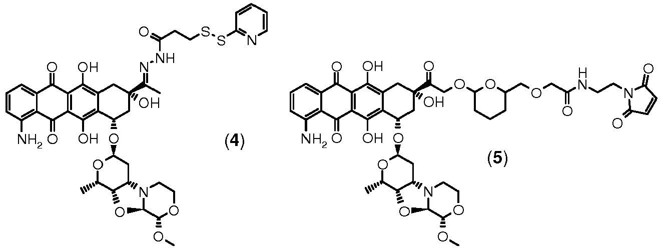 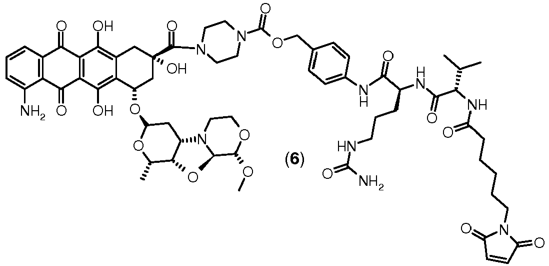 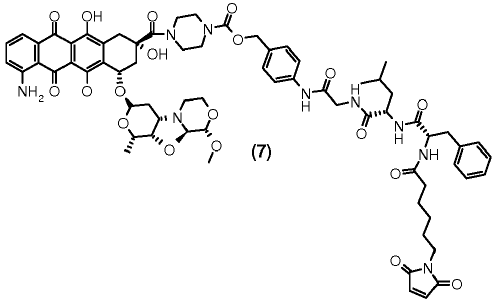 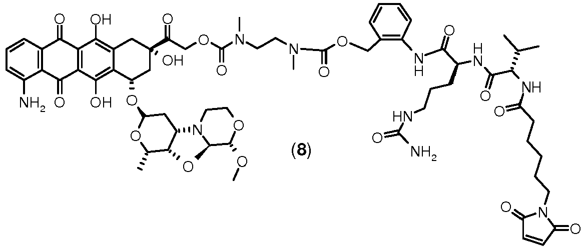 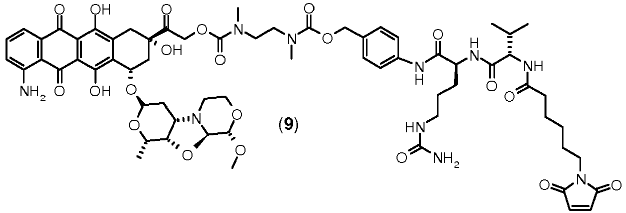 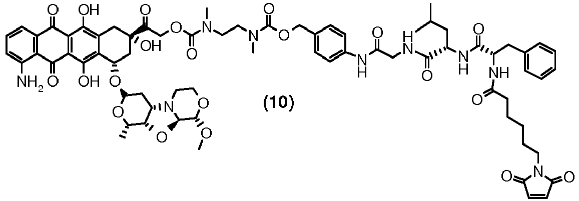 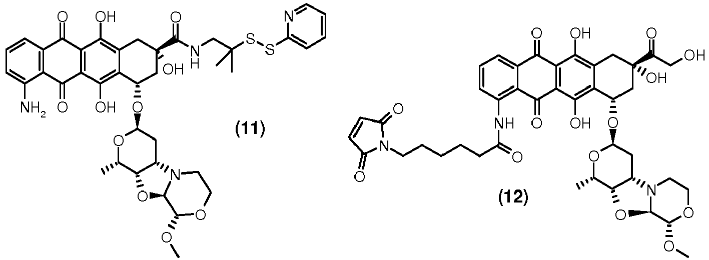 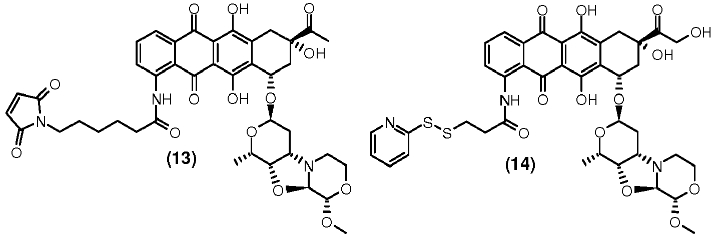 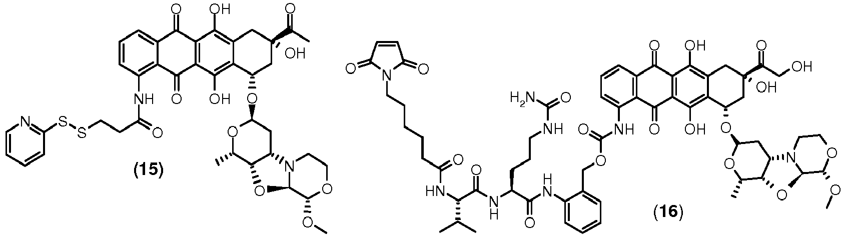 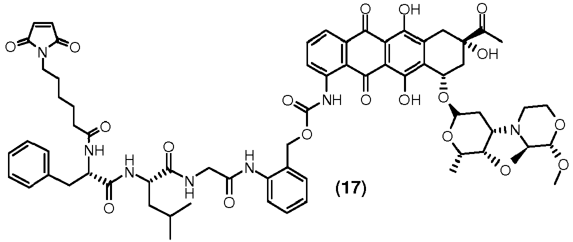 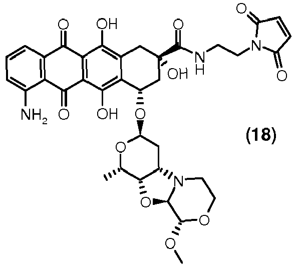 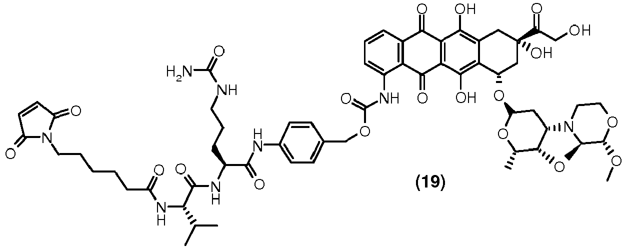 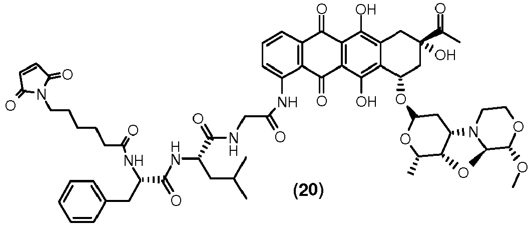 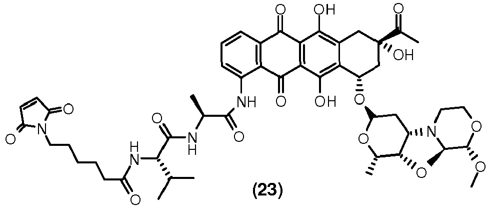 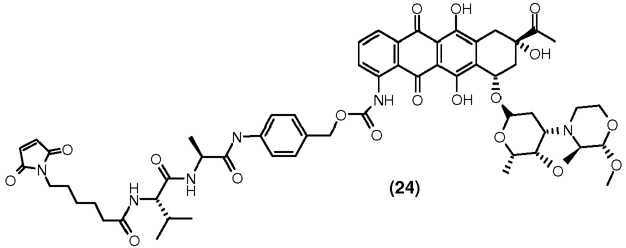 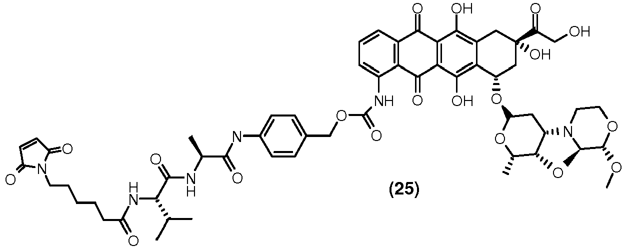 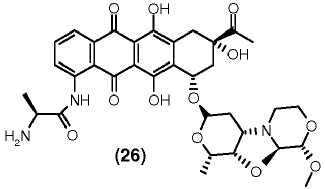 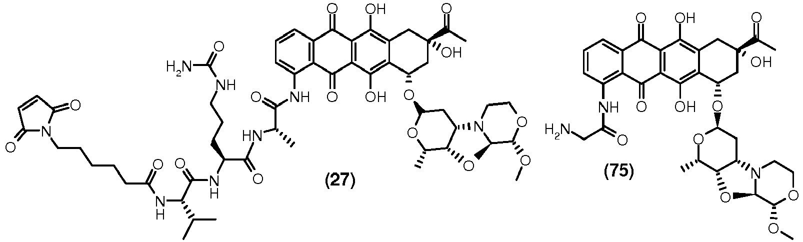 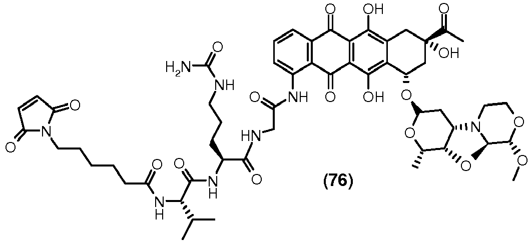 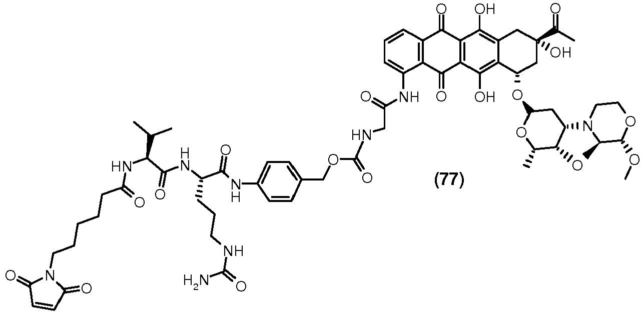 and

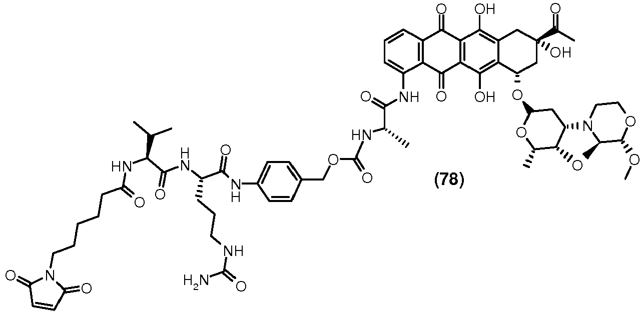

For a reference to any specific compound of formula (I) of the invention, optionally in the form of a pharmaceutically acceptable salt, see the experimental section and claims.

The present invention also provides a process for the preparation of a compound of formula (I) as defined above, by using the reaction routes and synthetic schemes described below, employing the techniques available in the art and starting materials readily available. The preparation of certain embodiments of the present invention is described in the examples that follow, but those of ordinary skill in the art will recognize that the preparations described may be readily adapted to prepare other embodiments of the present invention. For example, the synthesis of non-exemplified compounds according to the invention may be performed by modifications apparent to those skilled in the art, for instance by appropriately protecting interfering groups, by changing to other suitable reagents known in the art, or by making routine modifications of reaction conditions. Alternatively other reactions referred to herein or known in the art will be recognized as having adaptability for preparing other compounds of the invention.

### Methods of Synthesis

The derivatives of formula (I) may be prepared by several routes, employing organic chemistry reactions, conditions, and reagents known to those skilled in the art. The derivatives of formula (I) may be prepared by coupling each moiety either in a sequential fashion, i.e. by adding each moiety one by one, or in a concurrent fashion, i.e. by forming advanced intermediates and finally coupling them to obtain the desired derivatives.

The coupling reactions include non limiting examples such as:
(1) reaction of a nucleophilic or an electrophilic group of an anthracycline of formula (II)', (III)', (IV)' and (V)' with a bivalent reagent **L,** to form an intermediate **Ant-L** via a covalent bond, optionally followed by reaction with a bivalent reagent **W,** to form an intermediate **Ant-L-W** via a covalent bond, optionally followed by reaction with a bivalent reagent **Z**, to form an intermediate **Ant-L-W-Z** via a covalent bond, and finally optionally followed by reaction with a radical **RM** to form the final functionalized morpholinyl anthracycline derivative (I);
(2) reaction of a nucleophilic or an electrophilic group of an anthracycline of formula (II)', (III)', (IV)' and (V)' with a bivalent reagent **L-W-Z-,** to form the **Ant-L-W-Z** via a covalent bond, followed by reaction with a radical **RM** to form the final functionalized morpholinyl anthracycline derivative (I);
(3) reaction of a nucleophilic or an electrophilic group of an anthracycline of formula (II)', (III)', (IV)' and (V)' with a reagent **L-W-Z-RM** to form the final functionalized morpholinyl anthracycline derivative (I).

Functionalized derivatives (I) may then be conjugated with a variety of proteins, drug moieties, peptides, aptamers, polymers or nanoparticles to prepare drug-conjugates compounds.

Nucleophilic groups on intermediates include, but are not limited to: (i) N-terminal amine groups, (ii) side chain amine groups, e.g. lysine, (iii) side chain thiol groups, e.g. cysteine, (iv) hydroxyl groups. These groups are capable of reacting with electrophilic groups on linker moieties and linker reagents including: (i) active esters such as NHS (N-hydroxysuccinimide) esters, HOBt esters, haloformates, and acid halides; (ii) alkyl and benzyl halides such as haloacetamides; (iii) aldehydes, ketones, carboxyl, and maleimide groups.

### Preparation of anthracyclines of formula (II)', (III)', (IV)' and (V)'

In all reaction schemes shown in the following, the substituents **R1** or H-**R15**-NH- are in position 4 of the anthracycline moiety.

The anthracyclines of formula (II)', (III)', (IV)' and (V)', which are reacted in the preparation of the derivatives of formula (I), can be prepared according to any one of the alternative pathways summarized in **Scheme X** below; also summarized in **Scheme X** is the preparation of the intermediate compound of formula (XXIV) according to **Pathway F** and the preparation of the starting material compound of formula (XX) according to **Pathway G.**

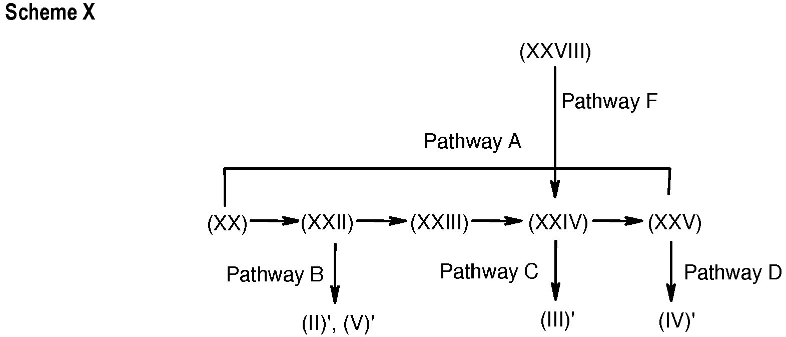

### Pathway A

An intermediate compound of formula (XXV), wherein R1 is NH₂, is prepared as summarized in Scheme A below.

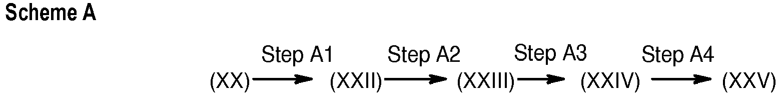

Accordingly, a process of the present invention comprises the following steps:
**A1)** reacting a compound of formula (XX) 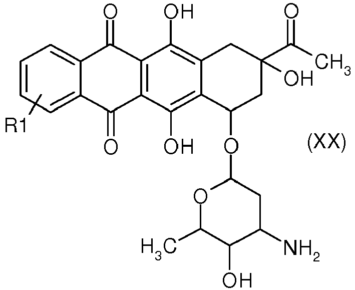 wherein **R1** is as defined above;
   with a compound of formula (XXI) 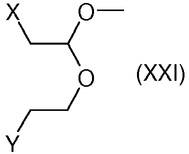 wherein **X** and **Y** are, the same or different, leaving groups, preferably halogens;
**A2)** reacting the resultant compound of formula (XXII) 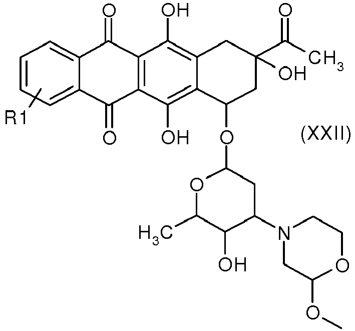 wherein **R1** is as defined above,
   with ethylorthoformate and bromine, then adding HBr;
**A3)** reacting the resultant compound of formula (XXIII) 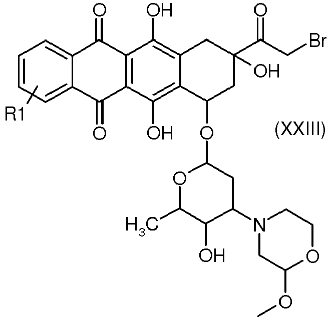 wherein **R1** is as defined above,
   with a formylating agent;
**A4)** oxidizing the resultant compound of formula (XXIV) 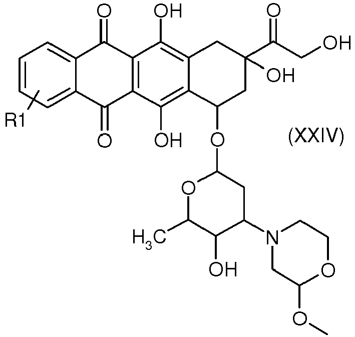 wherein **R1** is as defined above;
**A5)** reacting the resultant compound of formula (XXV) 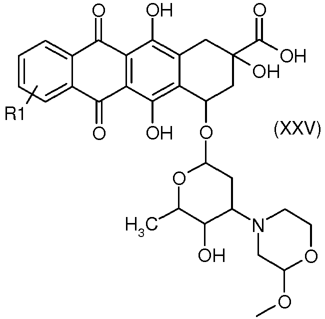 wherein **R1** is as defined above.

According to **step A1)** the reaction of the compound of formula (XX) with the compound of formula (XXI) is performed in an organic solvent, preferably DMF, at room temperature, following well known procedures reported in the art (see for example WO91/09046).

According to **step A2**) the reaction of the compound of formula (XXII) with ethylorthoformate and bromine and then with HBr is performed in two steps following well known procedures reported in the art (see for example Doxorubicin Anticancer Antibiotics Vol. 17, 1981, p.168; F. Arcamone et al. J. Med. Chem. 1974, 17, p. 335).

According to **step A3**) the reaction to obtain the compound of formula (XXIV) is performed following well known procedures reported in the art (see for example Doxorubicin Anticancer Antibiotics Vol. 17, 1981, p.168; US3803124). An example that is not intended to limit the method is the reaction of the compound of formula (XXIII) with sodium formate. The reaction is performed in CH₃CN or acetone or a mixture of them, at a temperature ranging from about 20 °C to reflux and for a time ranging from about 30 minutes to about 24 hours.

According to **step A4)** the oxidation of the compound of formula (XXIV) is performed with an oxidizing reagent, preferably NaIO₄. The reaction is performed in MeOH or water or a mixture of them, at a temperature ranging from about 20 °C to reflux and for a time ranging from about 30 minutes to about 24 hours.

According to **steps A6a)** and **A6b)** the reaction of the compound of formula (XXVI) first with DMDO and then the reaction of the resultant compound of formula (XXVII) with cyanuric chloride or with an iron(II) salt is carried out following well known procedures reported in the art (see e.g. GB2296495A; WO2012073217; WO9802446). Removal of the nitrogen and/or hydroxy protecting groups, if necessary, is performed following well known procedures reported in the art (see e.g. Protective Groups in Organic Synthesis; Theodora W. Greeen, Peter G. M. Wuts 4th edition).

### Pathway B

A compound of formula (II)', as defined above, wherein **R1** is -NH₂;
and **R2** is methyl, is prepared as summarized in **Scheme B** below.

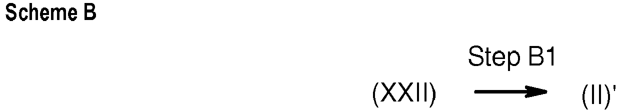

Accordingly, a process of the present invention comprises the following step:
**B1)** reacting the compound of formula (XXII) as defined in **Pathway A,** under the following conditions:
**B1a)** first with DMDO;
**B1b)** then treating the resultant compound of formula (XXVII) 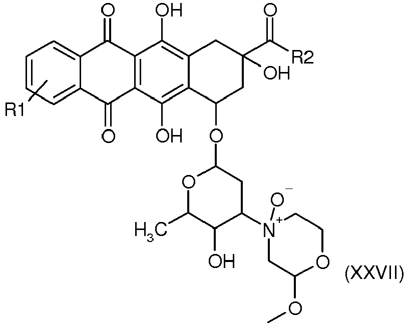 wherein **R1** and **R2** are as defined above, with cyanuric chloride or with an iron(II) salt, and finally, if desired, removing the nitrogen and/or hydroxy protecting group/s to obtain a compound of formula (II)' 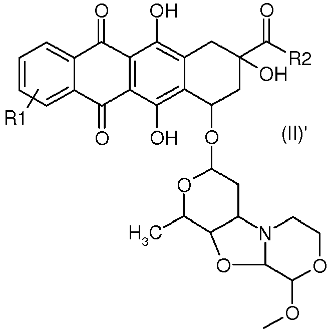 wherein **R1** and **R2** are as defined above;
   optionally converting a first compound of formula (II)' as defined above into a second compound of formula (II)' by known chemical reactions; and/or, if desired, converting a compound of formula (II)' into a pharmaceutically acceptable salt thereof or converting a salt into a free compound of formula (II)'.

It is noted that when **R1** is -NH₂, a derivative of formula (II)' corresponds to a derivative of formula (V)', therefore the above described preparation is intended to refer to the preparation of a derivative of formula (V)' too.

According to step **B1)** the reaction is respectively carried out as described above under **steps A6a)** and **A6b)**. According to **steps B1a)** and **B1b)** the reaction of the compound of formula (XXII) first with DMDO and then the reaction of the resultant compound of formula (XXVIII) with cyanuric chloride or with an iron(II) salt is carried out following well known procedures reported in the art (see e.g. GB2296495A; WO2012073217; WO9802446). Removal of the nitrogen and/or hydroxy protecting groups, if necessary, is performed following well known procedures reported in the art (see e.g. Protective Groups in Organic Synthesis; Theodora W. Greeen, Peter G. M. Wuts 4th edition).

### Pathway C

A compound of formula (III)', wherein **R1** is -NH₂;
is prepared as summarized in **Scheme C** below.

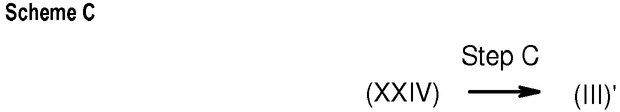

Accordingly, a process of the present invention comprises the following step:
**C)** reacting the compound of formula (XXIV) as defined in **Pathway A,** under the same conditions reported above under steps **B1a)** and **B1b)**.
to obtain a compound of formula (III)' 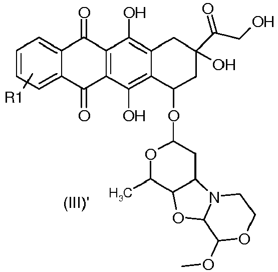 wherein **R1** is as defined above.

### Pathway D

A compound of formula (IV)', wherein R1 is -NH₂;
and **R2** hydroxy, is prepared as summarized in **Scheme D** below.

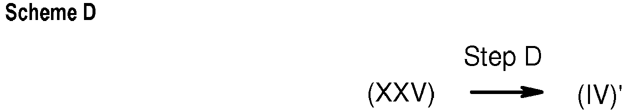

Accordingly, a process of the present invention comprises the following step:
**D)** reacting the compound of formula (XXV) as defined in **Pathway A,** under the same conditions reported above under steps **B1a)** and **B1b)**,
to obtain a compound of formula (IV)' 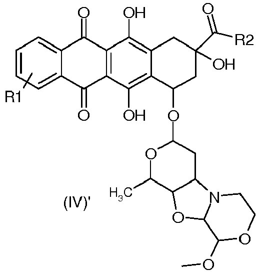 wherein **R1** is as defined above and **R2** is hydroxy.

### Pathway F

The derivative of formula (XXIV) as defined in **Pathway A** is alternatively prepared according to **Scheme F** below.

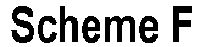

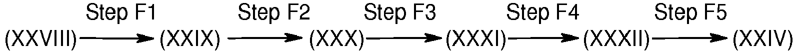

Accordingly, a process of the present invention comprises the following steps:
**F1)** reacting a compound of formula (XXVIII) 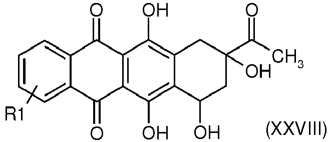 wherein **R1** is -NH₂, with bromine and potassium acetate;
**F2)** reacting the resultant compound of formula (XXIX) 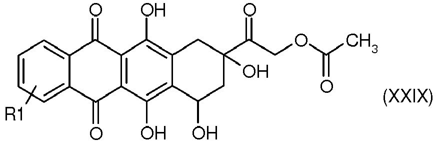 wherein **R1** is as defined above, with the sugar of formula (XXIXa) 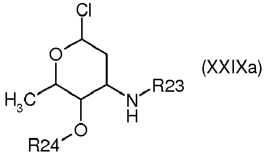 wherein **R23** and **R24** are independently hydrogen or a suitable nitrogen or hydroxy protecting group, as e.g. trifluoroacetyl or benzyl;
**F3)** reacting the resultant compound of formula (XXX) 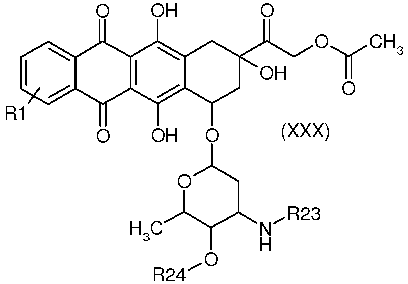 wherein **R1, R23** and **R24** are as defined above, with ethylorthoformate and pyridinium *p*-toluenesulfonate (PPTS);
**F4)** reacting the resultant compound of formula (XXXI) 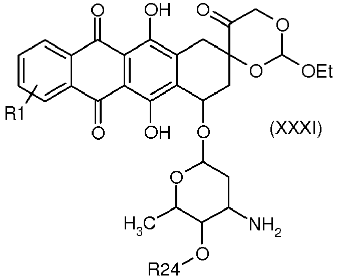 wherein **R1** and **R24** are as defined above, with a compound of formula (XXI) as defined above;
**F5)** deprotecting the resultant compound of formula (XXXII) 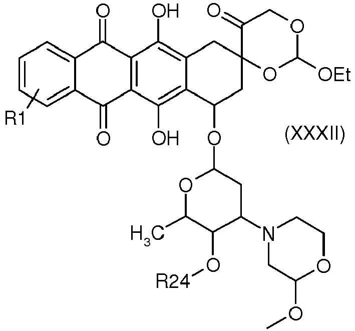 wherein **R1** and **R24** are as defined above, to yield the compound of formula (XXIV) as defined above.

According to **step F1)** the reaction is performed in an organic solvent preferably acetone or dioxane, at a temperature ranging from 20 °C to reflux and for a time ranging from about 30 minutes to about 24 hours.

According to **step F2)** the glicosidation reaction of the compound of formula (XXIX) is performed in presence of silver trifluoromethanesulfonate following well known procedures reported in the art (see for example GB2225781; GB2215332A).

According to **step F3)** the reaction to obtain the compound of formula (XXXI) is performed reacting the compound of formula (XXX) with ethylorthoformate and PPTS. The reaction is performed in an organic solvent, preferably DCM, at a temperature ranging from 0 °C to reflux and for a time ranging from about 30 minutes to about 24 hours. According to **step F4)** the reaction is performed as described above under step A1.

According to **step F5)** the removal of the hydroxy protecting group is performed following well known procedures reported in the art (see e.g. Protective Groups in Organic Synthesis; Theodora W. Greeen, Peter G. M. Wuts 4th edition).

Anthracyclines of formula (II)', (III)', (IV)' and (V)' are then reacted with suitable reagents to prepare the final functionalized morpholinyl anthracycline derivatives of formula (I). Such reagents can be, e.g., **L, L-W, L-W-Z, L-W-Z-RM,** etc. depending on whether a sequential or a concurrent method of synthesis is used to link the moieties in order to obtain the final derivatives of formula (I).

The compounds of formula (XXVIII) can be prepared as described in Pat. App. EP288268.

The compounds of the formula (XXI), (XXIXa) are either commercially available or can be prepared with known methods.

From all of the above, it is clear to the skilled person that when preparing the compounds of formula (I) according to any one of the aforementioned process variants, optional functional groups within the starting materials or the intermediates thereof, that could give rise to unwanted side reactions, need to be properly protected according to conventional techniques. Likewise, the conversion of these latter into the free deprotected compounds may be carried out according to known procedures.

As it will be readily appreciated, if the compounds of formula (I) prepared according to the process described above are obtained as mixture of isomers, their separation using conventional techniques into the single isomers of formula (I) is within the scope of the present invention.

### PHARMACOLOGY

The new morpholinyl anthracycline derivatives of the present invention are functionalized compounds useful as antitumor agents or useful to be conjugated to proteins, peptides, aptamers, polymers or nanoparticles.

A mammal, e.g. a human or animal, may therefore be treated by a method comprising administering thereto a pharmaceutically effective amount of a morpholinyl anthracycline derivative of formula (I). The condition of the human or animal may be ameliorated or improved in this way.

The evaluation of the cytotoxicity of the compounds of formula (I) is assessed as described below.

### In vitro cell proliferation assay

Human cancer cell lines were seeded in white 384 well-plates (1250 cells/well) in complete medium (RPMI1640 or E-MEM plus 10% Fetal bovine serum) and treated with compounds dissolved in 0.1% DMSO, 24 h after seeding. The cells were incubated at 37°C and 5% CO₂ and after 72 h the plates were processed using CellTiter-Glo assay (Promega) following the manufacturer's instruction.

CellTiter-Glo is a homogenous method based on the quantification of the ATP present, an indicator of metabolically active cells. ATP is quantified using a system based on luciferase and D-luciferin resulting into light generation. Briefly, 25 µL/well of reagent solution are added to each well and, after 5 minutes shaking, microplates are read by a luminometer. The luminescent signal is proportional to the number of live cells present in culture.

Dose-response curves were generated by sigmoid function interpolation of 8 concentration points and the antiproliferative activity of compounds was reported as the half-maximal inhibitory concentration (IC₅₀).

In particular, the cytotoxicity of compounds (**21**), (**22**) and (**26**) depicted below, that can be released from a compound of formula (I), has been assessed (**Table 1**):

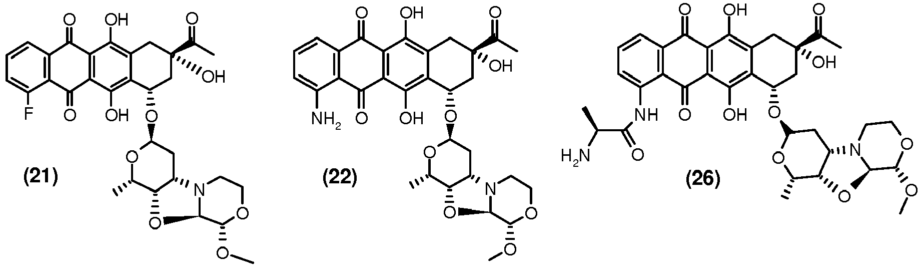

**Table 1**

| **Cell Line IC₅₀ nM** | | | | | | | |
|---|---|---|---|---|---|---|---|
| Cell Line | A2780 | HCC1954 | HCT-116 | HELA | MCF7 | MDA-MB_213 | MDA-MB-468 |
| Comp. **21** | 0.032 | 0.377 | 0.146 | 0.349 | 3.52 | 0.306 | 0.072 |
| Comp. **22** | 0.044 | 0.549 | 0.2 | 0.7 | 0.668 | 0.538 | 0.313 |
| Comp. **26** | 1.2 | 4.8 | 1.1 | 3.5 | 3.8 | nd | 2.3 |

These derivatives are encompassed respectively by formula (II)' (compound (**21**), when R1 is fluorine and R2 is methyl; compound (**22**), when R1 is amino and R2 is methyl) and by formula (V)' (compound (**22**), when R2 is methyl and R15 is null; compound (**26**), when R2 is methyl and R15 is -NR7-C₁-C₆ alkylcarbonyl*, with R7 being hydrogen and C₁-C₆ alkyl being -(*S*)CH(CH₃)-).

As can be appreciated by the skilled person from the above pharmacological data, these representative compounds, namely (**21**), (**22**) and (**26**), are particularly advantageous in antitumor therapy.

These compounds are non limiting examples of anthracyclines which can be released *in vivo* by derivatives of formula (I).

As an example, in the case of compound (**21**), it is expected to be released by a derivative of formula (I), wherein **Ant** has formula (IIa), R1 is fluorine and R2 is methyl, as depicted in **Scheme 1:**

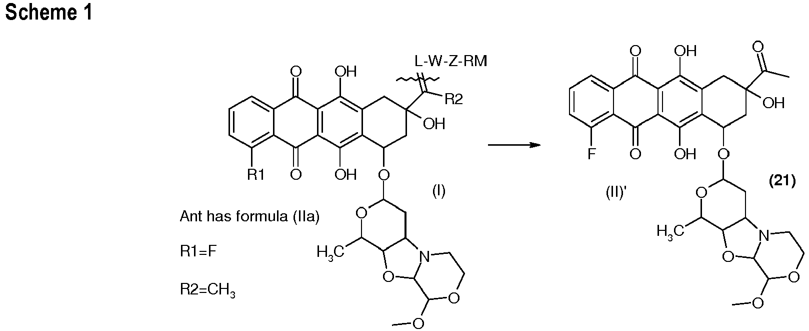

As a further example, in the case of compound (**22**), it is expected to be released either by a derivative of formula (I), wherein **Ant** has formula (IIa), R1 is amino and R2 is methyl, or by a derivative of formula (I), wherein **Ant** has formula (Va), R2 is methyl and R15 is null, as depicted in **Scheme 2:**

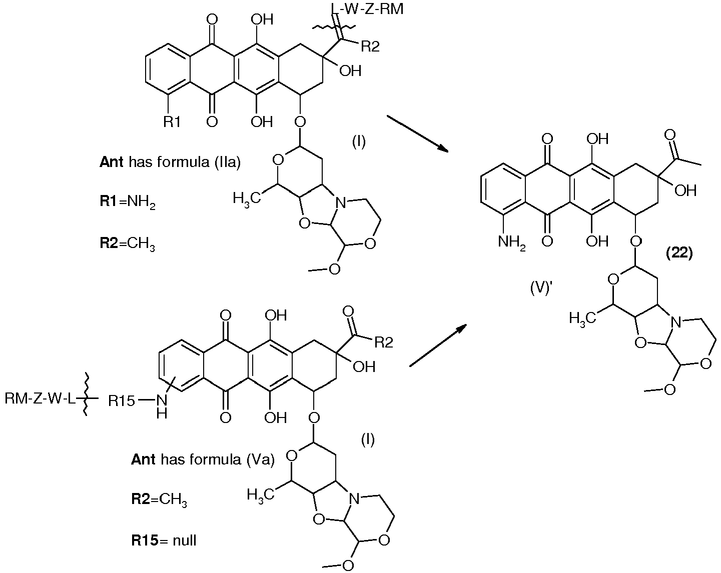

The compounds of formula (I) of the present invention can be administered either as single agents or, alternatively, in combination with known anticancer treatments such as radiation therapy or chemotherapy regimen, in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrix metalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g. to humans, can be administered by the usual routes and the dosage level depends upon the age, the weight, the conditions of the patient and the administration route.

For example, a suitable dosage adopted for oral administration of a compound of formula (I) may range from about 1 to about 300 mg per dose, from 1 to 5 times daily. The compounds of the invention can be administered in a variety of dosage forms, e.g. orally, in the form of tablets, capsules, sugar or film coated tablets, liquid solutions or suspensions; rectally in the form of suppositories; parenterally, e.g. subcutaneously, intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a carrier or a diluent.

The pharmaceutical compositions containing the compounds of the invention are usually prepared following conventional methods and are administered in a suitable pharmaceutical form. For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gum, gelatine methylcellulose, carboxymethylcellulose or polyvinyl pyrrolidone; disintegrating agents, e.g. starch, alginic acid, alginates or sodium starch glycolate; effervescing mixtures; dyestuffs; sweeteners; wetting agents, such as lecithin, polysorbates, laurylsulfates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, for instance, syrups, emulsions and suspensions. As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusions may contain, as a carrier, sterile water or preferably they may be in the form of sterile, aqueous, isotonic, saline solutions or they may contain propylene glycol as a carrier. The suppositories may contain, together with the active compound, a pharmaceutically acceptable carrier, e.g. cocoa butter, polyethylene glycol, a polyoxyethylene sorbitan fatty acid ester surfactant or lecithin.

With the aim at better illustrating the present invention, without posing any limitation to it, the following examples are now given.

### EXPERIMENTAL PART

The synthetic preparation of some morpholinyl anthracycline derivatives of formula (I) of the invention is described in the following examples. The compounds of the present invention, as prepared according to the following examples, were also characterized by ¹H-NMR and/or by Exact mass data ESI(+).

¹H-NMR spectra were recorded at a constant temperature of 28°C on a Varian INOVA 400 spectrometer operating at 400.50 MHz and equipped with a 5 mm z-axis PFG Indirect Detection Probe (¹H{¹⁵N-³¹P}).

Chemical shifts were referenced with respect to the residual solvent signals (DMSO-*d₆*: 2.50 ppm for ¹H, where not otherwise specified). Data are reported as follows: chemical shift (δ), multiplicity (s = singlet, d = doublet, t = triplet, q = quartet, br. s. = broad singlet, td = triplet of doublets, dd = doublet of doublets, ddd = doublet of doublets of doublets, m = multiplet, spt = septet), coupling constants (J, Hz), and number of protons.

Exact mass data ESI(+) were obtained on a Waters Q-Tof Ultima mass spectrometer directly connected with a Agilent 1100 micro-HPLC system as previously described (M. Colombo, F. Riccardi-Sirtori, V. Rizzo, Rapid Commun. Mass Spectrom. 2004, 18, 511-517).

In the examples below, as well as throughout the application, the following abbreviations have the following meanings. If not defined, the terms have their generally accepted meanings.

| ABBREVIATIONS | |
|---|---|
| DCC | *N,N'*-dicyclohexylcarbodiimide |
| DCM | dichloromethane |
| DIPEA | *N,N*-diisopropylethylamine |
| DMDO | dimethyldioxirane |
| DMF | *N,N*-dimethylformamide |
| DMSO | dimethyl sulfoxide |
| EDC | 1-ethyl-3-(3-dimethylaminopropyl)carbodiimide |
| Et₂O | diethyl ether |
| EtOAc | ethyl acetate |
| EtOH | ethanol |
| HCl | hydrochloric acid |
| HOBt | 1H-benzotriazol-1-ol |
| MeOH | methanol |
| Na₂SO₄ | sodium sulfate |
| NaHCO₃ | sodium hydrogen carbonate |
| NaOH | sodium hydroxide |
| PPTS | pyridinium *p*-toluenesulfonate |
| TEA | triethylamine |
| TFA | trifluoro acetic acid |
| THF | tetrahydrofuran |

### Example (A)

### Preparation of conjugates with MCM2 protein

1.5 mg (0.045 µmol) of MCM2 protein (corresponding to residues 10-294 of the full length sequence, see Ishimi et al., 2001 Journal Biological Chemistry, vol. 276, pages 42744-42752) were dissolved in 0.5 ml of phosphate buffered saline solution (pH 7.2), pH value was adjusted to 8.5 by addition of 55 µL of 1M NaHCO₃ (pH 8.5) and 0.040 - 0.080 µmol of a derivative of formula (I) were added through a 10 mg/ml DMSO solution. The reaction was incubated for 1 h at room temperature then the reaction mixture was desalted on a NAP-10 column conditioned in phosphate buffered saline solution and the fractions containing the protein were collected and pooled.

The MCM2 conjugate thus obtained was characterized by HPLC/ESI or Q-ToF2/nanoESI mass spectrometry analysis following procedures below.

### Procedure A

Conjugated MCM2-Comp. **(3) (Adduct 1),** obtained as described above, was incubated in acidic conditions (pH 3) in order to prompt hydrolysis of the hydrazone bond so to obtain the release of the anthracycline derivative (**22**) and the MCM2-linker residue.

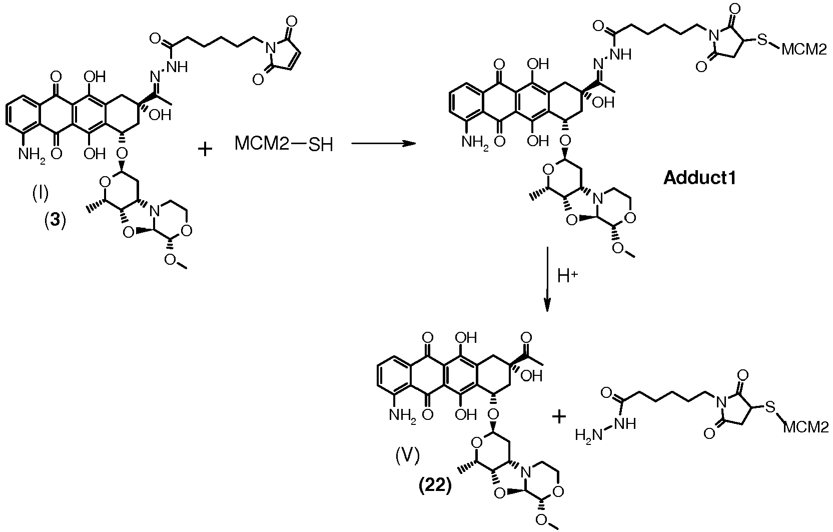

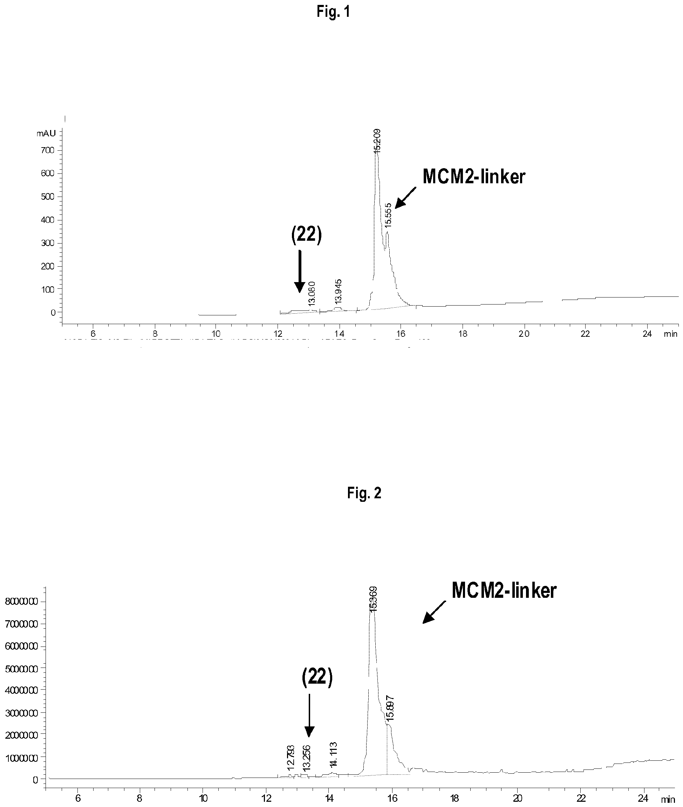

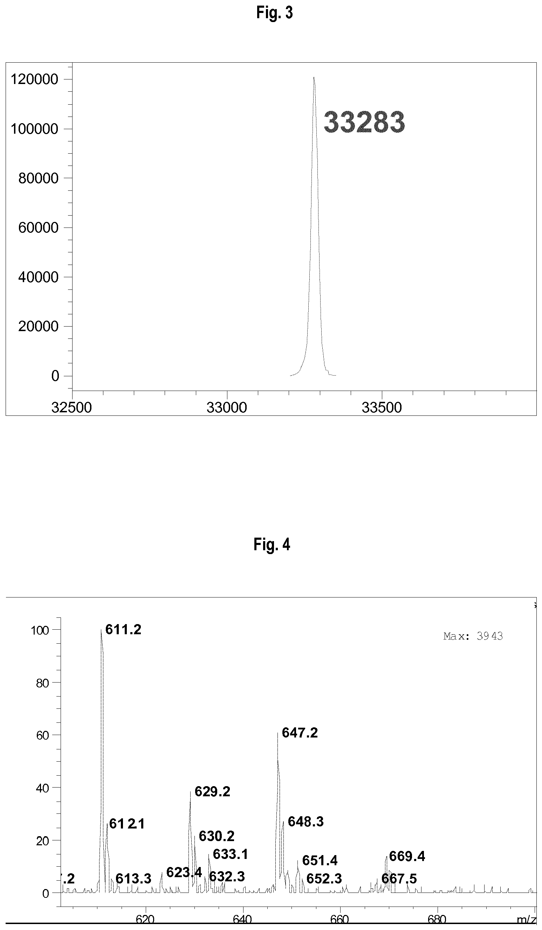

The sample was analyzed and products characterized by HPLC/MS analysis using linear gradient from 5-100% B (solvent A: 0.05% TFA in water; solvent B: 0.05% TFA in acetonitrile) as eluant. The detection was performed by recording the UV signal at 220 nm through a diode array system and the m/z signal through the quadrupole MSD in the 600-2000 acquisition range. Mass spectra from m/z signal integration under the protein peaks were deconvoluted using the Agilent Chemstation algorithm to obtain the MW of the corresponding protein species at -100 ppm accuracy (Fig. 1-4).
Fig. 1 shows the HPLC profile of Adduct 1, reporting the time (min) on the x axis and UV absorbance @ 220 nm (mAU) on the y axis.
**Fig. 2** shows the total ion current chromatogram of **Adduct 1,** reporting the time (min) on the x axis and intensity expressed as counts per second (cps) on the y axis.
**Fig. 3** shows the deconvoluted MS spectrum chromatographic peak at 15.2 min of the total ion current chromatogram of **Adduct 1,** shown in **Fig. 2****,** corresponding to MCM2-linker residue (expected MW 33282 Da; observed MW 33283 Da); the molecular weight is reported on the x axis while intensity expressed as counts per second (cps) is reported on the y axis.
**Fig. 4** shows the MS spectrum chromatographic peak at 13 min of Comp. **22,** shown in **Fig. 2** (expected m/z 611 Da; observed m/z 611.2 Da); the molecular weight (m/z) is reported on the x axis while intensity expressed as counts per second (cps) is reported on the y axis.

### Procedure B

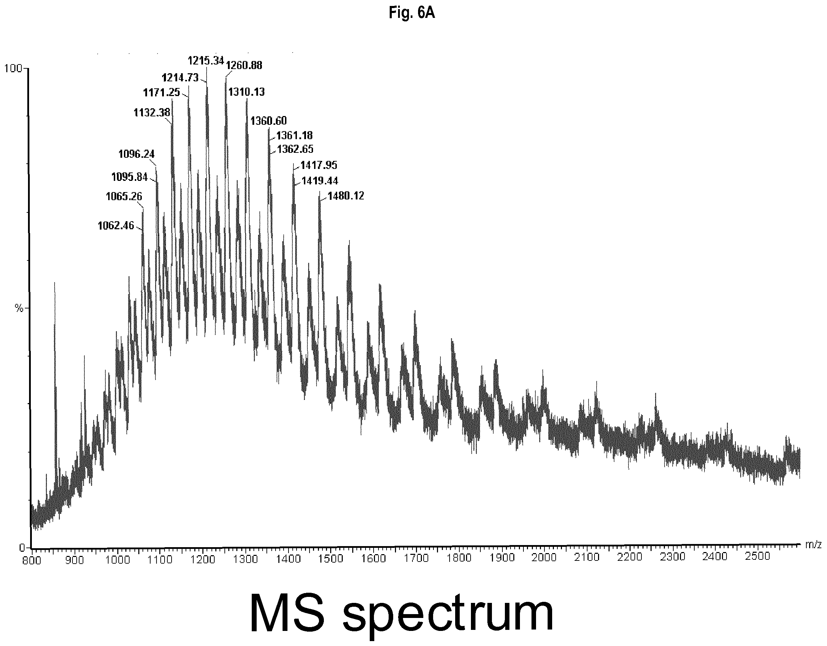

Characterization of **Adduct 1** was also performed by MS analysis at neutral pH conditions, using ammonium acetate 100 mM buffer pH 7. The sample was directly injected in a Q-ToF2 mass spectrometer (Micromass, UK), equipped with a nanoESI source, using a flow rate of 0.5 µL/min; the capillary voltage was set at 2 KV and the MS signal was collected in the 500-3000 m/z range (Fig. 6A-6B).

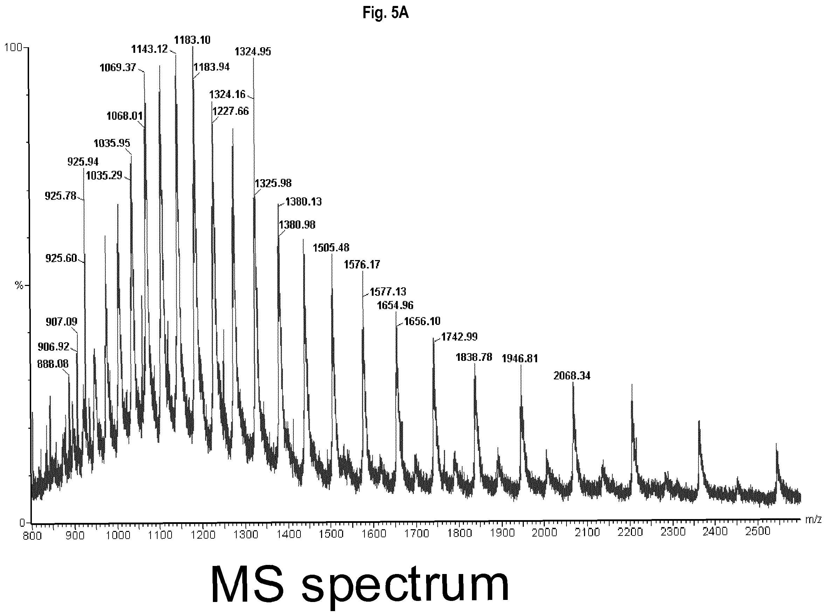

MCM2 was used as a standard to set the appropriate instrumental parameters. Sample was injected into the mass spectrometer (**Fig. 5A-5B**). Mass spectra were deconvoluted using the MaxEnt1 algorithm of the MassLynx 4.0 software to obtain the MW of the corresponding protein species at ∼100 ppm accuracy.
**Fig. 5A** shows the MS spectrum of the MCM2 standard protein; the molecular weight (m/z) is reported on the x axis while intensity expressed as counts per second (cps) is reported on the y axis.

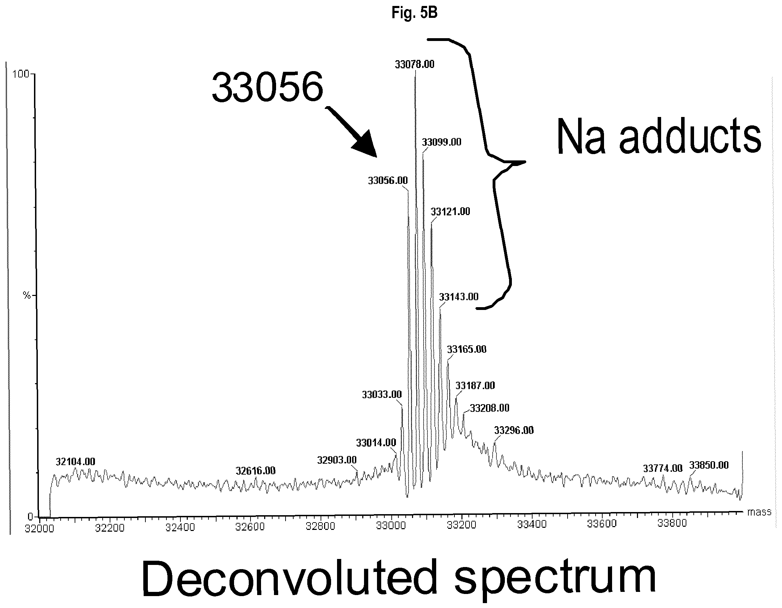

**Fig. 5B** shows the deconvoluted MS spectrum of the MCM2 standard protein (expected MW 33057 Da; observed MW 33056 Da); the molecular weight is reported on the x axis while intensity expressed as counts per second (cps) is reported on the y axis.
**Fig. 6A** shows the MS spectrum of the sample; the molecular weight (m/z) is reported on the x axis while intensity expressed as counts per second (cps) is reported on the y axis.

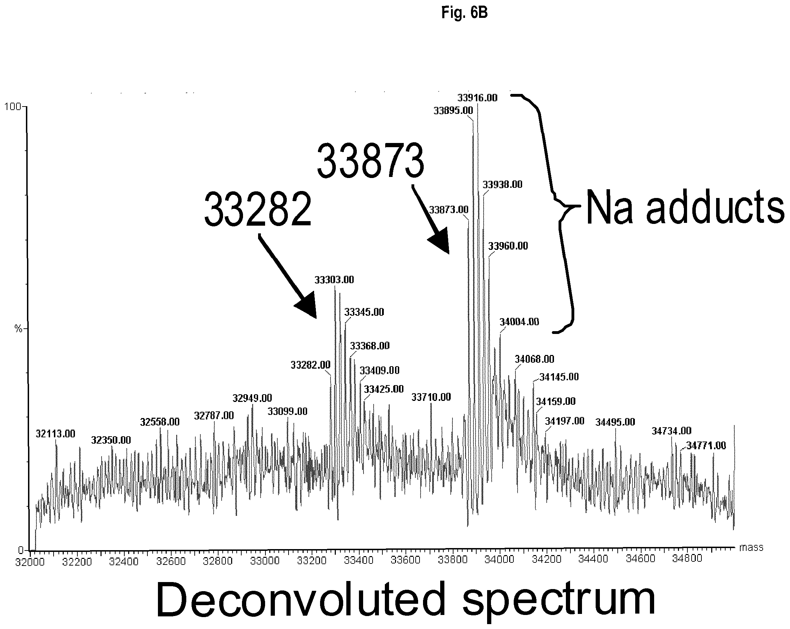

**Fig.6B** shows the deconvoluted MS spectrum of the sample (expected MW 33874 Da; observed MW 33873 Da); traces of the MCM2-linker specie were also detected (Expected MW 33282 Da; observed MW 33282 Da); the molecular weight is reported on the x axis while intensity expressed as counts per second (cps) is reported on the y axis.

### Example (B)

### Preparation of conjugates with cysteine

2 nmol of cysteine (Cys, MW 121 Da) have been reacted with 2 nmol of a functionalized derivative of formula (I), the reaction was incubated for 1 h at 21 °C in presence of borate buffer 50 mM pH 8, DTPA (diethylene triamine pentaacetic acid) 2 mM, NaCl 50 mM, obtaining the final conjugate. The conjugate was then analyzed by HPLC/ESI-MS using a reversed phase HPLC method (PLRP-S column 1000A 8µM 150 X 2.1 mm) on a 1100 Agilent HPLC instrument coupled with an Agilent 1946 single quadrupole mass spectrometry detector with an orthogonal ESI source.

### Example 1

### Synthesis of Comp. (4)

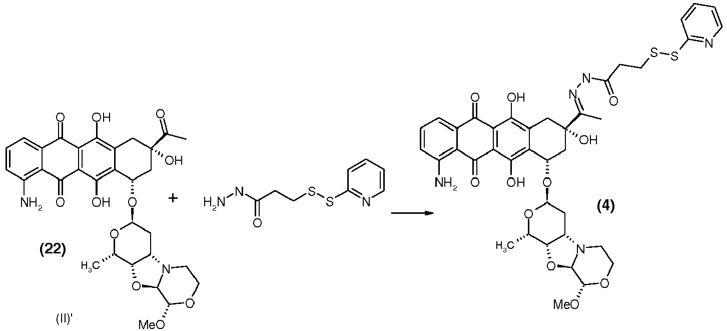

### Step 1

A solution of 3-(2-pyridyldithio)propionic acid hydrazide HCI (41.5 mg,) in anhydrous methanol (5 ml) was added to compound (II)' (Comp. **22,** see **Example 6**) (50 mg). The solution was stirred in the dark at room temperature for 20 hours. The course of the reaction was followed by reverse-phase HPLC-MS. After this period the solvent was evaporated and the residue was purified by flash column chromatography (MeOH:CH₂Cl₂) on silica gel (230-400 mesh), affording compound **(4).**
MS (ESI): 822 [M+H]⁺.

By analogous procedures and using the suitable starting materials, the following compounds were prepared: 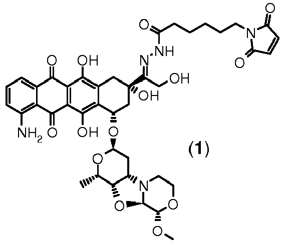 MS (ESI): 838 [M+H]⁺
¹H NMR (500 MHz, CH₃CN-*d*₃, temp 60 °C) 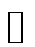 ppm 1.25 - 1.40 (m, 2 H), 1.32 (d, *J*=6.7 Hz, 3 H), 1.49 - 1.66 (m, 4 H), 1.60 - 1.71 (m, 1 H), 1.85 - 1.93 (m, 1 H), 2.22 - 2.33 (m, 1 H), 2.40 - 2.56 (m, 3H), 2.70 - 2.82 (m, 2 H), 3.05 - 3.15 (m, 2 H), 3.40 (s, 3 H), 3.40 - 3.45 (m, 3 H), 3.53 - 3.60 (m, 1 H), 3.75 - 3.83 (m, 1 H), 4.02 (dd, *J*=1.5, 6.8 Hz, 1 H), 4.07 - 4.23 (m, 3 H), 4.21 (br. s., 1 H), 4.32 (d, *J*=2.4 Hz, 1 H), 4.51 (d, *J*=2.4 Hz, 1 H), 5.15 - 5.24 (m, 1 H), 5.39 - 5.41 (m, 1 H), 6.69 (s, 2 H), 7.16 (d, *J*=8.4 Hz, 1 H), 7.22 (br. s., 2 H), 7.54 (dd, *J*=6.7, 8.4 Hz, 1 H), 7.64 (d, *J*=6.7 Hz, 1H), 8.45 (br. s., 1 H), 13.45 (s, 1 H), 13.82 (s, 1 H). 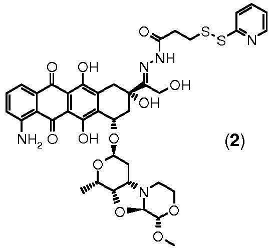 MS (ESI): 834 [M+H]⁺ 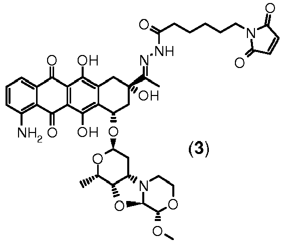 MS (ESI): 818 [M+H]⁺
¹H NMR (500 MHz, ACETONITRILE-*d*₃, temp 60 °C) δ ppm 1.25 - 1.35 (m, 2 H), 1.31 (d, J=6.7 Hz, 3 H), 1.49 - 1.66 (m, 4 H), 1.70 - 1.77 (m, 1 H), 1.85 - 1.93 (m, 1 H), 1.98 (s, 3 H), 2.22 - 2.33 (m, 1 H), 2.35 - 2.56 (m, 3H), 2.70 - 2.82 (m, 2 H), 3.05 - 3.20 (m, 2 H), 3.40 (s, 3 H), 3.40 - 3.48 (m, 3 H), 3.53 - 3.60 (m, 1 H), 3.74 - 3.80 (m, 1 H), 4.03 (dd, *J*=1.5, 6.8 Hz, 1 H), 4.07 - 4.13 (m, 1 H), 4.21 (br. s., 1 H), 4.30 (d, *J*=2.4 Hz, 1 H), 4.56 (d, *J*=2.4 Hz, 1 H), 5.15 - 5.24 (m, 1 H), 5.36 - 5.40 (m, 1 H), 6.69 (s, 2 H), 7.16 (d, *J*=8.4 Hz, 1 H), 7.21 (br. s., 2 H), 7.54 (dd, *J*=6.7, 8.4 Hz, 1 H), 7.64 (d, *J*=6.7 Hz, 1 H), 8.41 (br. s., 1 H), 13.46 (s, 1 H), 13.81 (s, 1 H).

### Example 2a

### Synthesis of Comp. (5)

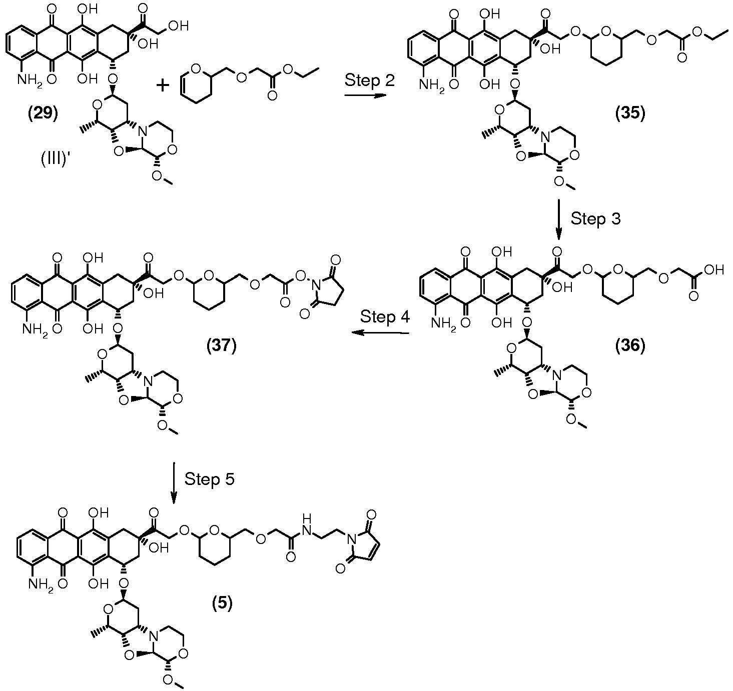

### Step 1

### Synthesis of intermediate: ethyl (3,4-dihydro-2H-pyran-2-ylmethoxy)acetate

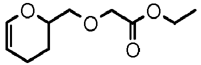

In a dried round bottomed flask under argon atmosphere, 60% sodium hydride (240 mg, 6.0 mmol) was rinsed three times with anhydrous w-pentane. A solution of 2-hydroxymethyl-3,4-dihydro-2H-pyran (570.8 mg, 5 mmol) in tetrahydrofuran (10 ml) was cooled at 0 °C and then added to the NaH. The reaction mixture was stirred at 0 °C until hydrogen evolution ended. A solution of ethyl bromoacetate (1253 mg, 7.5 mmol) in tetrahydrofuran (6 ml) was added to the reaction mixture and the stirring was continued at room temperature until disappearance of the starting alcohol (TLC analysis). After cooling, H₂O was added, and the solvent was evaporated under reduced pressure. The residue was purified by flash column chromatography (AcOEt/hexane = 1: 12) on silica gel (230-400 mesh), affording 626 mg (yield 57%) of ethyl (3,4-dihydro-2H-pyran-2-ylmethoxy)acetate as a colorless oil.
¹H NMR (401 MHz, DMSO-*d*₆) δ ppm 1.18 - 1.23 (m, 3 H) 3.55 - 3.59 (m, 2 H) 3.93 (m, J=10.08, 5.11, 5.11, 2.38 Hz, 1 H) 4.13 (q, J=7.07 Hz, 2 H) 4.14 (s, 2 H) 4.67 (dddd, J=6.14, 4.83, 2.56, 1.34 Hz, 1 H) 6.36 (dt, J=6.13, 1.75 Hz, 1 H).

### Step 2

### Synthesis of the intermediate (35)

To a solution of a compound of formula (III)' (Comp. **29,** see **Example 6**) (50 mg) in 4 ml of dry dichloromethane under argon atmosphere, ethyl (3,4-dihydro-2H-pyran-2-ylmethoxy)acetate from step 1 (118.5 mg,) and anhydrous p-toluenesulfonic acid (22.3 mg, 0.12 mmol) were added. The reaction mixture was stirred at room temperature for 4 hours, until no starting material was detectable (TLC analysis, MeOH:CH₂Cl₂). Sodium bicarbonate 10% aqueous solution was then added to the reaction mixture and the aqueous phase was extracted with dichloromethane (4 x 20 ml). The combined organic phases were dried over anhydrous sodium sulfate, filtered and evaporated under vacuum. The residue was purified by flash column chromatography (MeOH:CH₂Cl₂) on silica gel (230-400 mesh), affording the intermediate **(35)** as a mixture of four diastereoisomers.
MS (ESI): 827 [M+H]⁺.

### Step 3

### Synthesis of the acid intermediate (36)

The ethyl ester intermediate **(35)** obtained from step 2 (40 mg) cooled at 0 °C, was treated with aqueous 0.1 N sodium hydroxide (1.5 ml) under argon. The reaction mixture was stirred at 0 °C for 2 hours. The course of the reaction was followed by reverse-phase HPLC-MS. After that, the reaction mixture was brought to pH ≥ 8 with 10% acetic acid water solution, and extracted with n-butanol saturated with water (8 x 10 ml). The combined organic phases were dried over anhydrous sodium sulfate, filtered, and the solvent removed under vacuum. The residue was purified by flash column chromatography (MeOH:CH₂Cl₂) on silica gel (230-400 mesh), affording **(36)** as a diasteroisomeric mixture.
MS (ESI): 799 [M+H]⁺.

### Step 4

### Synthesis of the intermediate (37)

To a solution of the acid intermediate **(36)** obtained from step 3 of the process (2 mg) in dry dichloromethane (1 ml) cooled at 0 °C, N-hydroxysuccinimide (1 mg) and N,N'-dicyclohexylcarbodiimide (1 mg) were added. The reaction mixture was stirred at 0 °C for 2.5 h, until disappearance of the starting material (HPLC-MS analysis). The solvent evaporated under vacuum and the residue treated with ethyl ether (2 x 4 ml). The suspension was stirred for 10 minutes, the solid removed by filtration and the organic solution concentrated in vacuo, affording 2 mg of intermediate **(37)** as a mixture of diastereoisomers.
MS (ESI): 896 [M+H]⁺.

### Step 5

### The title Comp. (5)

To a solution of **(37)** obtained from step 4 of the process (1 mg) in dry dichloromethane (100 µL), N-(2-aminoethyl)maleimide trifluoroacetate salt (2eq) were added. The solution was stirred at room temperature 4 hours until no starting material was detectable (HPLC-MS analysis), and the solvent evaporated under vacuum. The residue was purified by flash column chromatography (MeOH:CH₂Cl₂) on silica gel (230-400 mesh), affording compound **(5)** as a mixture of diastereoisomers.
MS(ESI): 921 [M+H]⁺

### Example 2b

### Synthesis of Comp. (9)

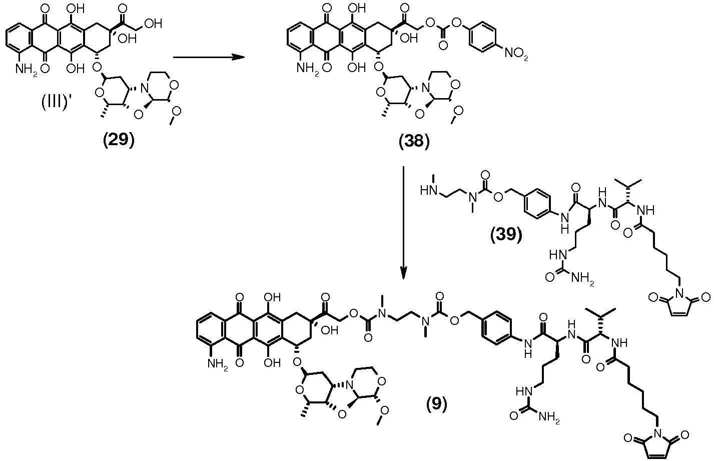

A solution of compound (III)' (Comp. **29,** see **Example 6**) (10 mg), bis(4-nitrophenyl) carbonate (22 mg) and triethylamine (0.021 ml,) in 1 ml of dry DCM and 1 ml of dry THF was stirred under nitrogen atmosphere for 6 hours. Solvents were evaporated and the residue was treated with diethylether. The resulting precipitate **(38)** was filtered, washed with diethylether and dried under vacuum. This compound was dissolved in dry DCM (2 ml, with 10% of dry DMF) and *N*-[6-(2,5-dioxo-2,5-dihydro-1*H*-pyrrol-1-yl)hexanoyl]-L-valyl-N⁵-carbamoyl-N-{4-[({methyl[2-(methylamino)ethyl]carbamoyl}oxy)methyl]phenyl}-L-ornithinamide hydrochloride **(39)** (prepared as reported in **Example 8**) (30 mg, 0.04 mmol) and triethylamine (0.016 ml, 0.11 mmol) were added. The reaction mixture was stirred overnight under nitrogen atmosphere, solvents were evaporated and the title compound (**9**) was obtained after column chromatography purification (DCM / MeOH).
MS(ESI): 1339 [M+H]⁺

By analogous procedures and using the suitable starting materials the following compounds were prepared: 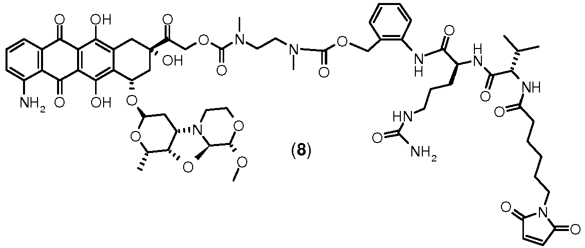 MS(ESI): 1339 [M+H]⁺ 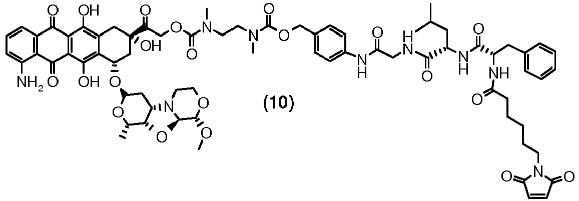 MS(ESI): 1400 [M+H]⁺

### Example 3

### Preparation of Comp. (18)

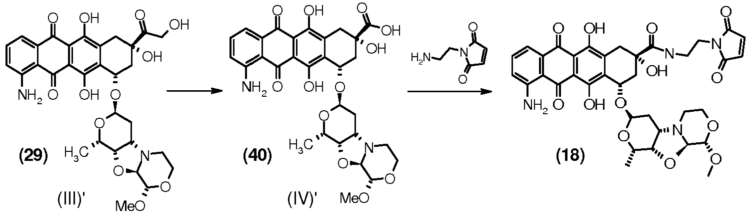

### Step 1

### Preparation of intermediate (40)

To a solution of compound (III)' (Comp. **29,** see **Example 6**) in 3 ml of methanol and 2 ml of H₂O, a solution of NaIO₄ (5.1 mg, 0.0238 mmol) in 1 ml of H₂O was added. The reaction mixture was stirred at room temperature for 3 hours, until no starting material was detectable (TLC and HPLC analysis). The solvents were removed under reduced pressure and the crude acid **(40)** was used without further purifications in the next step.
MS (ESI): 613 [M+H]⁺

### Step 2

### The title Comp. (18)

To a solution of the crude intermediate **(40)** (4.4 mg) in anhydrous dichloromethane (1.5 ml) under argon atmosphere, anhydrous triethylamine (2.2 mg), TBTU (4.4 mg) and commercially available N-(2-aminoethyl)maleimide trifluoroacetate salt (3.6 mg) were added. The reaction mixture was stirred at room temperature for 30 min, until disappearance of the starting material (HPLC-MS analysis). The solvent was evaporated under vacuum and the residue was then purified by flash column chromatography (EtOH:CH₂Cl₂) on silica gel (230-400 mesh), affording the title compound **(18).**
MS (ESI): 735 [M+H]⁺.

By analogous procedures and using the suitable starting materials the following compounds were prepared: 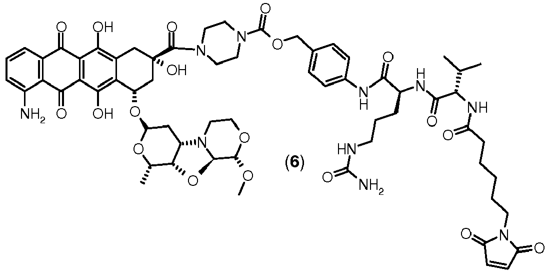 MS (ESI): 1279 [M+H]⁺ 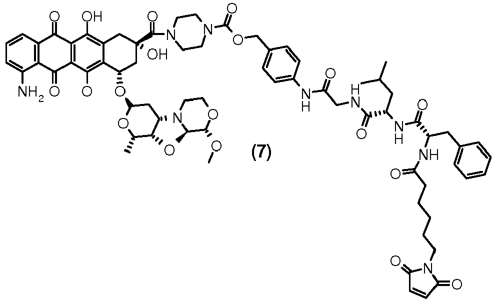 MS (ESI): 1340 [M+H]⁺. 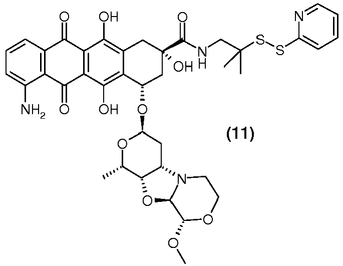 MS (ESI): 809 [M+H]⁺.

### Example 4a

### Synthesis of the Comp. (12)

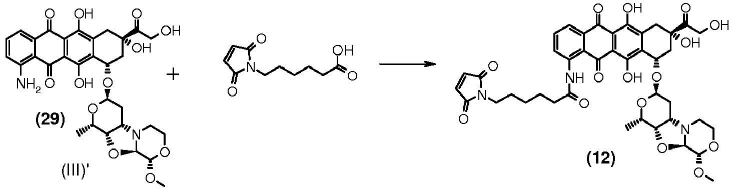

To a solution of the crude intermediate (III)' (Comp. **29,** see **Example 6,** 4.4 mg) in anhydrous dichloromethane (1.5 ml) under argon atmosphere, anhydrous triethylamine (1.5 mg), TBTU (2.7 mg) and commercially available 1-{6-[(2,5-dioxopyrrolidin-1-yl)oxy]-6-oxohexyl}-1*H*-pyrrole-2,5-dione (1.8 mg) were added. The reaction mixture was stirred at room temperature for 30 min, until disappearance of the starting material (HPLC-MS analysis). The solvent was evaporated under vacuum and the residue was then purified by flash column chromatography (EtOH:CH₂Cl₂) on silica gel (230-400 mesh), affording the title compound **(12).**
MS (ESI): 820 [M+H]⁺.

By analogous procedures and using the suitable starting materials the following compounds were prepared: 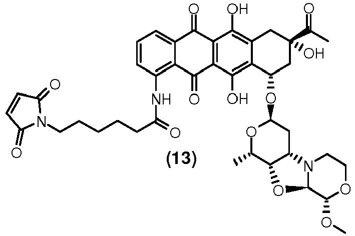 MS (ESI): 804 [M+H]⁺. 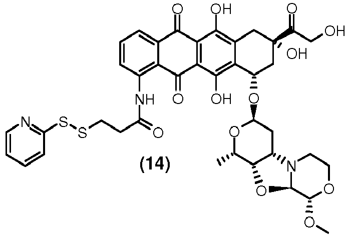 MS (ESI): 824 [M+H]⁺. 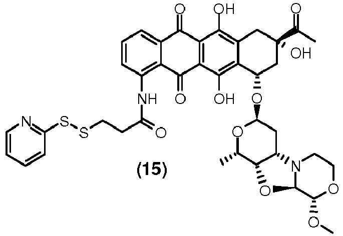 MS (ESI): 808 [M+H]⁺. 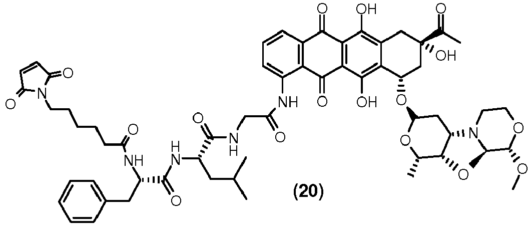 MS (ESI): 1121 [M+H]⁺.

### Example 4b

### Synthesis of the Comp. (16)

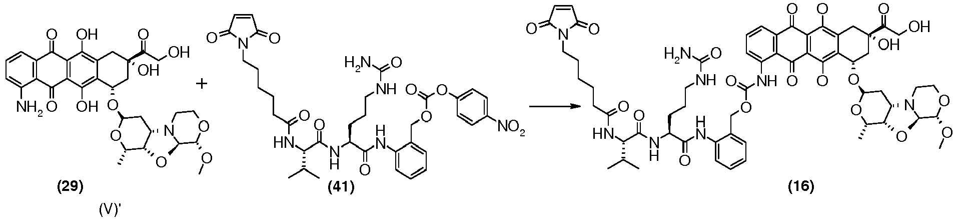

A solution of compound (V)'(Comp. **29,** see **Example 6**, 10 mg) was dissolved in dry DCM (2 ml), with 10% of dry DMF) and *N*-[6-(2,5-dioxo-2,5-dihydro-1*H*-pyrrol-1-yl)hexanoyl]-L-valyl-*N*⁵-carbamoyl-*N*-[2-({[(4-nitrophenoxy) carbonyl]oxy}methyl)phenyl]-L-ornithinamide **(41)** (prepared as reported in EP0624377A2 and EP2357006A2, 15 mg) and triethylamine (0.0033 ml) were added. The reaction mixture was stirred overnight under nitrogen atmosphere, solvents were evaporated and the title compound **(16)** was obtained after column chromatography purification (DCM / MeOH).
MS (ESI): 1225 [M+H]⁺.

By analogous procedures and using the suitable starting materials the following compounds were prepared: 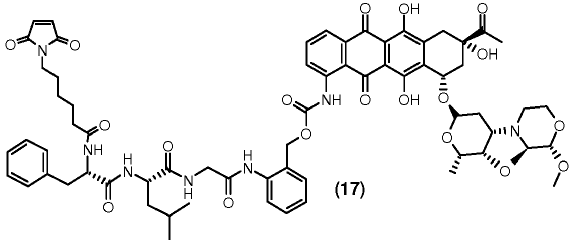 MS (ESI): 1270 [M+H]⁺. 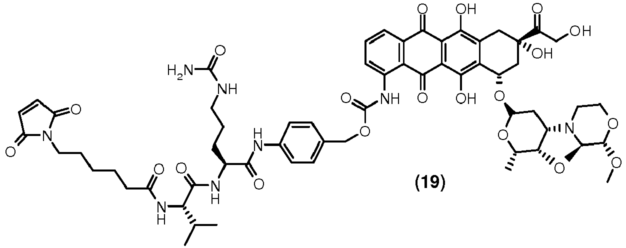 MS (ESI): 1225 [M+H]⁺. 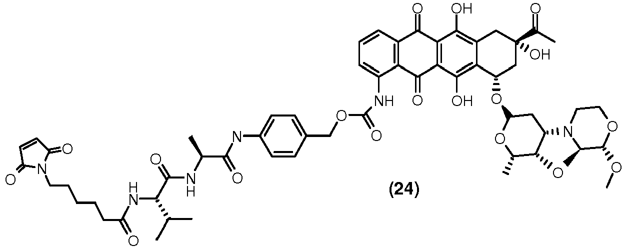 MS (ESI): 1123 [M+H]⁺ 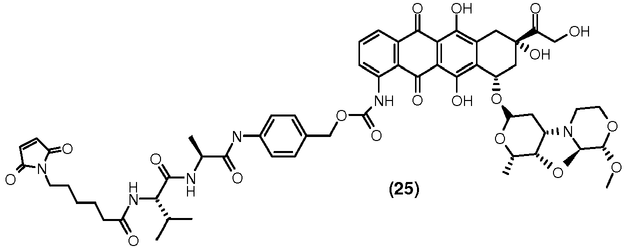 MS (ESI): 1139 [M+H]⁺

### Example 4b

### Synthesis of the Comp. (23)

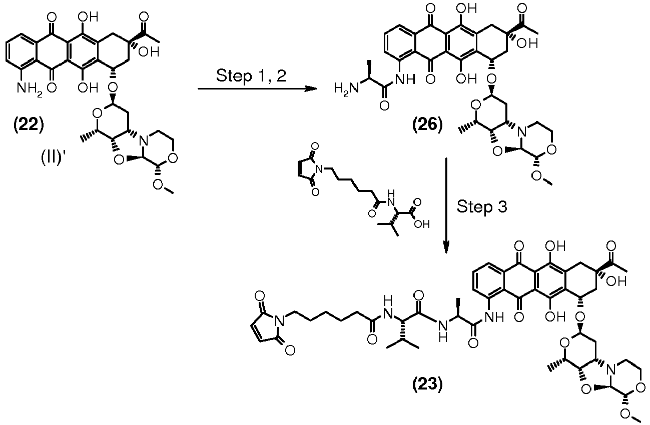

### Step 1

### Synthesis of the intermediate: 9H-fluoren-9-ylmethyl [(2S)-1-{[(8S,10S)-8-acetyl-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-5,12-dioxo-5,7,8,9,10,12-hexahydrotetracen-1-yl]amino}-1-oxopropan-2-yl]carbamate (Comp. 42)

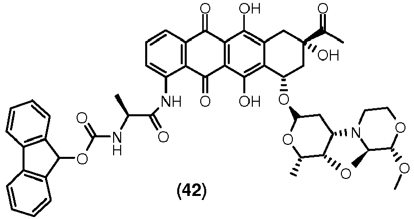

In a dried round-bottomed flask, a solution of Fmoc-L-Alanine (50 mg, 1.6 mmol) in 9 ml of dry dichloromethane was treated with SOCl₂ (190 mg g, 1.61 mmol) and the mixture refluxed for 2 hours under argon atmosphere. The solvent and the excess of SOCl₂ were removed under vacuum to yield the crude intermediate 9H-fluoren-9-ylmethyl [(2S)-1-chloro-1-oxopropan-2-yl]carbamate.

To a solution of a compound (II)' (Comp. **22,** see **Example 6**) (10 mg, 0.0164 mmol) in 5 ml of dry dichloromethane under argon atmosphere, 9H-fluoren-9-ylmethyl [(2S)-1-chloro-1-oxopropan-2-yl]carbamate (30 mg, 0.091 mmol) and 5 ml of saturated NaHCO₃ aqueous solution were added. The reaction mixture was stirred at room temperature for 4 hours, until no starting material was detectable (TLC analysis, AcOEt/toluene). The organic phase was separated, washed with water and dried over anhydrous Na₂SO₄. The solvent was evaporated under vacuum and the residue was purified by flash chromatography (eluant: AcOEt/toluene = 4/6) on silica gel (230-400 mesh) affording compound **(42)** (7.2 mg, red wax).
MS (ESI): 904 [M+H]⁺.

### Step 2

### Synthesis of the Comp. (26)

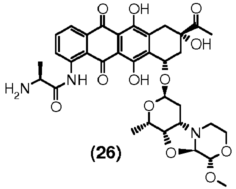

The intermediate 9H-fluoren-9-ylmethyl [(2*S*)-1-{[(8*S*,10*S*)-8-acetyl-6,8,11-trihydroxy-10-{[(1*S*,3*R*,4a*S*,9*S*,9a*R*,10a*S*)-9-methoxy-1-methyloctahydro-1*H*-pyrano[4',3':4,5][1,3]oxazolo[2,3-*c*][1,4]oxazin-3-yl]oxy}-5,12-dioxo-5,7,8,9,10,12-hexahydrotetracen-1-yl]amino}-1-oxopropan-2-yl]carbamate **(42)** obtained from step 1 (7.2 mg, 0.008) was dissolved in dry dichloromethane (1.5 ml) and treated with piperidine (20.4 mg, 0.24 mmol). The reaction mixture was stirred at room temperature for 4 hours until disappearance of the starting material (HPLC-MS analysis). The reaction mixture was diluted with dichloromethane and washed with water. The organic phases were dried over anhydrous sodium sulfate, and the solvent evaporated under vacuum. The residue was purified by flash column chromatography (eluant: AcOEt/toluene = 4/6; then EtOH/CH₂Cl₂ = 1/1) on silica gel (230-400 mesh), affording the compound **(26)** (2.5 mg, red wax).
MS (ESI): 682 [M+H]⁺.
¹H NMR (401 MHz, CH₃CN-*d*₃) 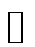 ppm 1.29 (d, *J*=6.7 Hz, 3 H), 1.38 (d, *J*=6.8 Hz, 3 H), 1.64 - 1.74 (m, 1 H), 1.86 - 1.91 (m, 1 H), 2.02 - 2.07 (m, 1 H), 2.35 (s, 3 H), 2.38 - 2.48 (m, 1 H), 2.68 - 2.81 (m, 2 H), 2.93 - 3.14 (m, 2 H), 3.38 (s, 3 H), 3.44 - 3.48 (m, 1 H), 3.54 - 3.60 (m, 1 H), 3.64 (q, *J*=6.8 Hz, 1 H), 3.71 - 3.79 (m, 1 H), 4.01 - 4.06 (m, 1 H), 4.07 - 4.13 (m, 1 H), 4.27 (d, *J*=2.7 Hz, 1 H), 4.55 (d, *J*=2.7 Hz, 1 H), 5.19 - 5.24 (m, 1 H), 5.35 - 5.40 (m, 1 H), 7.86 (dd, *J*=7.8, 8.5 Hz, 1 H), 8.10 (d, *J*=7.8 Hz, 1 H), 9.23 (d, *J*=8.5 Hz, 1 H).

By analogous procedures and using the suitable starting materials the following compound was prepared:

### N-[(8S,10S)-8-acetyl-6,8,11-trihydroxy-10-{[(1S,3R,4aS,9S,9aR,10aS)-9-methoxy-1-methyloctahydro-1H-pyrano [4',3':4,5][1,3]oxazolo[2,3-c][1,4]oxazin-3-yl]oxy}-5,12-dioxo-5,7,8,9,10,12-hexahydrotetracen-1-yl]glycinamide (Comp. 75)

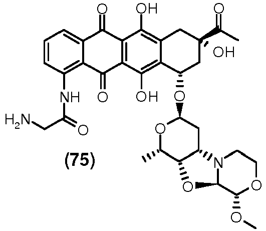

MS (ESI): 668 [M+H]⁺.

### Step 3

### The title comp. (23)

To a solution of comp. **(26)** (1.8 mg, 0.00264 mmol) obtained from step 3 in dry dichloromethane (3 ml), *N*-[6-(2,5-dioxo-2,5-dihydro-1*H*-pyrrol-1-yl)hexanoyl]-L-valine (4.9 mg, 0.016 mmol) and *O*-(Benzotriazol-1-yl)-*N,N,N',N'-*tetramethyluronium tetrafluoroborate (5.1 mg, 0.016 mmol) were added. The reaction mixture was stirred at room temperature for 4 hours until disappearance of the starting material (HPLC-MS analysis) The reaction mixture was stirred at room temperature for 20 hours until disappearance of the starting material (HPLC-MS analysis).

The reaction mixture was diluted with dichloromethane and washed with water. The organic phases were dried over anhydrous sodium sulfate, and the solvent evaporated under vacuum. The residue was purified by flash column chromatography (eluant: AcOEt/toluene = 4/6) on silica gel (230-400 mesh), affording compound **(23)** (1.9 mg, red wax).
MS (ESI): 974 [M+H]⁺.

By analogous procedures and using the suitable starting materials the following compounds were prepared:

### N-[6-(2,5-dioxo-2,5-dihydro-1H-pyrrol-1-yl)hexanoyl]-L-valyl-N⁵-carbamoyl-L-ornithyl-N-[(8S,10S)-8-acetyl-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4] oxazin-3-yl]oxy}-5,12-dioxo-5,7,8,9,10,12-hexahydrotetracen-1-yl]-L-alaninamide (Comp. 27)

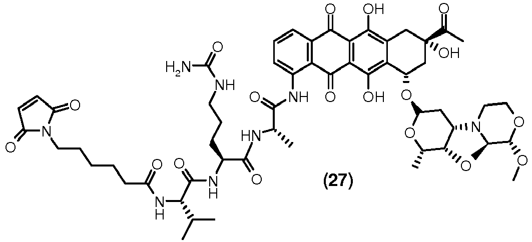

MS (ESI): 1131 [M+H]⁺.

### N-[6-(2,5-dioxo-2,5-dihydro-1H-pyrrol-1-yl)hexanoyl]-L-valyl-N⁵-carbamoyl-L-ornithyl-N-[(8S,10S)-8-acetyl-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4] oxazin-3-yl]oxyl-5,12-dioxo-5,7,8,9,10,12-hexahydrotetracen-1-yl]glycinamide (Comp. 76)

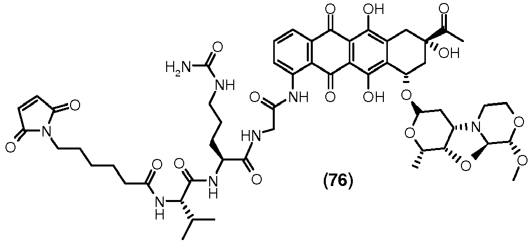

MS (ESI): 1117 [M+H]⁺.

### N-[6-(2,5-dioxo-2,5-dihydro-1H-pyrrol-1-yl)hexanoyl]-L-valyl-N-[4-({[(2-{[(8S,10S)-8-acetyl-6,8,11-trihydroxy-10-{[(3S,4aS,9S,9aR,10aS)-9-methoxyoctahydro-1H-pyrano[4',3':4,5][1,3]oxazolo[2,3-c][1,4]oxazin-3-yl]oxy}-5,12-dioxo-5,7,8,9,10, 12-hexahydrotetracen-1-yl]amino}-2-oxoethyl)carbamoyl]oxy}methyl)phenyl]-N⁵-carbamoyl-L-ornithinamide (Comp. 77)

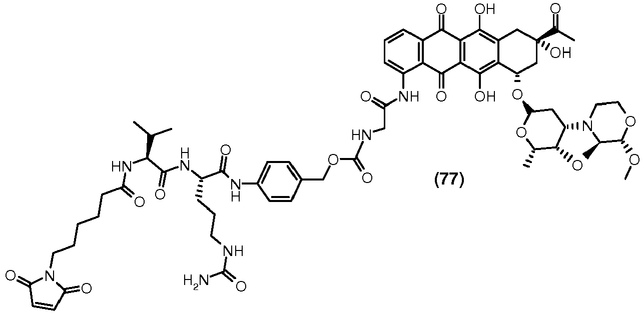

MS (ESI): 1266 [M+H]⁺.

### N-[6-(2,5-dioxo-2,5-dihydro-1H-pyrrol-1-yl)hexanoyl]-L-valyl-N-{4-[({[(2S)-1-{[(8S,10S)-8-acetyl-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-5,12-dioxo-5,7,8,9,10,12-hexahydrotetracen-1-yl]amino}-1-oxopropan-2-yl]carbamoyl}oxy)methyl]phenyl}-N⁵-carbamoyl-L-ornithinamide (Comp. 78)

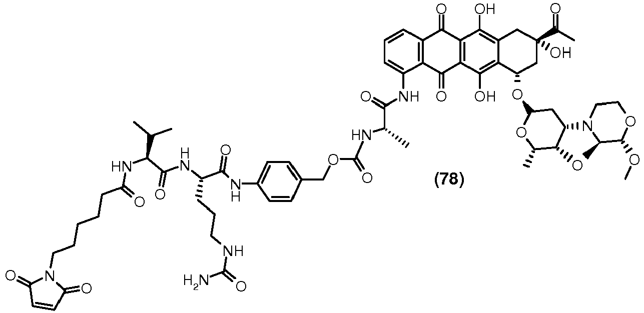

MS (ESI): 1280 [M+H]⁺.

### Example 5

### Step A1, Step B1 (according to A6a and A6b)

### (8S,10S)-8-acetyl-1-fluoro-6,8,11-trihydroxy-10-{[(1S,3R,4aS,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 21)

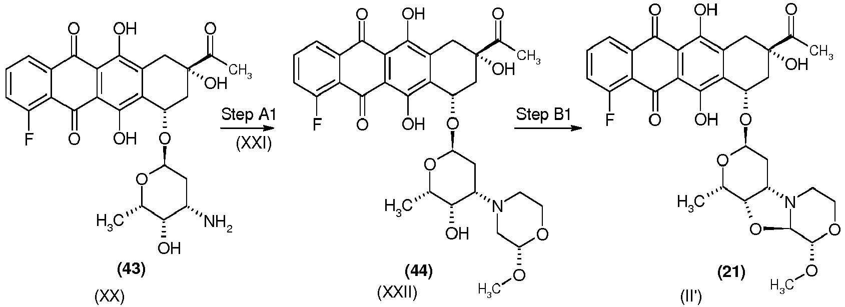

### Step A1

### (1S,3S)-3-acetyl-10-fluoro-3,5,12-trihydroxy-6,11-dioxo-1,2,3,4,6,11-hexahydrotetracen-1-yl 2,3,6-trideoxy-3-[(2S)-2-methoxymorpholin-4-yl]-α-L-lyxo-hexopyranoside [(XXII)] (Comp. 44)

(1*S*,3*S*)-3-acetyl-10-fluoro-3,5,12-trihydroxy-6,11-dioxo-1,2,3,4,6,11-hexahydrotetracen-1-yl 3-amino-2,3,6-trideoxy-α-L-*lyxo*-hexopyranoside (70.0 mg, 0.136 mmol) [prepared as reported in WO90/09392] was dissolved in dry DMF (3 ml); a solution of di*i*sopropylethylamine (106 mg, 0.82 mmol) in dry DMF (2 ml) and a solution of (1S)-2-iodo-1-(2-iodoethoxy)-1-methoxyethane (XXI) (965 mg, 2.71 mmol) in dry DMF (10 ml) were added. The reaction mixture was stirred at room temperature in the dark for 48 hours, until no starting material was detectable (HPLC analysis). The reaction mixture was then diluted with DCM and washed with water. The organic phase was dried over anhydrous Na₂SO₄, the solvent was evaporated under vacuum and the residue was purified by flash chromatography (eluant: EtOH/DCM; 0.2/9.8) on silica gel (230-400 mesh) affording the desired product **(44)** (35 mg, red wax).
MS (ESI): 616 [M+H]⁺.
¹H NMR (500 MHz, CHCl₃-*d*) δ ppm 1.39 (d, *J*=6.71 Hz, 3 H) 1.78 - 1.85 (m, 2 H) 2.09 - 2.14 (m, 1 H) 2.46 - 2.56 (m, 3 H) 2.61 (dd, *J*=11.41, 3.97 Hz, 1 H) 3.03 (d, *J*=19.04 Hz, 1 H) 3.27 (dd, *J*=19.10, 1.77 Hz, 1 H) 3.40 (s, 3 H) 3.57 (ddd, *J*=11.57, 5.34, 3.11 Hz, 1 H) 3.70 (s, 1 H) 3.92 - 3.99 (m, 1 H) 4.04 (q, *J*=6.47 Hz, 1 H) 4.48 - 4.52 (m, 1 H) 4.67 (s, 1 H) 5.28 - 5.30 (m, 1 H) 5.56 (br. s., 1 H) 7.54 (dd, *J*=10.44, 8.48 Hz, 1 H) 7.83 (td, *J*=7.97, 4.58 Hz, 1 H) 8.25 (d, *J*=7.69 Hz, 1 H) 13.31 (s, 1 H) 13.72 (s, 1 H)

By analogous procedure the following compound is prepared:

### (1S,3S)-10-fluoro-3,5,12-trihydroxy-3-(hydroxyacetyl)-6,11-dioxo-1,2,3,4,6,11-hexahydrotetracen-1-yl 2,3,6-trideoxy-3-[(2S)-2-methoxymorpholin-4-yl]-α-L-lyxo-hexopyranoside [(XXII)] (Comp. 45)

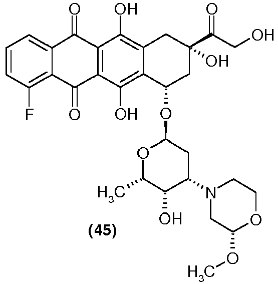

MS (ESI): 632 [M+H]⁺.

### Step B1 (A6a)

### (1S,3S)-3-acetyl-10-fluoro-3,5,12-trihydroxy-6,11-dioxo-1,2,3,4,6,11-hexahydrotetracen-1-yl (3ξ)-2,3,6-trideoxy-3-[(2S)-2-methoxy-4-oxidomorpholin-4-yl]-α-L-threo-hexopyranoside [(XXII)] (Comp. 46)

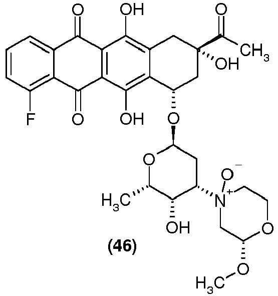

(1S,3S)-3-acetyl-10-fluoro-3,5,12-trihydroxy-6,11-dioxo-1,2,3,4,6,11-hexahydrotetracen-1-yl 2,3,6-trideoxy-3-[(2*S*)-2-methoxymorpholin-4-yl]-α-L-*lyxo*-hexopyranoside (28 mg, 0.045 mmol) [prepared as reported in step A1] was dissolved in DCM (3.0 ml). The solution was treated with a 0.1 M solution of DMDO in acetone (0.8 ml) at room temperature for 30 minutes, until no starting material was detectable (HPLC analysis). The reaction mixture was then concentrated to dryness under vacuum, affording the desired intermediate **(46)** (red wax, 24.1 mg).
MS (ESI): 632 [M+H]⁺.
¹H NMR (500 MHz, CH₃CN-*d*₃) δ ppm 1.23 (d, *J*=6.7 Hz, 3 H) 1.96 - 2.00 (m, 1 H) 2.10 (m, 1 H) 2.35 (s, 3H) 2.33 - 2.38 (m, 1 H) 2.56 - 2.64 (m, 2 H) 2.94 - 3.00 (m, 1 H) 3.07 - 3.12 (m, 1 H) 3.13 -3.16 (m, 1 H) 3.23 - 3.29 (m, 1 H) 3.37 (s, 3 H) 3.38 - 3.46 (m, 2 H) 3.86 - 3.95 (m, 1 H) 3.99 (q, *J*=6.7 Hz, 1 H) 4.14 (s, 1 H) 4.22 - 4.29 (m, 1 H) 4.32 (br. s., 1 H) 4.91 (dd, *J*=8.1, 2.3 Hz, 1 H) 5.20 (dd, *J*=4.6, 1.9 Hz, 1 H) 5.60 (d, *J*=3.9 Hz, 1 H) 7.62 (dd, *J*_{HH}=8.3, *J*_{HF}=10.8 Hz, 1 H) 7.91 (m, 1 H) 8.20 (d, *J*_{HH}=7.7 Hz, 1 H) 13.26 (br. s., 1 H) 13.69 (br. s., 1 H)

By analogous procedure the following compound is prepared:

### (1S,3S)-10-fluoro-3,5,12-trihydroxy-3-(hydroxyacetyl)-6,11-dioxo-1,2,3,4,6,11-hexahydrotetracen-1-yl (3ξ)-2,3,6-trideoxy-3-[(2S)-2-methoxy-4-oxidomorpholin-4-yl]-α-L-threo-hexopyranoside [(XXII)] (Comp. 47)

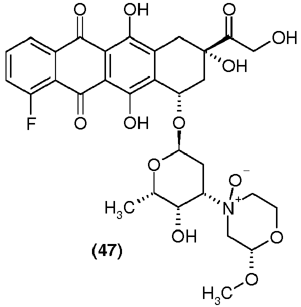

MS (ESI): 648 [M+H]⁺.

### Step B1 (A6b)

### The title Comp. (21)

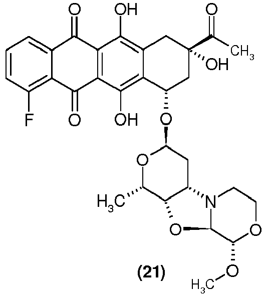

To a solution of compound (1*S*,3*S*)-3-acetyl-10-fluoro-3,5,12-trihydroxy-6,11-dioxo-1,2,3,4,6,11-hexahydrotetracen-1-yl (3ξ)-2,3,6-trideoxy-3-[(2*S*)-2-methoxy-4-oxidomorphoiin-4-yl]-α-L-*threo*-hexopyranoside [(XXII)] (20 mg, 0.032 mmol) in 5.0 ml of dry CH₃CN, K₂CO₃ (13.2 mg, 0.096 mmol) and cyanuric chloride (11.8 mg, 0.064 mmol) were added. The reaction mixture was vigorously stirred in the dark at room temperature for 20 minutes, until no starting material was detectable. A solution of 3-amino-1,2-propanediol (17.5 mg, 0.192 mmol) in water (0.84 ml) was then added to the reaction mixture and the aqueous phase was extracted with DCM (4 x 10 ml). The combined organic phases were dried over anhydrous Na₂SO₄, filtered and evaporated under vacuum. The crude was purified by flash column chromatography (AcOEt/toluene; 4/6) on silica gel (230-400 mesh), affording 7.0 mg of title compound **(21)** as red solid.
MS (ESI): m/z 614 [M+H]⁺.
¹H NMR (500 MHz, CH₃CN-*d*₃) δ ppm 1.29 (d, *J*=6.6 Hz, 3 H) 1.68 - 1.73 (m, 1 H) 1.86 - 1.91 (m, 1 H) 2.05 (dd, *J*=14.8, 4.3 Hz, 1 H) 2.34 (s, 3 H) 2.42 - 2.47 (m, 1 H) 2.67 - 2.81 (m, 2 H) 2.93 - 2.98 (m, 1 H) 3.05 - 3.11 (m, 1 H) 3.37 (s, 3 H) 3.42 - 3.47 (m, 1 H) 3.52 - 3.58 (m, 1 H) 3.71 - 3.76 (m, 1 H) 4.03 (dd, *J*=7.1, 1.8 Hz, 1 H) 4.06 - 4.12 (m, 1 H) 4.26 (d, *J*=2.8 Hz, 1 H) 4.53 (d, *J*=2.8 Hz, 1 H) 4.54 (s, 1 H) 5.20 (dd, *J*=4.3, 2.1 Hz, 1 H) 5.35 (t, *J*=5.5 Hz, 1 H) 7.60 (dd, *J*_{HH}=8.3, *J*_{HF}=11.6 Hz, 1 H) 7.89 (m, 1 H) 8.19 (dd, *J*_{HH}=7.7, *J*_{HF}=0.8 Hz, 1 H) 13.25 (br. s., 1 H) 13.61 (br. s., 1 H)

Analogously, by using the suitable starting material, the following compounds are prepared:

### (8S,10S)-1-fluoro-6,8,11-trihydroxy-8-(hydroxyacetyl)-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxyl-7,8,9,10-tetrahydrotetracene-5,12-dione (Comp. 28)

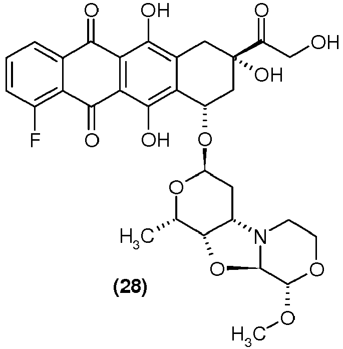

MS (ESI): 630 [M+H]⁺.

### (8S,10S)-8-acetyl-6,8,11-trihydroxy-1-[(2-hydroxyethyl)amino]-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 30)

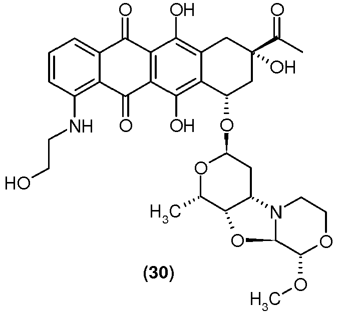

MS (ESI): 655 [M+H]⁺.

### (8S,10S)-6,8,11-trihydroxy-8-(hydroxyacetyl)-1-[(2-hydroxyethyl)amino]-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 31)

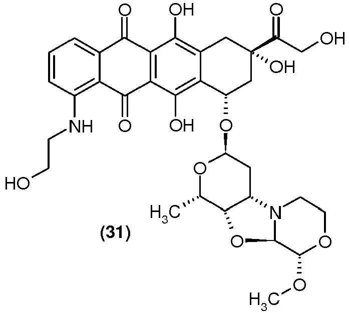

MS (ESI): 671 [M+H]⁺.

### (8S,10S)-8-acetyl-1-[(2-aminoethyl)amino]-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 32)

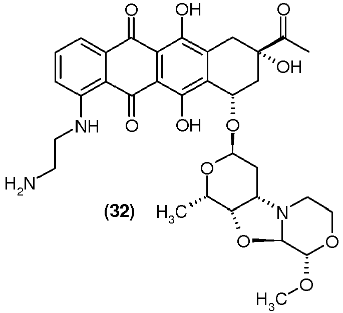

MS (ESI): 654 [M+H]⁺.

### (8S,10S)-1-[(2-aminoethyl)amino]-6,8,11-trihydroxy-8-(hydroxyacetyl)-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 33)

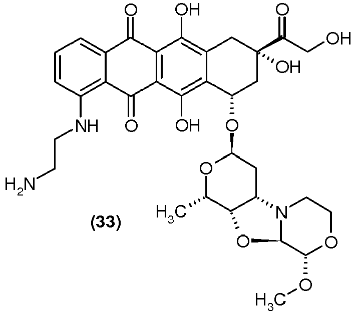

MS (ESI): 670 [M+H]⁺.

### Example 6

### Step A1, Step B1 (according to A6a and A6b)

### (8S,10S)-8-acetyl-1-amino-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione [(II)'] (Comp. 22)

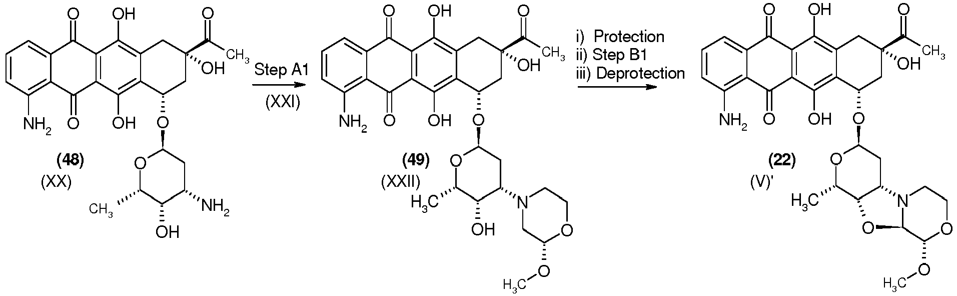

### Step A1

### (1S,3S)-3-acetyl-10-amino-3,5,12-trihydroxy-6,11-dioxo-1,2,3,4,6,11-hexahydrotetracen-1-yl 2,3,6-trideoxy-3-[(2S)-2-methoxymorpholin-4-yl]-α-L-lyxo-hexopyranoside [(XXII)] (Comp. 49)

(1*S*,3*S*)-3-acetyl-10-amino-3,5,12-trihydroxy-6,11-dioxo-1,2,3,4,6,11-hexahydrotetracen-1-yl 3-amino-2,3,6-trideoxy-α-L-*lyxo*-hexopyranoside (165.0 mg, 0.322 mmol) (Comp. **48**, prepared as reported in **Example 7**) was dissolved in dry DMF (3.0 mL); a solution of diisopropylethylamine (221 mg, 1.71 mmol) in dry DMF (3 ml) and a solution of (1S)-2-iodo-1-(2-iodoethoxy)-1-methoxyethane (XXI) (2.0 g, 5.64 mmol) in dry DMF (10 ml) were added. The reaction mixture was stirred at room temperature in the dark for 48 hours, until no starting material was detectable (HPLC analysis). The reaction mixture was then diluted with DCM and washed with water. The organic phase was dried over anhydrous Na₂SO₄, the solvent was evaporated under vacuum and the residue was purified by flash chromatography (eluant: EtOH/DCM; 0.2/9.8) on silica gel (230-400 mesh) affording the desired product **(49)** (105 mg, red solid).
MS (ESI): 613 [M+H]⁺.
¹H NMR (500 MHz, CH₃CN-*d*₃) δ ppm 1.22 - 1.28 (m, 3 H) 1.65 - 1.83 (m, 2 H) 2.30 - 2.36 (m, 4 H) 2.40 (dd, *J*=11.22, 4.94 Hz, 3 H) 2.46 - 2.54 (m, 2 H) 2.87 - 2.96 (m, 1 H) 3.04 - 3.11 (m, 1 H) 3.32 (s, 3 H) 3.50 (ddd, *J*=11.34, 6.51, 2.83 Hz, 1 H) 3.65 (br. s., 1 H) 3.77 - 3.89 (m, 1 H) 4.04 (d, *J*=6.51 Hz, 1 H) 4.44 (dd, *J*=4.63, 2.41 Hz, 1 H) 5.16 (d, *J*=1.99 Hz, 1 H) 5.43 - 5.47 (m, 1 H) 7.12 - 7.16 (m, 1 H) 7.24 (br. s., 1 H) 7.50 - 7.55 (m, 1 H) 7.58 - 7.61 (m, 1 H)

By analogous procedure the following compounds is prepared:

### (1S,3S)-10-amino-3,5,12-trihydroxy-3-(hydroxyacetyl)-6,11-dioxo-1,2,3,4,6,11-hexahydrotetracen-1-yl 2,3,6-trideoxy-3-[(2S)-2-methoxymorpholin-4-yl]-α-L-lyxo-hexopyranoside (Comp. 50)

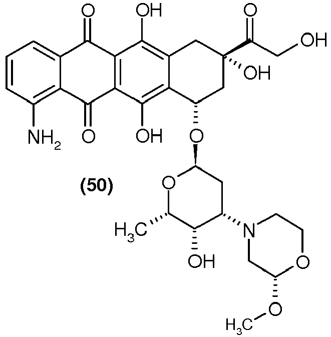

MS (ESI): 629 [M+H]⁺.

### Protection

### N-[(8S,10S)-8-acetyl-6,8,11-trihydroxy-5,12-dioxo-10-({2,3,6-trideoxy-3-[(2S)-2-methoxymorpholin-4-yl]-α-L-lyxo-hexopyranosyl}oxy)-5,7,8,9,10,12-hexahydrotetracen-1-yl]-2,2,2-trifluoroacetamide (Comp. 51)

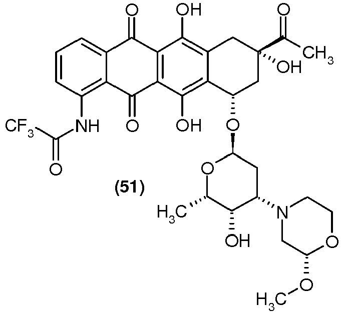

(1*S*,3*S*)-3-acetyl-10-amino-3,5,12-trihydroxy-6,11-dioxo-1,2,3,4,6,11-hexahydrotetracen-1-yl 2,3,6-trideoxy-3-[(2S)-2-methoxymorpholin-4-yl]-α-L-*lyxo*-hexopyranoside **(49)** (80.0 mg, 0.130 mmol) was dissolved in dry DCM (11 ml) and trifluoroacetic anhydride (273.0 mg, 1.3 mmol) was added. The reaction mixture was stirred at room temperature in the dark for 30 minutes, until no starting material was detectable (HPLC analysis). The reaction mixture was then diluted with DCM and washed with saturated NaHCO₃ aqueous solution (3 X 10 ml), and then with water (1 X 10 ml). The organic phase was dried over anhydrous Na₂SO₄, the solvent was evaporated under vacuum and the residue thus obtained was treated with MeOH (10 ml) at room temperature for 15 minutes, and then evaporated under vacuum affording the desired product **(51)** (77.0 mg, red wax).
MS (ESI): 709 [M+H]⁺.
¹H NMR (500 MHz, CH₃CN-*d*₃) δ ppm 1.25 (d, *J*=6.59 Hz, 3 H) 1.76 (dd, *J*=8.68, 2.61 Hz, 2 H) 2.27 - 2.45 (m, 8 H) 2.52 (t, *J*=10.99 Hz, 2 H) 2.96 - 3.03 (m, 1 H) 3.08 - 3.15 (m, 1 H) 3.30 - 3.33 (m, 3 H) 3.50 (ddd, *J*=11.27, 6.57, 2.61 Hz, 1 H) 3.66 (br. s., 1 H) 3.79 - 3.87 (m, 1 H) 4.05 (q, *J*=6.62 Hz, 1 H) 4.44 (dd, *J*=4.70, 2.35 Hz, 1 H) 5.17 (d, *J*=2.27 Hz, 1 H) 5.45 (s, 1 H) 7.97 (t, *J*=8.15 Hz, 1 H) 8.24 (d, *J*=7.50 Hz, 1 H) 8.99 (d, *J*=8.18 Hz, 1 H)

### Step B1 (A6a)

### N-[(8S,10S)-8-acetyl-6,8,11-trihydroxy-5,12-dioxo-10-({(3ξ)-2,3,6-trideoxy-3-[(2S)-2-methoxy-4-oxidomorpholin-4-yl]-α-L-threo-hexopyranosyl}oxy)-5,7,8,9,10,12-hexahydrotetracen-1-yl]-2,2,2-trifluoroacetamide [(XXII)] (Comp. 52)

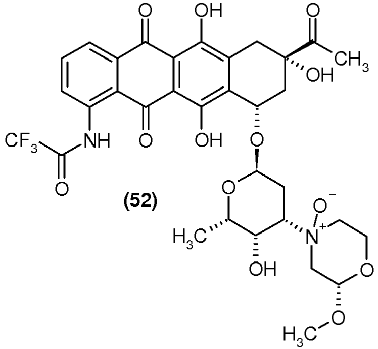

*N*-[(8*S*,10*S*)-8-acetyl-6,8,11-trihydroxy-5,12-dioxo-10-({2,3,6-trideoxy-3-[(2*S*)-2-methoxymorpholin-4-yl]-α-L-*lyxo-*hexopyranosyl}oxy)-5,7,8,9,10,12-hexahydrotetracen-1-yl]-2,2,2-trifluoroacetamide **(51)** (72.0 mg, 0.102 mmol) was dissolved in DCM (6.4 ml). The solution was treated with a 0.1 M solution of DMDO in acetone (1.7 ml) at room temperature for 30 minutes, until no starting material was detectable (HPLC analysis). The reaction mixture was then concentrated to dryness under vacuum, affording the desired intermediate **(52)** (red wax, 73.0 mg).
MS (ESI): 725 [M+H]⁺.
¹H NMR (500 MHz, CH₃CN-*d*₃) δ ppm 1.23 (d, *J*=6.51 Hz, 3 H) 2.32 - 2.39 (m, 4 H) 2.57 (d, *J*=4.54 Hz, 1 H) 2.74 (d, *J*=11.65 Hz, 1 H) 2.96 - 3.02 (m, 1 H) 3.08 - 3.15 (m, 1 H) 3.25 - 3.45 (m, 7 H) 3.57 (br. s., 1 H) 3.92 (d, *J*=12.79 Hz, 1 H) 4.04 (d, *J*=6.88 Hz, 1 H) 4.18 (s, 1 H) 4.21 - 4.28 (m, 1 H) 4.92 (dd, *J*=8.21, 2.08 Hz, 1 H) 5.19 (d, *J*=2.19 Hz, 1 H) 5.61 (d, *J*=3.71 Hz, 1 H) 7.97 (t, *J*=8.10 Hz, 1 H) 8.23 (d, *J*=7.64 Hz, 1 H) 8.98 (d, *J*=8.32 Hz, 1 H)

By analogous procedure the following compound can be prepared:

### N-[(8S,10S)-6,8,11-trihydroxy-8-hydroxyacetyl-5,12-dioxo-10-({(3ξ)-2,3,6-trideoxy-3-[(2S)-2-methoxy-4-oxidomorpholin-4-yl]-α-L-threo-hexopyranosyl}oxy)-5,7,8,9,10,12-hexahydrotetracen-1-yl]-2,2,2-trifluoroacetamide (Comp. 53)

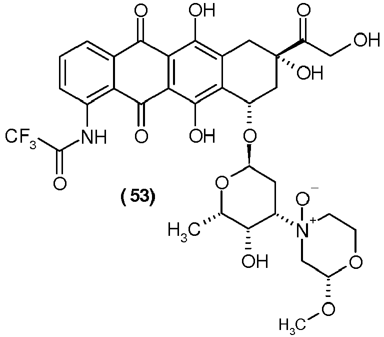

MS (ESI): 645 [M+H]⁺.

### Step B1 (A6b)

### N-[(8S,10S)-8-acetyl-6,8,11-trihydroxy-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-5,12-dioxo-5,7,8,9,10,12-hexahydrotetracen-1-yl]-2,2,2-trifluoroacetamide (Comp. 34)

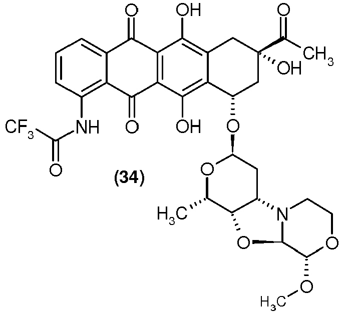

To a solution of compound *N*-[(8*S*,10*S*)-8-acetyl-6,8,11-trihydroxy-5,12-dioxo-10-({(3ξ)-2,3,6-trideoxy-3-[(2*S*)-2-methoxy-4-oxidomorpholin-4-yl]-α-L-*threo*-hexopyranosyl}oxy)-5,7,8,9,10,12-hexahydrotetracen-1-yl]-2,2,2-trifluoroacetamide (**52**) (60.0 mg, 0.083 mmol) in 13 ml of dry CH₃CN, K₂CO₃ (34.4 mg, 0.249 mmol) and cyanuric chloride (30.6 mg, 0.166 mmol) were added. The reaction mixture was vigorously stirred in the dark at room temperature for 15 minutes, until no starting material was detectable. A solution of 3-amino-1,2-propanediol (45.3 mg, 0.5 mmol) in water (0.22 ml) was then added to the reaction mixture and the aqueous phase was extracted with DCM (4 x 10 ml). The combined organic phases were dried over anhydrous Na₂SO₄, filtered and evaporated under vacuum. The crude was purified by flash column chromatography (AcOEt/toluene; 4/6) on silica gel (230-400 mesh), affording 12.0 mg of compound **(34)** as red solid.
MS (ESI): 707 [M+H]⁺.
¹H NMR (500 MHz, CH₃CN-*d*₃) δ ppm 1.29 (d, *J*=6.58 Hz, 4 H) 1.70 (d, *J*=15.21 Hz, 1 H) 1.90 (d, *J*=15.59 Hz, 2 H) 2.04 - 2.08 (m, 2 H) 2.45 (d, *J*=14.98 Hz, 1 H) 2.69 - 2.76 (m, 1 H) 2.77 - 2.83 (m, 1 H) 2.97 (s, 1 H) 3.08 - 3.14 (m, 2 H) 3.38 (s, 4 H) 3.45 (d, *J*=6.88 Hz, 2 H) 3.56 (d, *J*=5.22 Hz, 2 H) 3.74 (s, 1 H) 4.04 (d, *J*=1.89 Hz, 2 H) 4.09 (d, *J*=6.88 Hz, 1 H) 4.26 (d, *J*=2.72 Hz, 1 H) 4.52 - 4.54 (m, 2 H) 5.22 (br. s., 1 H) 5.36 (t, *J*=5.60 Hz, 1 H) 7.98 (t, *J*=8.06 Hz, 1 H) 8.26 (d, *J*=7.87 Hz, 1 H) 9.00 (d, *J*=8.10 Hz, 1 H)

### Deprotection

### The title Comp. (22)

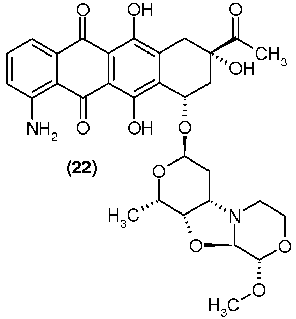

The *N*-[(8*S*,10*S*)-8-acetyl-6,8,11-trihydroxy-10-{[(1*S*,3*R*,4a*S*,9*S*,9a*R*,10a*S*)-9-methoxy-1-methyloctahydro-1*H-*pyrano[4',3':4,5][1,3]oxazolo[2,3-c][1,4]oxazin-3-yl]oxy}-5,12-dioxo-5,7,8,9,10,12-hexahydrotetracen-1-yl]-2,2,2-trifluoroacetamide intermediate **(34)** (4.8 mg, 0,00679 mmol) was cooled at 0 °C and 0.1 N NaOH aqueous solution (0.5 ml) was added. The reaction mixture was stirred in the dark 0 °C for 15 minutes, until no starting material was detectable. The reaction mixture was then diluted with H₂O and extracted with DCM (4x5 ml). The combined organic phases were washed with saturated aqueous NaCl solution (1 X 10 ml), dried over anhydrous Na₂SO₄, filtered and evaporated under vacuum, affording 4.0 mg of title compound as red solid.
MS (ESI): 611 [M+H]⁺.
¹H NMR (500 MHz, CH₃CN-*d*₃) δ ppm 1.70 (dt, *J*=15.06, 5.82 Hz, 2 H) 1.87 (dt, *J*=15.17, 5.54 Hz, 1 H) 2.34 (s, 3 H) 2.43 (d, *J*=14.41 Hz, 1 H) 2.68 - 2.81 (m, 2 H) 2.91 - 2.96 (m, 1 H) 3.07 (d, *J*=18.66 Hz, 1 H) 3.37 (s, 3 H) 3.44 (q, *J*=5.87 Hz, 1 H) 3.51 - 3.61 (m, 2 H) 3.74 (ddd, *J*=11.63, 8.25, 2.96 Hz, 1 H) 4.01 - 4.04 (m, 1 H) 4.06 - 4.13 (m, 1 H) 4.26 (d, *J*=2.66 Hz, 1 H) 4.54 (d, *J*=2.58 Hz, 1 H) 5.21 (br. s., 1 H) 5.37 (t, *J*=5.61 Hz, 1 H) 7.16 (d, *J*=8.57 Hz, 1 H) 7.54 (t, *J*=7.89 Hz, 1 H) 7.62 (d, *J*=7.06 Hz, 1 H)

Analogously, by using the suitable starting material, the following compound is prepared:

### (8S,10S)-1-amino-6,8,11-trihydroxy-8-(hydroxyacetyl)-10-{[(1S,3R,4aS,9S,9aR,10aS)-9-methoxy-1-methyloctahydro-1H-pyrano[4',3':4,5][1,3]oxazolo[2,3-c][1,4]oxazin-3-yl]oxy}-7,8,9,10-tetrahydrotetracene-5,12-dione (Comp. 29)

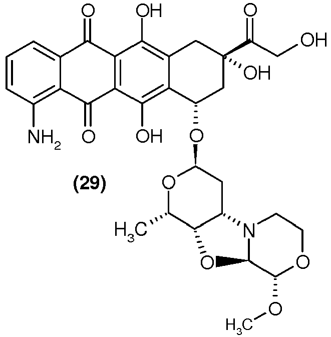

MS (ESI): 627[M+H]⁺.

### Example 7

### Step G2, Deprotection, Protection, Step G3, Deprotection

### (1S,3S)-3-acetyl-10-amino-3,5,12-trihydroxy-6,11-dioxo-1,2,3,4,6,11-hexahydrotetracen-1-yl 3-amino-2,3,6-trideoxy-L-lyxo-hexopyranoside (XX) (Comp. 48)

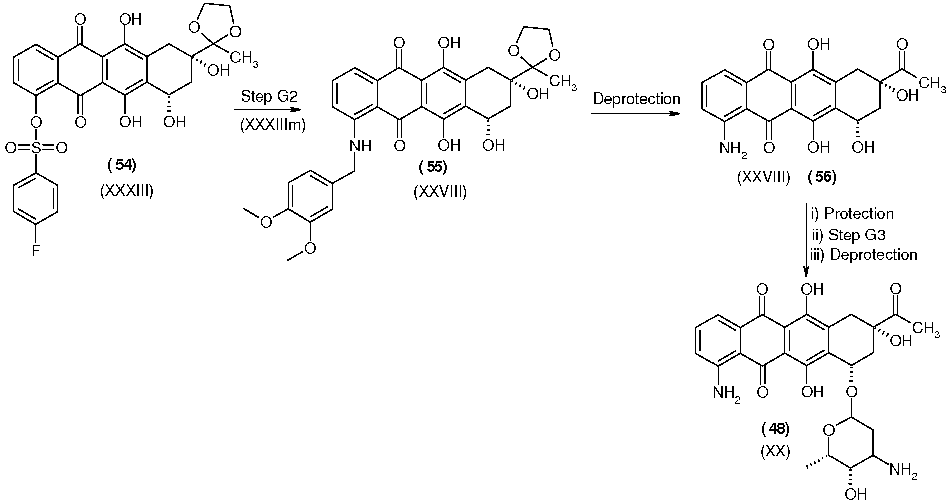

### Step G2

### Synthesis of the intermediate (8S,10S)-1-[(3,4-dimethoxybenzyl)amino]-6,8,10,11-tetrahydroxy-8-(2-methyl-1,3-dioxolan-2-yl)-7,8,9,10-tetrahydrotetracene-5,12-dione (XXVIII) (Comp. 55)

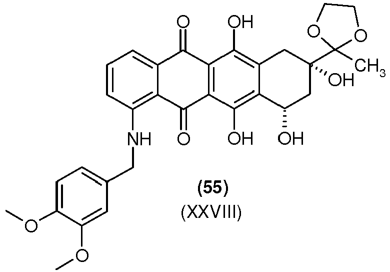

To a solution of (8*S*,10*S*)-6,8,10,11-tetrahydroxy-8-(2-methyl-1,3-dioxolan-2-yl)-5,12-dioxo-5,7,8,9,10,12-hexahydrotetracen-1-yl 4-fluorobenzenesulfonate **(54)** (400 mg, 0.682 mmol) [prepared as reported in GB2215332] in THF (10 ml), 3-4 dimethoxybenzylamine (0.532 mg, 3.1 mmol) was added. The solution was heated at 60 ° C and stirred for 24 hours in the dark. Then, the solvent was partially removed under vacuum, the dark violet precipitate collected by filtration, washed with THF (3 ml) and then with Et₂O (10 ml). The solid was finally dried in the oven under vacuum at 30 °C to yield the title intermediate **(55)** (188 mg, yield 48%).

Analogously, by using the suitable amines, the following compounds were prepared:

### (8S,10S)-6,8,10,11-tetrahydroxy-1-[(2-hydroxyethyl)amino]-8-(2-methyl-1,3-dioxolan-2-yl)-7,8,9,10-tetrahydrotetracene-5,12-dione (Comp. 57)

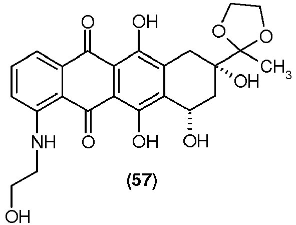

MS (ESI): 472 [M+H]⁺.
¹H NMR (499.75 MHz, DMSO-*d*₆) δ ppm 1.33 (s, 3 H), 1.82 (dd, *J* = 14.3, 4.3 Hz, 1 H), 2.20 (d, *J* = 14.3 Hz, 1 H), 2.67 (d, *J* = 18.7 Hz, 1 H), 3.10 (d, *J* = 18.7 Hz, 1 H), 3.45-3.49 (m, 2 H), 3.68-3.72 (m, 2 H), 3.92 - 4.01 (m, 4 H), 5.00 (t, *J* = 5.1 Hz, 1 H), 5.05-5.11 (m, 1 H), 5.35 (d, *J* = 7.6 Hz, 1 H), 5.44 (s, 1 H), 7.35 (d, *J* = 8.7 Hz, 1 H), 7.56 (d, *J* = 6.8 Hz, 1 H), 7.70 (dd, *J* = 8.7, 6.8 Hz, 1 H), 9.61 (t, *J* = 5.1 Hz, 1 H), 13.52 (br. s., 1 H), 13.74 (br. s., 1 H)

### (8S,10S)-1-[(2-aminoethyl)amino]-6,8,10,11-tetrahydroxy-8-(2-methyl-1,3-dioxolan-2-yl)-7,8,9,10-tetrahydrotetracene-5,12-dione (Comp. 58)

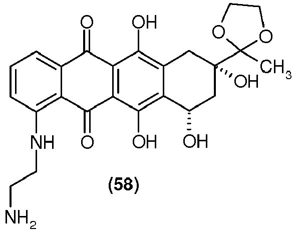

MS (ESI): 471[M+H]⁺.

### Deprotection

### Synthesis of the intermediate (8S,10S)-8-acetyl-1-amino-6,8,10,11-tetrahydroxy-7,8,9,10-tetrahydrotetracene-5,12-dione (XXVIII) (Comp. 56)

To cold trifluoroacetic acid (2 ml), (8*S*,10*S*)-1-[(3,4-dimethoxybenzyl)amino]-6,8,10,11-tetrahydroxy-8-(2-methyl-1,3-dioxolan-2-yl)-7,8,9,10-tetrahydrotetracene-5,12-dione **(55)** (133 mg, 0.230 mmol) and 2 drops of anisole were added. The solution was stirred at 5 °C for 20 min and then at room temperature for 2 hours until no starting material was detectable. The reaction was diluted with water (5 ml), neutralized with NaHCO₃ solution then, the aqueous phase extracted with DCM (3 x 50 ml). The combined organic phases were dried over anhydrous Na₂SO₄, filtered, the solvent evaporated under vacuum and the crude treated with Et₂O (10 ml). The dark violet precipitate was collected by filtration and dried in the oven under vacuum at 30 °C to yield the desired intermediate (82 mg, yield 93%).
MS (ESI): 384 [M+H]⁺.
¹H NMR (400.5 MHz, DMSO-*d*₆) δ ppm 1.99 (dd, *J* = 14.4, 4.6 Hz, 1 H), 2.13 - 2.19 (m, 1 H), 2.30 (s, 3 H), 2.88 - 2.95 (m, 1 H), 2.98 - 3.05 (m, 1 H), 5.07 (m, 1 H), 5.29 (br. s., 1 H), 6.07 (s, 1 H), 7.24 (dd, *J* = 8.3, 1.1 Hz, 1 H), 7.51 (dd, *J* = 7.3, 1.1 Hz,, 1H), 7.55 - 7.60 (m, 1 H), 8.05 (br. s., 2H), 13.49 (br. s., 1 H), 13.85 (br. s., 1 H)

Analogously, by using the suitable starting material, the following compounds were prepared:

### (8S,10S)-8-acetyl-6,8,10,11-tetrahydroxy-1-[(2-hydroxyethyl)amino]-7,8,9,10-tetrahydrotetracene-5,12-dione (Comp. 59)

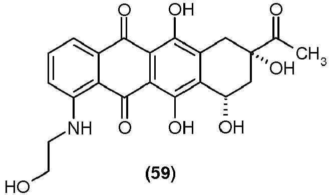

MS (ESI): 428 [M+H]⁺.
¹H NMR (500 MHz, DMSO-*d*₆) δ ppm 1.98 (dd, *J* = 14.2, 4.6 Hz, 1 H), 2.16 (d, *J* = 14.2 Hz, 1 H), 2.31 (s, 3 H), 2.88 - 2.94 (m, 1 H), 2.98 - 3.04 (m, 1 H), 3.46-3.49 (m, 2 H), 3.68-3.72 (m, 2 H), 5.01 (t, *J* = 5.1 Hz, 1 H), 5.05-5.10 (m, 1 H), 5.30 (d, *J* = 6.7 Hz, 1 H), 6.10 (s, 1 H), 7.35 (d, *J* = 8.7 Hz, 1 H), 7.56 (d, *J* = 7.1 Hz, 1 H), 7.70 (dd, *J* = 8.7, 7.1 Hz, 1 H), 9.62 (t, *J* = 5.1 Hz, 1 H), 13.47 (br. s., 1 H), 13.76 (br. s., 1 H)

### (8S,10S)-8-acetyl-1-[(2-aminoethyl)amino]-6,8,10,11-tetrahydroxy-7,8,9,10-tetrahydrotetracene-5,12-dione (Comp. 60)

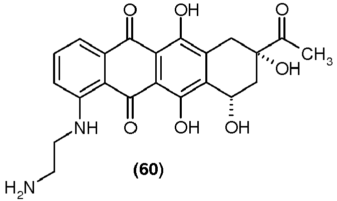

MS (ESI): 427[M+H]⁺.
¹H NMR (500 MHz, DMSO-*d*₆) δ ppm 1.97 (dd, *J* = 14.2, 4.6 Hz, 1 H), 2.14 (d, *J* = 14.2 Hz, 1 H), 2.31 (s, 3 H), 2.88 - 2.94 (m, 1 H), 2.98 - 3.04 (m, 1 H), 3.05-3.22 (m, 4 H), 5.05-5.10 (m, 1 H), 5.30 (d, *J* = 6.7 Hz, 1 H), 6.10 (s, 1 H), 7.35 (d, *J* = 8.7 Hz, 1 H), 7.54 (d, *J* = 7.1 Hz, 1 H), 7.70 (dd, *J* = 8.7, 7.1 Hz, 1 H), 9.60 (t, *J* = 5.1 Hz, 1 H), 13.46 (br. s., 1 H), 13.76 (br. s., 1 H)

### Protection

### Synthesis of the intermediate N-[(8S,10S)-8-acetyl-6,8,10,11-tetrahydroxy-5,12-dioxo-5,7,8,9,10,12-hexahydrotetracen-1-yl]-2,2,2-trifluoroacetamide (Comp. 61)

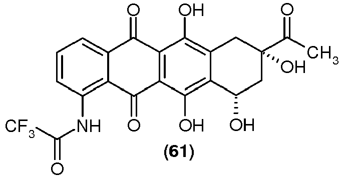

The intermediate (8S,10S)-8-acetyl-1-amino-6,8,10,11-tetrahydroxy-7,8,9,10-tetrahydrotetracene-5,12-dione (56) (600.0 mg, 1.56 mmol) was dissolved in dry DCM (120 ml) and trifluoroacetic anhydride (1.2 ml) was added. The reaction mixture was stirred at room temperature in the dark for 5 minutes, until no starting material was detectable (HPLC analysis). The reaction mixture was then diluted with DCM (100 ml) and washed with satured NaHCO₃ aqueous solution (3 X 100 ml), and then with water (1 X 100 ml). The organic phase was dried over anhydrous Na₂SO₄, and the solvent was removed under vacuum. The residue thus obtained was purified by flash chromatography (eluant: CH₃COCH₃/DCM; 0.3/9.7) on silica gel (230-400 mesh) affording the desired product (494.1 mg, red solid).
MS (ESI): 480 [M+H]⁺.
¹H NMR (500 MHz, CHCl₃-*d*) δ ppm 2.22 (dd, *J*=14.5, 4.9 Hz, 1 H), 2.36 - 2.41 (m, 1 H), 2.45 (s, 3 H), 3.01 (d, *J*=18.7 Hz, 1 H), 3.23 (dd, *J*=18.7, 2.2 Hz, 1 H), 3.81 (d, *J*=5.2 Hz, 1 H), 4.54 (s, 1 H), 5.35 (m, 1 H), 7.93 (dd, *J*=8.4, 7.7 Hz, 1 H), 8.29 (dd, *J*=7.7, 1.1 Hz, 1 H), 9.12 (dd, *J*=8.4, 1.1 Hz, 1 H), 13.29 (br.s., 1H), 13.29 (s, 1H), 13.46 (s, 1H).

Analogously, by using the suitable starting material, the following compound is prepared:

### N-(2-{[(8S,10S)-8-acetyl-6,8,10,11-tetrahydroxy-5,12-dioxo-5,7,8,9,10,12-hexahydrotetracen-1-yl]amino}ethyl)-2,2,2-trifluoroacetamide (Comp. 62)

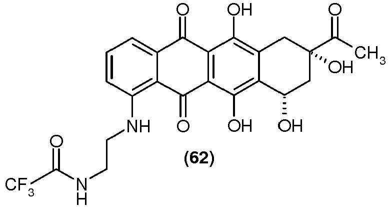

MS (ESI): 523 [M+H]⁺.

### Step G3

### Synthesis of N-[(8S,10S)-8-acetyl-6,8,11-trihydroxy-5,12-dioxo-10-({2,3,6-trideoxy-3-[(trifluoroacetyl)amino]-L-lyxo-hexopyranosyl}oxy)-5,7,8,9,10,12-hexahydrotetracen-1-yl]-2,2,2-trifluoroacetamide (Comp. 63)

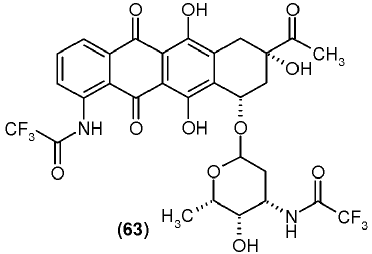

In a dry three-necked round-bottomed flask under argon atmosphere, the intermediate *N*-[(8*S*,10*S*)-8-acetyl-6,8,10,11-tetrahydroxy-5,12-dioxo-5,7,8,9,10,12-hexahydrotetracen-1-yl]-2,2,2-trifluoroacetamide (**61**) (480.0 mg, 1.0 mmol) was dissolved in dry DCM (110 ml), and powdered molecular sieves (4 Å, 20.0 mg) were added. The reaction mixture was cooled at 10 °C; the solution of silver trifluoromethanesulfonate (334.0 mg, 1.3 mmol) in dry Et₂O (15 ml) and the solution of 2,3,6-trideoxy-4-*O*-(trifluoroacetyl)-3-[(trifluoroacetyl)amino]-δ-L-*lyxo*-hexopyranosyl chloride (511.4 mg, 1.43 mmol) in dry DCM (15 ml) were simultaneously added. The reaction mixture was stirred at 10 °C in the dark for 45 minutes, until no starting material was detectable (HPLC analysis). Saturated NaHCO₃ aqueous solution (50 ml) was added and the reaction mixture was stirred at room temperature for 30 minutes, and then filtered through a celite pad. The organic phase was separated, washed with water and dried over anhydrous Na₂SO₄. The solvent was removed under vacuum, and the residue thus obtained was cooled at 0 °C and treated with MeOH (20 ml) and solid NaHCO₃ for 15 minutes. The solvent was evaporated under vacuum and the residue was purified by flash chromatography (eluant: CH₃COCH₃/DCM; 0.5/9.5) on silica gel (230-400 mesh) affording the desired product (320.0 mg, red solid).
MS (ESI): 705 [M+H]⁺.
¹H NMR (500 MHz, CH₃CN-*d*₃) δ ppm 1.22 (d, *J*=6.47 Hz, 3 H) 1.77 (dd, *J*=13.18, 4.64 Hz, 1 H) 2.00 (td, *J*=13.15, 3.97 Hz, 1 H) 2.10 (d, *J*=10.13 Hz, 1 H) 2.33 - 2.35 (m, 1 H) 2.87 - 3.00 (m, 1 H) 3.01 - 3.13 (m, 1 H) 3.22 (br. s., 1 H) 3.61 (br. s., 1 H) 4.09 - 4.16 (m, 1 H) 4.22 (q, *J*=6.47 Hz, 1 H) 4.29 (s, 1 H) 5.10 (br. s., 1 H) 5.40 (d, *J*=3.54 Hz, 1 H) 7.32 (d, *J*=7.81 Hz, 1 H) 7.89 (t, *J*=7.63 Hz, 1 H) 8.11 (d, *J*=7.08 Hz, 1 H) 8.89 (d, *J*=8.30 Hz, 1 H) 13.08 (br. s., 2 H)

Analogously, by using the suitable starting material, the following compounds are prepared:

### N-(2-{[(8S,10S)-8-acetyl-6,8,11-trihydroxy-5,12-dioxo-10-({2,3,6-trideoxy-3-[(trifluoroacetyl)amino]-L-lyxo-hexopyranosyl}oxy)-5,7,8,9,10,12-hexahydrotetracen-1-yl]amino}ethyl)-2,2,2-trifluoroacetamide (Comp. 64)

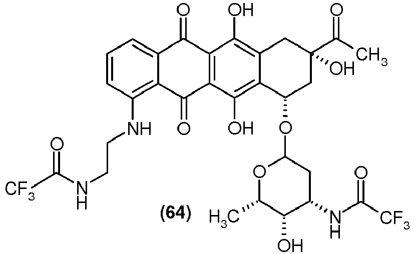

MS (ESI): 748 [M+H]⁺.

### (1S,3S)-3-acetyl-3,5,12-trihydroxy-10-[(2-hydroxyethyl)amino]-6,11-dioxo-1,2,3,4,6,11-hexahydrotetracen-1-yl 2,3,6-trideoxy-3-[(trifluoroacetyl)amino]-L-/yxo-hexopyranoside (Comp. 65)

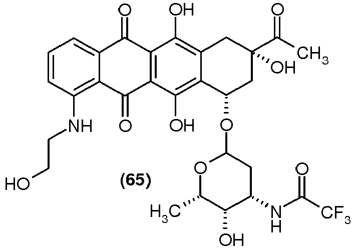

MS (ESI): 653[M+H]⁺.

### Deprotection

### The title Comp. (48)

The intermediate *N*-[(8*S*,10*S*)-8-acetyl-6,8,11-trihydroxy-5,12-dioxo-10-({2,3,6-trideoxy-3-[(trifluoroacetyl)amino]-α-L-*lyxo*-hexopyranosyl}oxy)-5,7,8,9,10,12-hexahydrotetracen-1-yl]-2,2,2-trifluoroacetamide (**63**) (340.2 mg, 0.432 mmol) was cooled at 0 °C under argon and treated with a 0.1 N NaOH aqueous solution (12 ml). The reaction mixture was stirred in the dark at 0 °C for 1 hour, until no starting material was detectable (HPLC analysis). The reaction mixture was then diluted with DCM (50 ml), washed with saturated NaHCO₃ aqueous solution (3 X 30 ml), then with water (1 X 30 ml) and finally with saturated NaCl solution (1 X 30 ml). The organic phase was dried over anhydrous Na₂SO₄, the solvent was removed under vacuum affording the desired product (180.0 mg, red solid).
MS (ESI): 513[M+H]⁺.
¹H NMR (500 MHz, DMSO-*d*₆)δ pm 1.14 (d, *J*=6.52 Hz, 2 H) 1.47 (dd, *J*=12.61, 4.33 Hz, 1 H) 1.60 (d, *J*=3.30 Hz, 1 H) 2.06 - 2.21 (m, 2 H) 2.24 - 2.27 (m, 3 H) 2.86 (d, *J*=12.58 Hz, 1 H) 2.88 - 3.01 (m, 2 H) 3.28 (br. s., 1 H) 4.09 (d, *J*=6.29 Hz, 1 H) 4.45 (br. s., 1 H) 4.94 (t, *J*=4.22 Hz, 1 H) 5.19 (d, *J*=3.53 Hz, 1 H) 5.44 (s, 1 H) 7.24 (d, *J*=8.28 Hz, 1 H) 7.50 (d, *J*=7.13 Hz, 1 H) 7.51 - 7.52 (m, 0 H) 7.55 - 7.59 (m, 1 H) 8.06 (br. s., 2 H)

Analogously, by using the suitable starting material, the following compounds are prepared:

### (1S,3S)-3-acetyl-10-[(2-aminoethyl)amino]-3,5,12-trihydroxy-6,11-dioxo-1,2,3,4,6,11-hexahydrotetracen-1-yl 3-amino-2,3,6-trideoxy-L-lyxo-hexopyranoside (Comp. 66)

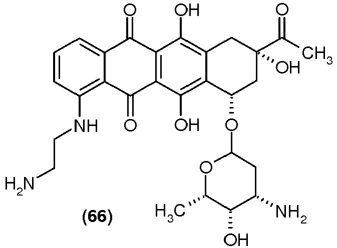

MS (ESI): 556 [M+H]⁺.

### (1S,3S)-3-acetyl-3,5,12-trihydroxy-10-[(2-hydroxyethyl)amino]-6,11-dioxo-1,2,3,4,6,11-hexahydrotetracen-1-yl 3-amino-2,3,6-trideoxy-L-lyxo-hexopyranoside (Comp. 67)

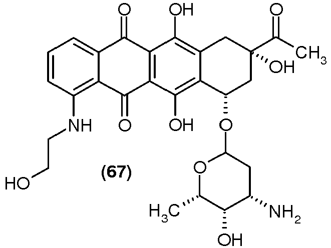

MS (ESI): 557 [M+H]⁺.

### Example 8

### Preparation of the intermediate N-[6-(2,5-dioxo-2,5-dihydro-1H-pyrrol-1-yl)hexanoyl]-L-valyl-N⁵-carbamoyl-N-{4-[({methyl[2-(methylamino)ethyl] carbamoyl}oxy)methyl]phenyl}-L-ornithinamide hydrochloride (Comp. 39)

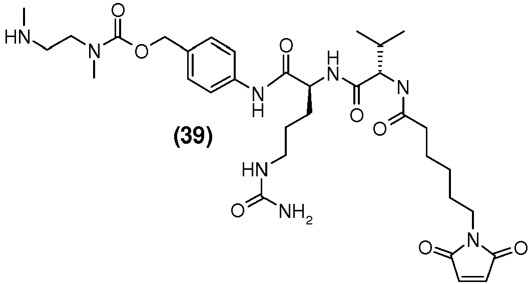

### Step a

*N*-[(9*H*-fluoren-9-ylmethoxy)carbonyl]-L-valyl-N⁵-carbamoyl-N-[4-(hydroxymethyl)phenyl]-L-ornithinamide (1.3 g, 2.16 mmol) (prepared as reported in EP0624377A2) and bis(4-nitrophenyl) carbonate (1.32 g, 4.34 mmol) were dissolved in 6 ml of dry DMF under nitrogen atmosphere, DIPEA (0.75 ml, 4.35 mmol) was added and the resulting solution was stirred an hour at room temperature. Diethylether (120 ml) was added, the resulting precipitate is filtered off, washed with diethylether and dried under vacuum affording N-[(9H-fluoren-9-ylmethoxy)carbonyl]-L-valyl-N⁵-carbamoyl-N-[4-({[(4-nitrophenoxy)carbonyl]oxy}methyl)phenyl]-L-ornithinamide (**68**) (1.47 g, 89% yield). 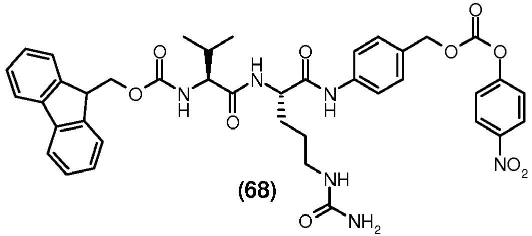 MS (ESI): 767 [M+H]⁺.
¹H NMR (400 MHz, DMSO-d₆) δ 0.86 (d, J = 6.7 Hz, 3 H), 0.88 (d, J = 6.7 Hz, 3 H), 1.30 - 1.52 (m, 2 H), 1.60 (m, 1 H), 1.69 (m, 1 H), 1.99 (m, 1 H), 2.90 - 3.10 (m, 2 H), 3.93 (dd, J = 8.9, 7.0 Hz, 1 H), 4.14 - 4.34 (m, 3 H), 4.42 (m, 1 H), 5.24 (s, 2 H), 5.39 (s, 2 H), 5.97 (t, J = 5.5 Hz, 1 H), 7.32 (m, 2 H), 7.42 (m, 5 H), 7.55 (m, 2 H), 7.65 (d, J = 8.4 Hz, 2 H), 7.74 (t, J = 7.9 Hz, 2 H), 7.88 (d, J = 7.6 Hz, 2 H), 8.12 (d, J = 7.4 Hz, 1 H), 8.31 (m, 2 H), 10.12 (s, 1 H)

### Step b

*N*-[(9*H*-fluoren-9-ylmethoxy)carbonyl]-L-valyl-N⁵-carbamoyl-N-[4-({[(4-nitrophenoxy)carbonyl]oxy}methyl)phenyl]-L-ornithinamide (310 mg, 0.404 mmol) and 2-(biphenyl-4-yl)propan-2-yl methyl[2-(methylamino)ethyl]carbamate (**68**) (132 mg, 0.404 mmol) were dissolved in dry DMF (6 ml) and stirred under nitrogen atmosphere at room temperature for 3 hours. Solvent was evaporated and the resulting crude producy was purified by column chromatography (DCM / EtOH = 9 / 1) affording *N*-[(9*H*-fluoren-9-ylmethoxy)carbonyl]-L-valyl-N-{4-[10-(biphenyl-4-yl)-4,7,10-trimethyl-3,8-dioxo-2,9-dioxa-4,7-diazaundec-1-yl]phenyl}-N⁵-carbamoyl-L-ornithinamide (**69**) (200 mg, 52% yield). 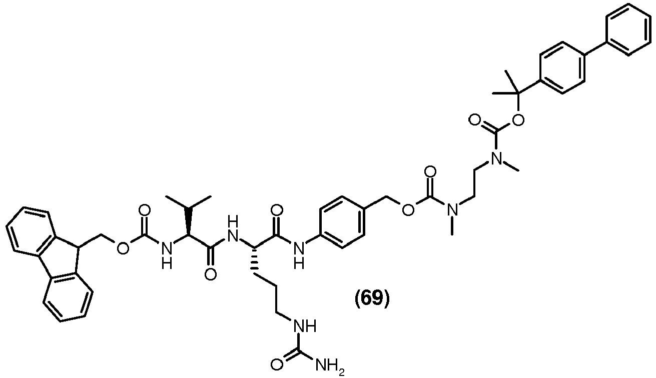 MS (ESI): 954 [M+H]⁺.
¹H NMR (500 MHz, DMSO-d₆) δ 0.86 (m, 6 H), 1.21-1.64 (m, 4H), 1.69 (s., 6 H), 2.00 (m, 1 H), 2.62 - 3.08 (m, 10 H), 3.44 (m, 2 H), 3.93 (t, J = 7.6 Hz, 1 H), 4.22 (m, 1 H), 4.30 (m, 1 H), 4.42 (m, 1 H), 4.94 - 5.03 (m, 2 H), 5.39 (s, 2 H), 5.96 (m, 1 H), 7.25 - 7.46 (m, 11 H), 7.45 - 7.66 (m, 6 H), 7.74 (m, 2 H), 7.89 (m, 2 H), 8.11 (br.s., 1 H), 10.06 (br. s., 1 H)

### Step c

To a solution of *N*-[(9*H*-fluoren-9-ylmethoxy)carbonyl]-L-valyl-N-{4-[10-(biphenyl-4-yl)-4,7,10-trimethyl-3,8-dioxo-2,9-dioxa-4,7-diazaundec-1-yl]phenyl}-N⁵-carbamoyl-L-ornithinamide **(69)** (200 mg, 0.21 mmol) in dry DMF (6 ml) was added piperidine (0.105 ml, 1 mmol). The resulting solution was stirred at room temperature for 2 hours, concentrated to dryness to afford crude L-valyl-N-{4-[10-(biphenyl-4-yl)-4,7,10-trimethyl-3,8-dioxo-2,9-dioxa-4,7-diazaundec-1-yl]phenyl}-N⁵-carbamoyl-L-ornithinamide which was used without further purification. The crude intermediate was dissolved in dry DCM (6 ml) and 1-{6-[(2,5-dioxopyrrolidin-1-yl)oxy]-6-oxohexyl}-1H-pyrrole-2,5-dione (130 mg, 0.42 mmol) and triethylamine (0.088 ml, 0.63 mmol) were added. The resulting solution was stirred at room temperature overnight, solvents were evaporated and the residue was purified by column chromatography (DCM / EtOH = 10 / 1) affording *N*-[6-(2,5-dioxo-2,5-dihydro-1*H*-pyrrol-1-yl)hexanoyl]-L-valyl-N-{4-[10-(biphenyl-4-yl)-4,7,10-trimethyl-3,8-dioxo-2,9-dioxa-4,7-diazaundec-1-yl]phenyl}-N⁵-carbamoyl-L-ornithinamide **(70)** (150 mg, 77% yield). 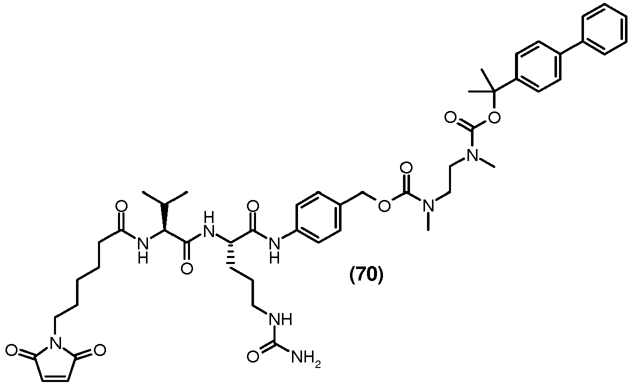 MS (ESI): 925 [M+H]⁺.
¹H NMR (500 MHz, DMSO-d₆) δ 0.81 (d, J = 6.7 Hz, 3 H), 0.84 (d, J = 6.7 Hz, 3 H), 1.18 (m, 2 H), 1.36 (m, 1 H), 1.40-1.52 8m, 6H), 1.60 (m, 1 H), 1.69 (s, 6 H), 1.97 (m, 1 H), 2.08 - 2.23 (m, 2 H), 2.63 - 2.96 (m, 8 H), 2.90 (m, 1 H), 3.01 (m, 1 H), 3.23- 3.34 (m, 2H), 3.36 (m, 2 H), 3.45 (m, 2 H), 4.19 (t, J = 7.4 Hz, 1 H), 4.38 (m, 1 H), 4.95 - 5.05 (m, 2 H), 5.40 (s, 2 H), 5.97 (d, J = 5.6 Hz, 1 H), 6.99 (s, 2 H), 7.25-7.41 (m, 5 H), 7.45 (m, 2 H), 7.53 - 7.66 (m, 6 H), 7.80 (d, J =9 Hz, 1 H), 8.08 (d, J = 7.0 Hz, 1 H), 9.99 (m, 1 H), 10.76 (br.s., 1 H).

### Step e

### The title intermediate (39)

*N*-[(9*H*-fluoren-9-ylmethoxy)carbonyl]-L-valyl-N-{4-[10-(biphenyl-4-yl)-4,7,10-trimethyl-3,8-dioxo-2,9-dioxa-4,7-diazaundec-1-yl]phenyl}-N⁵-carbamoyl-L-ornithinamide (50 mg, 0.054 mmol) was dissolved in 5 ml of dry THF and hydrochloric acid (4M in dioxane, 0.3 ml) was added. The solution was stirred for 3 minutes. The resulting precipitate was filtered off, washed with THF and dried under vacuum so affording the title compound (XX)' (36 mg, 92% yield). MS (ESI): 687 [M+H]⁺.
¹H NMR (500 MHz, methanol-d₄) δ 0.97 (d, J = 6.8,Hz, 3 H), 0.98 (d, J = 6.8,Hz, 3 H), 1.31 (m, 2 H), 1.54 - 1.67 (m, 6 H), 1.76 (m, 1 H), 1.89 (m, 1 H), 2.08 (m, 1 H), 2.28 (t, J = 7.5 Hz, 2 H), 2.64 - 2.75 (m, 3 H), 2.98 (s, 3 H), 3.11 (m, 1 H), 3.20 (m, 3 H), 3.48 (t, J = 7.1 Hz, 2 H), 3.61 (t, J = 5.7 Hz, 2 H), 4.14 (d, J = 7.5,Hz 1 H), 4.50 (dd, J = 9.1, 4.9 Hz, 1 H), 5.11 (s, 2 H), 6.79 (s, 2 H), 7.35 (d, J = 8.3 Hz, 2 H), 7.60 (d, J = 8.3 Hz, 2 H)

By analogous procedures and using the suitable starting materials the following compounds was prepared: 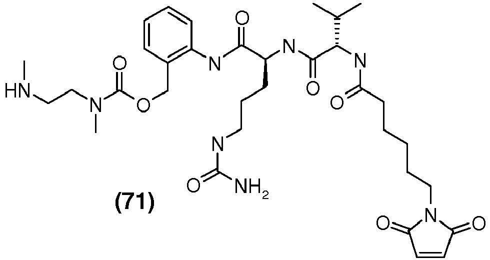 MS (ESI): 687 [M+H]⁺.

### Example 9

### Preparation of N-[6-(2,5-dioxo-2,5-dihydro-1H-pyrrol-1-yl)hexanoyl]-L-valyl-N⁵-carbamoyl-N-(4-{[(piperazin-1-ylcarbonyl)oxy]methyl}phenyl)-L-ornithinamide (Comp. 72)

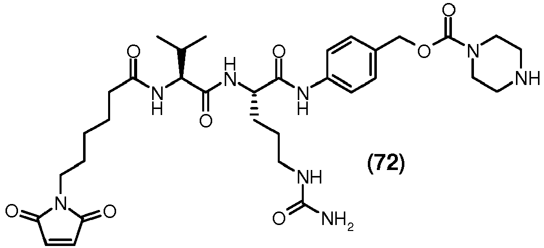

### Step 1

### N-[6-(2,5-dioxo-2,5-dihydro-1H-pyrrol-1-yl)hexanoyl]-L-valyl-N-{4-[({[4-(tert-butoxycarbonyl)piperazin-1-yl]carbonyl}oxy)methyl]phenyl}-N⁵-carbamoyl-L-ornithinamide

N-[6-(2,5-dioxo-2,5-dihydro-1H-pyrrol-1-yl)hexanoyl]-L-valyl-N⁵-carbamoyl-N-[4-({[(4-nitrophenoxy)carbonyl]oxy}methyl)phenyl]-L-ornithinamide (prepared as reported in EP0624377A2 and EP2357006A2, 30 mg, 0.041 mmol) was reacted with tert-butyl piperazine-1-carboxylate (5.3 mg, 0.0287 mmol) in anhydrous DMSO under argon atmosphere at room temperature. The reaction mixture was stirred for 1 h, until disappearance of the starting material (HPLC-MS analysis). Diethyl ether (80 ml) was then added to the reaction mixture and the precipitate thus obtained was collected by filtration to give N-[6-(2,5-dioxo-2,5-dihydro-1H-pyrrol-1-yl)hexanoyl]-L-valyl-N-{4-[({[4-(tert-butoxycarbonyl)piperazin-1-yl]carbonyl}oxy)methyl]phenyl}-N⁵-carbamoyl-L-ornithinamide as a yellow solid, 22.0 mg) was isolated and used without further purification in the next step.
MS (ESI): 785 [M+H]⁺.

### Step 2

### The title Comp. (72)

The intermediate of step 1 (22.0 mg) was treated with trifluoroacetic acid (327 mg, 2.87 mmol) in anhydrous dichloromethane (0.12 ml). The reaction mixture was stirred at room temperature for 15 minutes, until disappearance of the starting material (HPLC-MS analysis). After that, the reaction mixture was treated with diethyl ether (20 ml) and the residue thus obtained was rinsed with diethyl ether (2 x 10 ml): the compound **(72)** was thus isolated and used without further purification for the preparation of Comp. **(6),** following procedure reported in the **Example 3.**
MS (ESI): 685 [M+H]⁺.

## Claims

1. A morpholinyl anthracycline derivative of formula (I)
Ant-L-W-Z-RM (I)
wherein
**RM** is a radical selected among the groups: 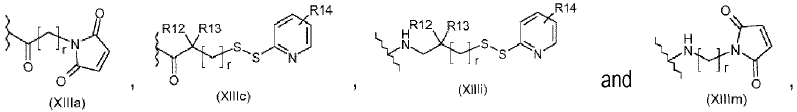 wherein
**r** is an integer from 0 to 7;
**R12** and **R13** are, each independently, hydrogen or methyl, and
**R14** is hydrogen;
the wavy line indicates the attachment point in the derivative of formula (I);
**Z** is null or a peptidic, non peptidic or hybrid - peptidic and non peptidic - linker selected among: 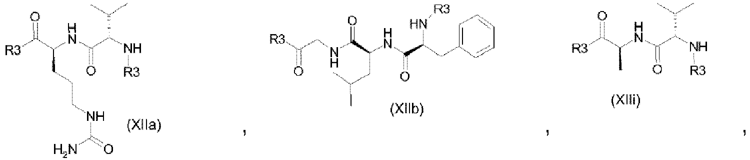 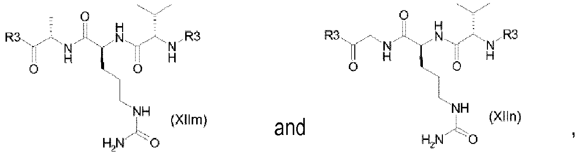 wherein
one of the two **R3** substituents is a tether to **Ant,** to **W** or to L, and the other is null;
**W** is null or a group selected among: 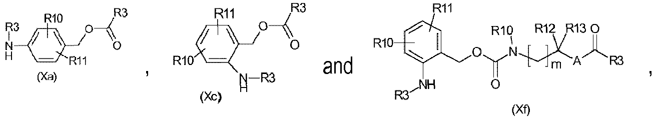 wherein
one of the two **R3** substituents is a tether to **L,** or to **Ant** when **L** is null, and the other is null;
**R10** and **R11** are hydrogen;
**m** is an integer from 0 to 3;
**A** is N-CH₃; and
**R12** and **R13** are hydrogen;
**L** is null or a moiety selected among: 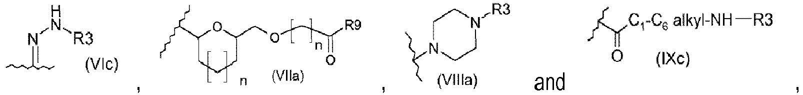 wherein
C₁-C₆ alkyl is a straight or branched chain;
**R3** is null or hydrogen, and the wavy line indicates the attachment point to **Ant;**
**R9** is null;
n is an integer from 0 to 2;
**Ant** is an anthracycline moiety selected from the formulas (IIa), (IIIa), (IVa) and (Va): 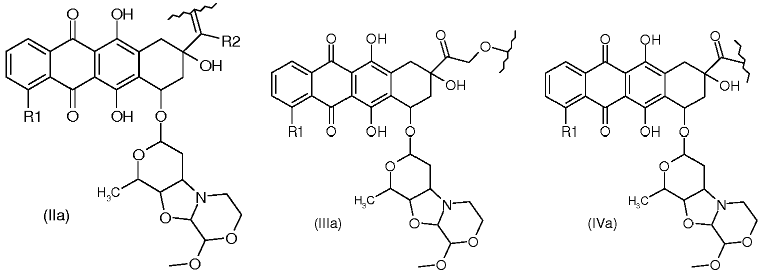 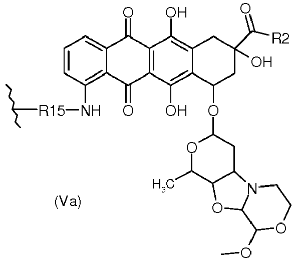
wherein the wavy line indicates the attachment
to the moiety **L,** or
to the group **W,** or
to the linker **Z,** or
to the radical **RM;**
**R1** is NH₂;
**R2** is methyl or hydroxymethyl;
**R15** is null;
or a pharmaceutically acceptable salt thereof.

2. A morpholinyl anthracycline derivative of formula (I), according to claim 1, wherein the anthracycline moiety **(Ant)** has formula (IIa) and **L** is null or a group of formula (VIc): 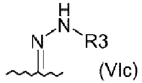 wherein:
**R3** is null, and
the wavy line indicates the attachment point to **Ant;**
or a pharmaceutically acceptable salt thereof.

3. A morpholinyl anthracycline derivative of formula (I), according to claim 1, wherein the anthracycline moiety **(Ant)** has formula (IIIa) and **L** is null or a group of formula (VIIa): 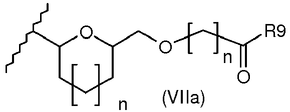 wherein:
**R9** is null;
**n** is an integer from 0 to 2, and
the wavy line indicates the attachment point to **Ant;**
or a pharmaceutically acceptable salt thereof.

4. A morpholinyl anthracycline derivative of formula (I), according to claim 1, wherein the anthracycline moiety **(Ant)** has formula (IVa) and **L** is null or a group of formula (VIIIa): 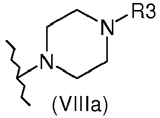 wherein:
**R3** is null, and
the wavy line indicates the attachment point to **Ant;**
or a pharmaceutically acceptable salt thereof.

5. A morpholinyl anthracycline derivative of formula **(I),** according to claim 1, wherein the anthracycline moiety **(Ant)** has formula (Va) and **L** is null or a group of formula (IXc): 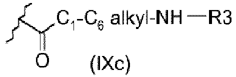 wherein:
C₁-C₆ alkyl is a straight or branched chain,
**R3** is null or hydrogen, and
the wavy line indicates the attachment point to **Ant;**
or a pharmaceutically acceptable salt thereof.

6. A morpholinyl anthracycline derivative of formula (I) according to claim 1, or a pharmaceutically acceptable salt thereof, which is selected from the group consisting of the following compounds **(1)-(20), (23)-(27), (75)-(78):**

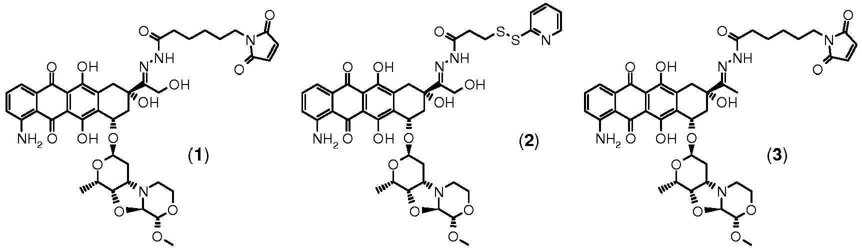

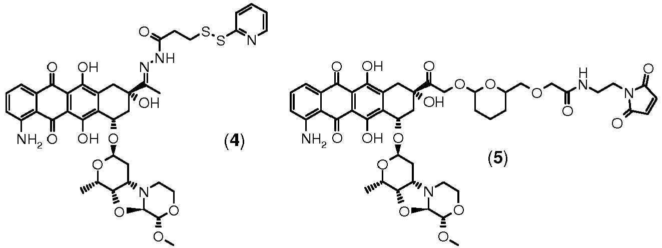

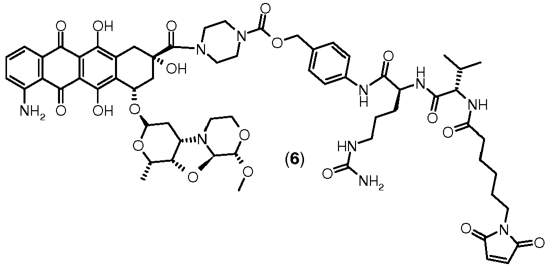

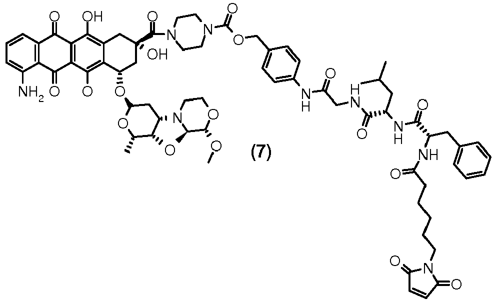

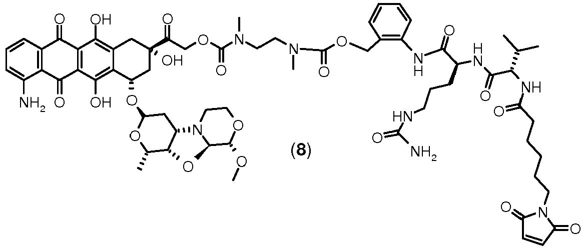

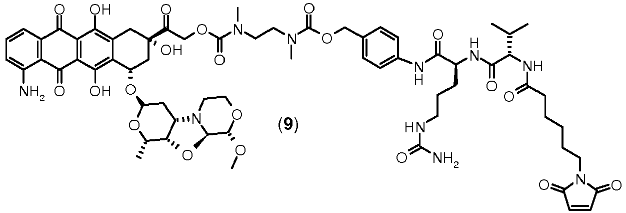

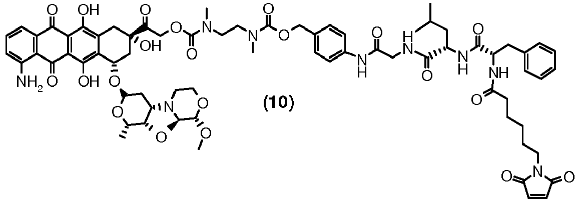

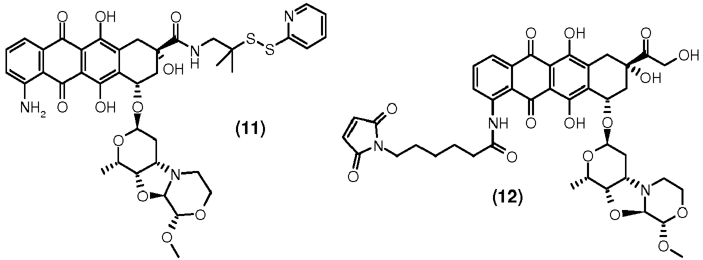

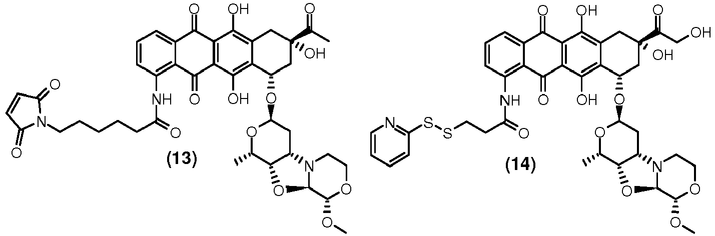

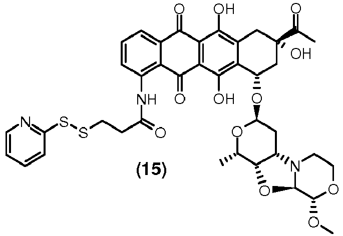

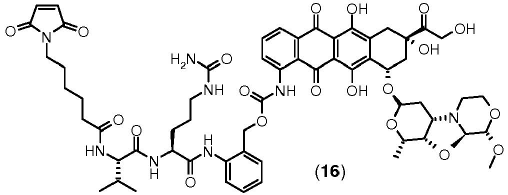

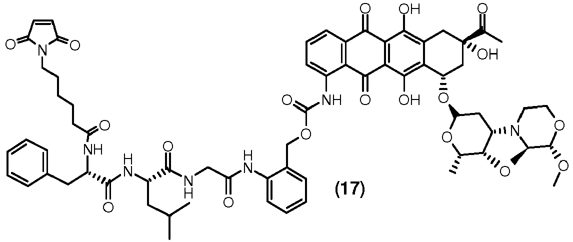

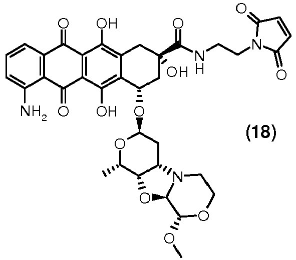

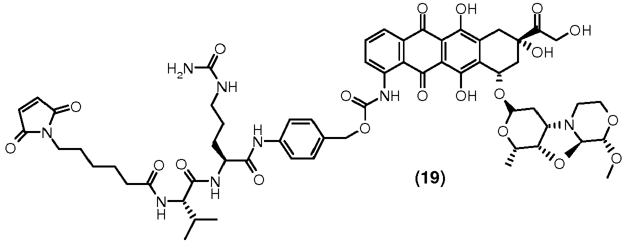

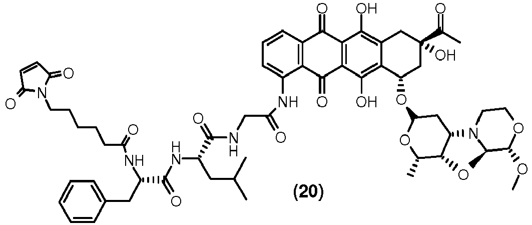

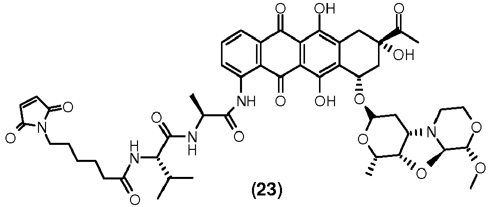

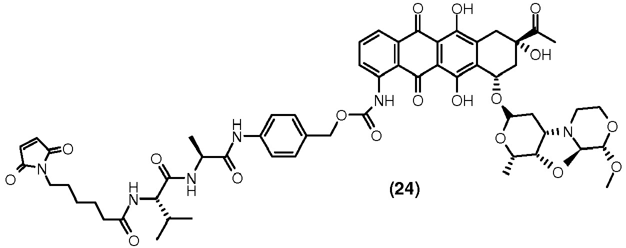

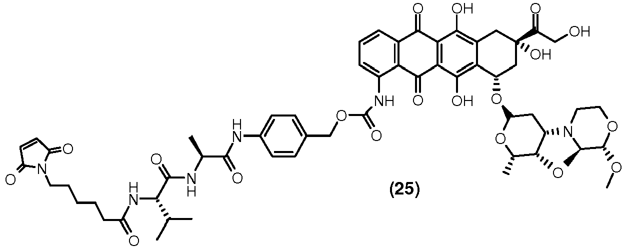

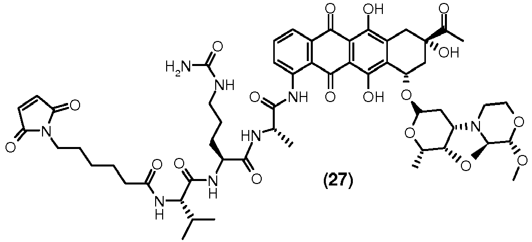

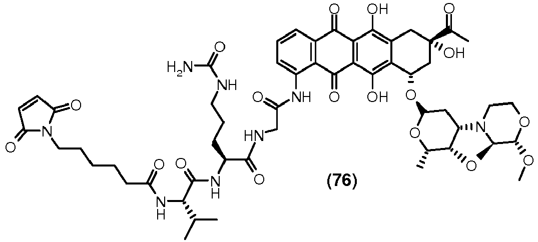

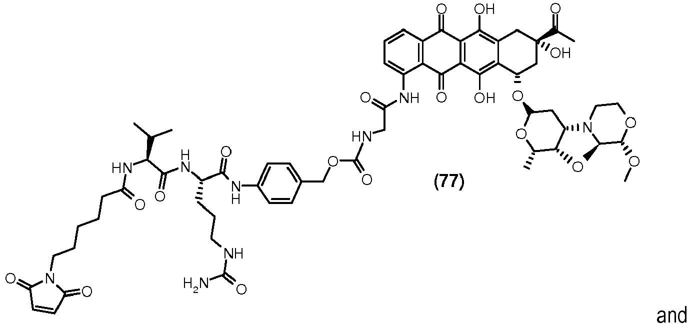

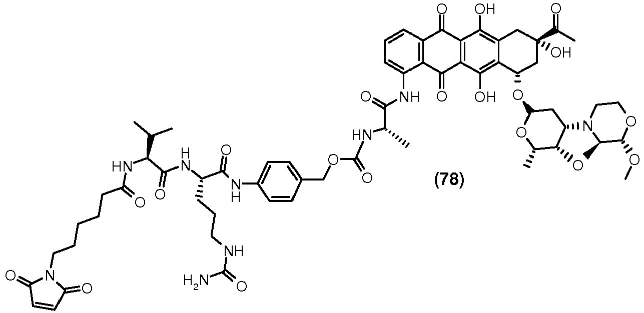

7. A pharmaceutical composition comprising a therapeutically effective amount of a morpholinyl anthracycline derivative of formula (I) or a pharmaceutically acceptable salt thereof, as defined in claim 1, and at least one pharmaceutically acceptable excipient, carrier or diluent.

8. A pharmaceutical composition according to claim 7 further comprising one or more chemotherapeutic agents.

9. A product comprising a morpholinyl anthracycline derivative of formula (I) or a pharmaceutically acceptable salt thereof, as defined in claim 1, and one or more chemotherapeutic agents, as a combined preparation for simultaneous, separate or sequential use in anticancer therapy.

10. A morpholinyl anthracycline derivative of formula (I) or a pharmaceutically acceptable salt thereof, as defined in claim 1, for use as a medicament.

11. A morpholinyl anthracycline derivative of formula (I) or a pharmaceutically acceptable salt thereof, as defined in claim 1, for use in a method of treating cancer.

12. A morpholinyl anthracycline derivative for use according to claim 11, **characterized in that** the cancer is selected from the group consisting of carcinomas, including bladder, breast, colon, kidney, liver, lung, comprising small cell lung cancer, esophagus, gall-bladder, ovary, pancreas, stomach, cervix, thyroid, prostate, and skin carcinoma, including squamous cell carcinoma; hematopoietic tumors of lymphoid lineage, including leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma and Burkitt's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannoma; and other tumors, including melanoma, seminoma, teratocarcinoma, osteosarcoma, xeroderma pigmentosum, keratoxanthoma, thyroid follicular cancer, Kaposi's sarcoma and mesothelioma.

13. Use of morpholinyl anthracycline derivative of formula (I) or a pharmaceutically acceptable salt thereof, as defined in claim 1, in the preparation of conjugates.

## Patentansprüche

1. Morpholinylanthracyclinderivat der Formel (I)
**Ant-L-W-Z-RM** **(I)**
wobei
**RM** ein Radikal ist, ausgewählt aus den folgenden Gruppen: 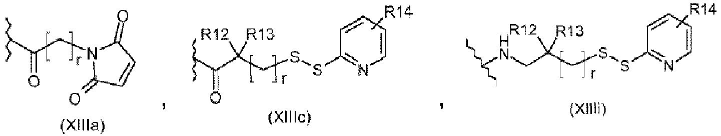 und 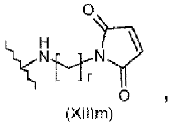 worin
**r** eine ganze Zahl von 0 bis 7 ist;
**R12** und **R13** unabhängig voneinander Wasserstoff oder Methyl sind, und
**R14** Wasserstoff ist;
die gewellte Linie den Verbindungspunkt im Derivat der Formel (I) anzeigt;
**Z** nicht vorhanden ist oder ein peptidischer, nicht peptidischer oder hybrider - peptidischer und nicht peptidischer - Linker, ausgewählt aus den Folgenden: 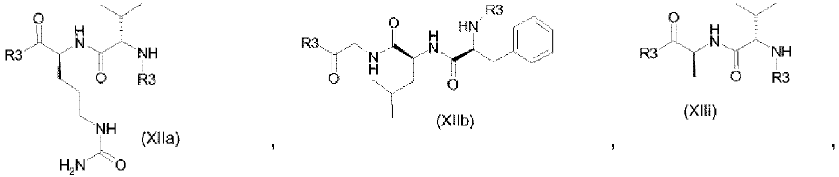 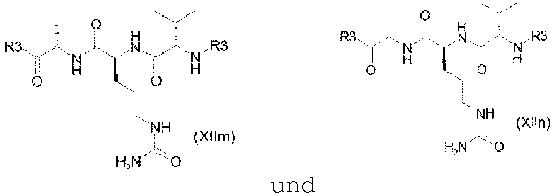 worin
einer der beiden **R3** Substituenten ein Tether zu **Ant,** zu **W** oder zu **L** ist und der andere nicht vorhanden ist;
**W** nicht vorhanden ist oder eine Gruppe, ausgewählt aus den Folgenden: 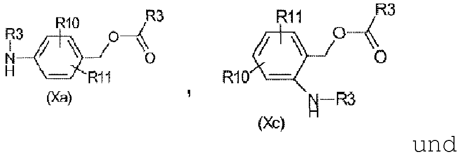 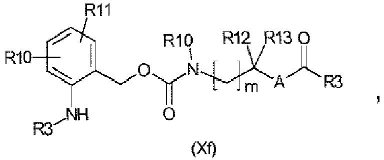 worin
einer der beiden **R3** Substituenten ein Tether zu **L** oder zu **Ant** ist, wenn L nicht vorhanden ist, und der andere nicht vorhanden ist;
**R10** und **R11** Wasserstoff sind;
**m** eine ganze Zahl von 0 bis 3 ist;
**A** N-CH₃ ist; und
**R12** und **R13** Wasserstoff sind;
**L** nicht vorhanden ist oder eine Gruppe, ausgewählt aus den Folgenden: 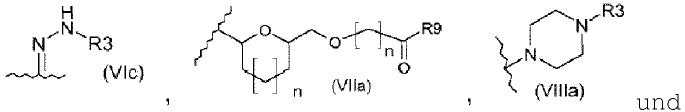 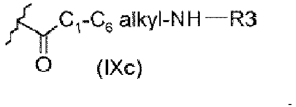 worin
**C₁-C₆** Alkyl eine gerade oder verzweigte Kette ist,
**R3** nicht vorhanden oder Wasserstoff ist und die gewellte Linie den Verbindungspunkt zu **Ant** anzeigt;
**R9** nicht vorhanden ist;
**n** eine ganze Zahl von 0 bis 2 ist;
**Ant** eine Anthracyclingruppe ist, ausgewählt aus den Formeln (IIa), (IIIa), (IVa) und (Va): 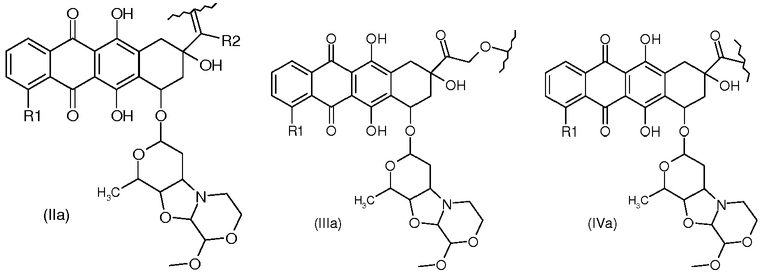 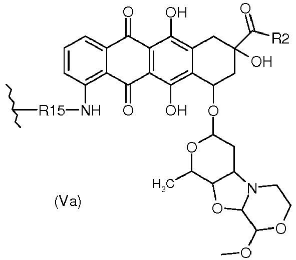
worin die gewellte Linie den Verbindungspunkt anzeigt zur Gruppe **L** oder
zur Gruppe **W** oder
zum Linker **Z** oder
zum Radikal **RM;**
**R1** NH₂ ist;
**R2** Methyl oder Hydroxymethyl ist;
**R15** nicht vorhanden ist;
oder ein pharmazeutisch verträgliches Salz davon.

2. Morpholinylanthracyclinderivat der Formel (I) nach Anspruch 1, wobei die Anthracyclingruppe **(Ant)** die Formel (IIa) aufweist und **L** nicht vorhandenist oder eine Gruppe der Formel (VIc): 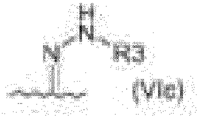 worin:
**R3** nicht vorhanden ist und
die gewellte Linie den Verbindungspunkt zu **Ant** anzeigt;
oder ein pharmazeutisch verträgliches Salz davon.

3. Morpholinylanthracyclinderivat der Formel (I) nach Anspruch 1, wobei die Anthracyclingruppe **(Ant)** die Formel (IIIa) aufweist und **L** nicht vorhanden ist oder eine Gruppe der Formel (VIIa) : 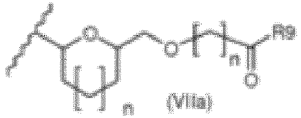 worin:
**R9** nicht vorhanden ist;
**n** eine ganze Zahl von 0 bis 2 ist und
die gewellte Linie den Verbindungspunkt zu **Ant** anzeigt;
oder ein pharmazeutisch verträgliches Salz davon.

4. Morpholinylanthracyclinderivat der Formel (I) nach Anspruch 1, wobei die Anthracyclingruppe **(Ant)** die Formel (IVa) aufweist und **L** nicht vorhanden ist oder eine Gruppe der Formel (VIIIa) : 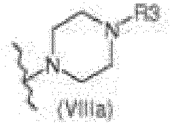 worin:
**R3** nicht vorhanden ist;
die gewellte Linie den Verbindungspunkt zu **Ant** anzeigt;
oder ein pharmazeutisch verträgliches Salz davon.

5. Morpholinylanthracyclinderivat der Formel (I) nach Anspruch 1, wobei die Anthracyclingruppe **(Ant)** die Formel (Va) aufweist und **L** nicht vorhanden ist oder eine Gruppe der Formel (IXc): 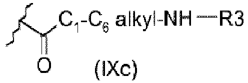 worin:
**C₁-C₆** Alkyl eine gerade oder verzweigte Kette ist;
**R3** nicht vorhanden ist oder Wasserstoff; und
die gewellte Linie den Verbindungspunkt zu **Ant** anzeigt;
oder ein pharmazeutisch verträgliches Salz davon.

6. Morpholinylanthracyclinderivat der Formel (I) nach Anspruch 1 oder ein pharmazeutisch verträgliches Salz davon, das ausgewählt ist aus der Gruppe, bestehend aus den folgenden Verbindungen **(1)-(20), (23)-(27), (75)-(78) :**

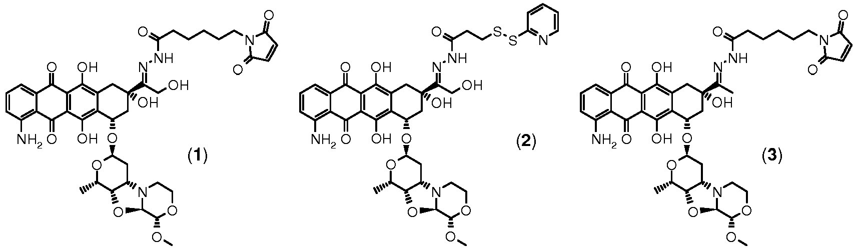

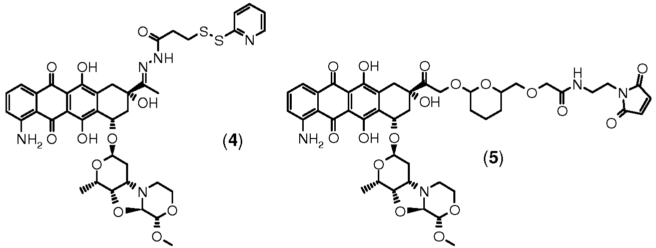

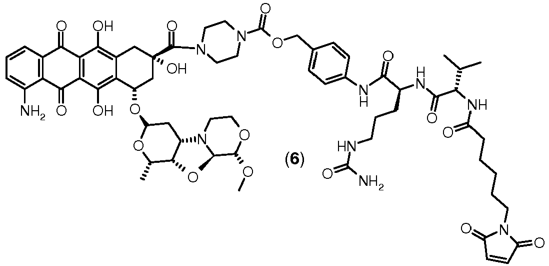

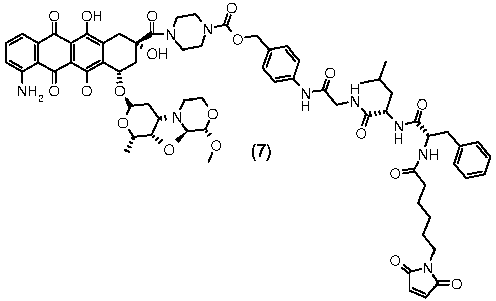

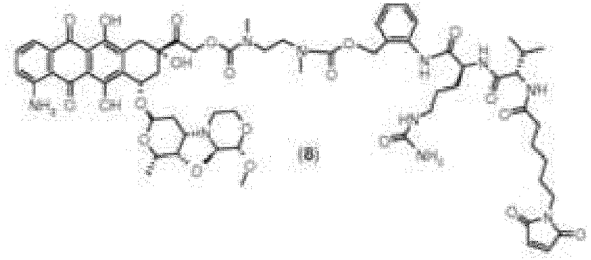

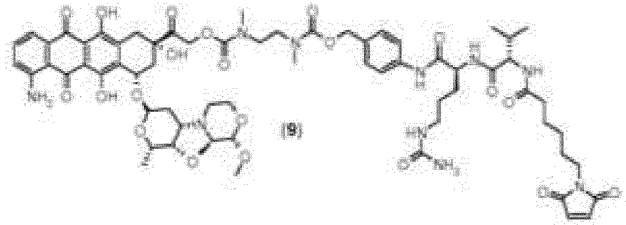

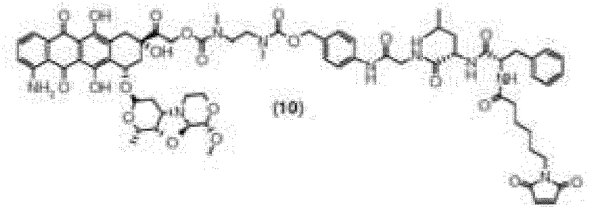

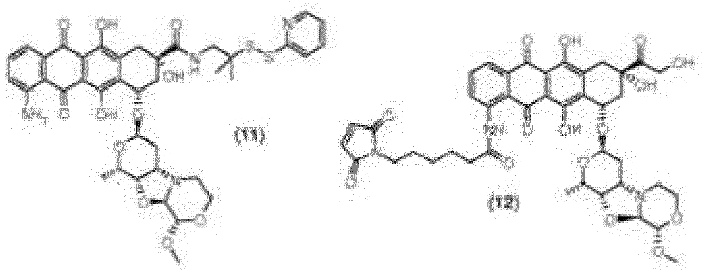

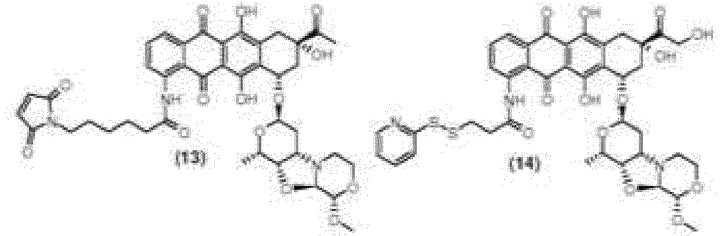

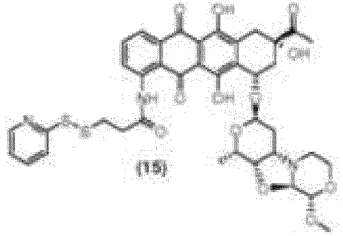

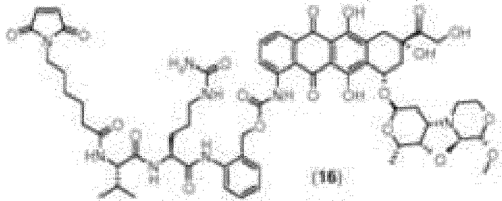

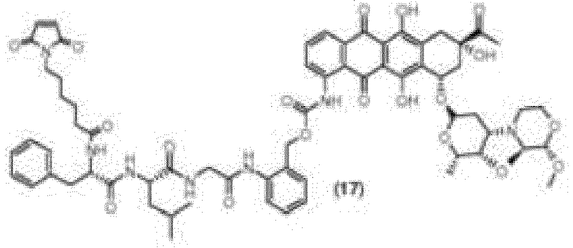

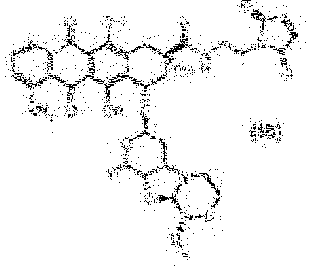

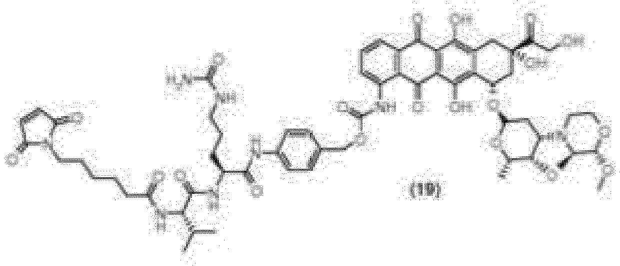

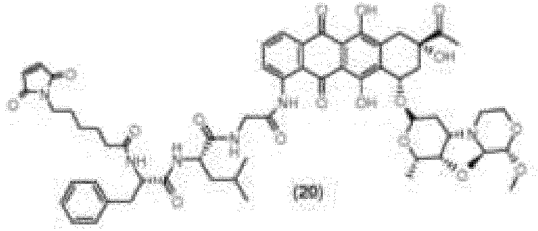

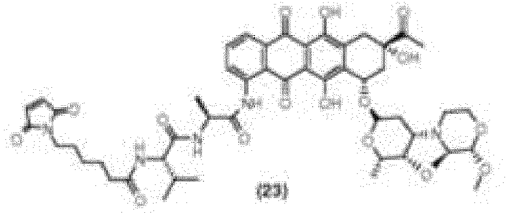

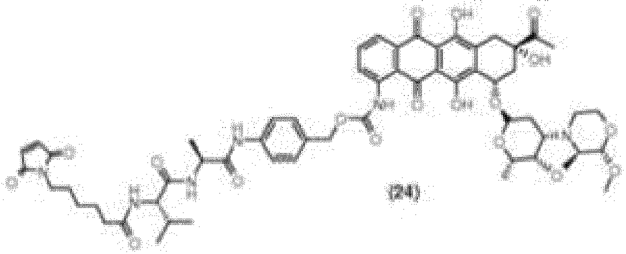

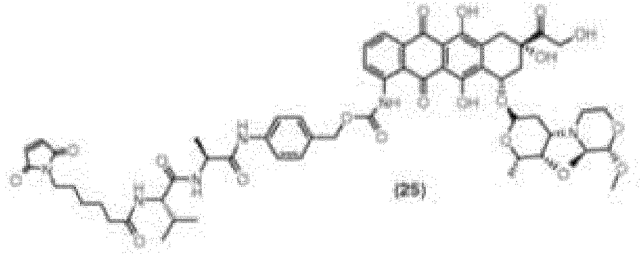

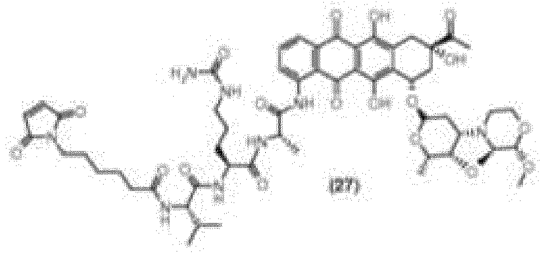

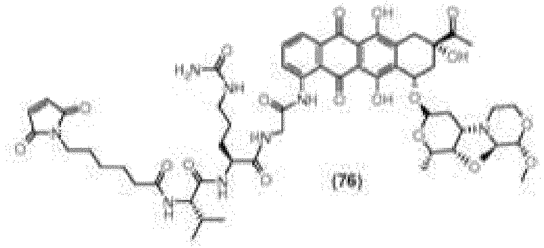

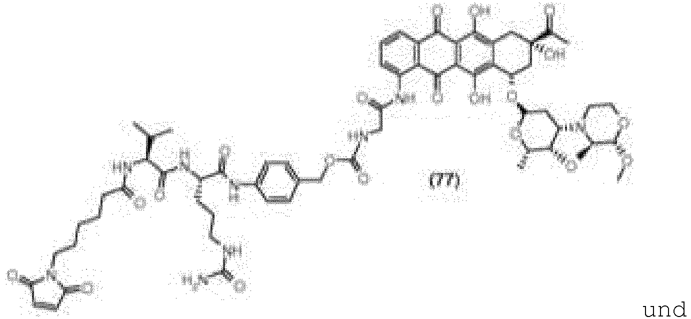

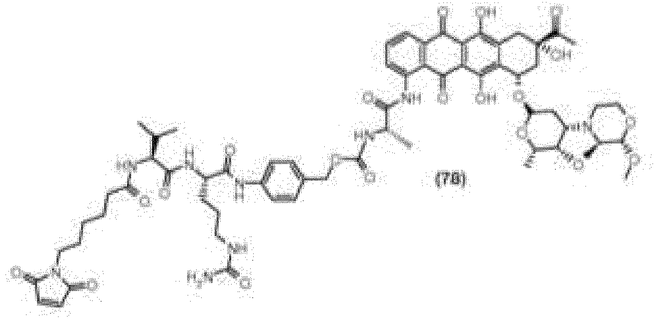

7. Pharmazeutische Zusammensetzung, die eine therapeutisch wirksame Menge eines Morpholinylanthracyclinderivats der Formel (I) umfasst oder eines pharmazeutisch verträglichen Salzes davon, wie in Anspruch 1 definiert, und mindestens einen pharmazeutisch verträglichen Hilfsstoff, Träger oder Verdünnungsmittel.

8. Pharmazeutische Zusammensetzung nach Anspruch 7, die ferner ein oder mehrere Chemotherapeutika umfasst.

9. Produkt, das ein Morpholinylanthracyclinderivat der Formel (I) oder ein pharmazeutisch verträgliches Salz davon umfasst, wie in Anspruch 1 definiert, und ein oder mehrere Chemotherapeutika, als Kombinationspräparat für die gleichzeitige, getrennte oder aufeinanderfolgende Verwendung in der Krebstherapie.

10. Morpholinylanthracyclinderivat der Formel (I) oder ein pharmazeutisch verträgliches Salz davon, wie in Anspruch 1 definiert, zur Verwendung als Arzneimittel.

11. Morpholinylanthracyclinderivat der Formel (I) oder ein pharmazeutisch verträgliches Salz davon, wie in Anspruch 1 definiert, zur Verwendung in einem Verfahren zur Behandlung von Krebs.

12. Morpholinylanthracyclinderivat zur Verwendung nach Anspruch 11, **dadurch gekennzeichnet, dass** der Krebs ausgewählt ist aus der Gruppe, bestehend aus Karzinomen, einschließlich Blasen-, Brust-, Dickdarm-, Nieren-, Leber-, Lungen-, umfassend kleinzelligen Lungenkrebs, Speiseröhren-, Gallenblasen-, Eierstock-, Bauchspeicheldrüsen-, Magen-, Gebärmutterhals-, Schilddrüsen-, Prostata- und Hautkarzinom, einschließlich Plattenepithelkarzinom; hämatopoetischen Tumoren lymphatischer Abstammung, einschließlich Leukämie, akute lymphatische Leukämie, akute lymphoblastische Leukämie, B-Zell-Lymphom, T-Zell-Lymphom, Hodgkin-Lymphom, Non-Hodgkin-Lymphom, Haarzell-Lymphom und Burkitt-Lymphom; hämatopoetischen Tumoren myeloischer Abstammung, einschließlich akuter und chronischen myeloischen Leukämien, myelodysplastischem Syndrom und Promyelozytenleukämie; Tumoren mesenchymalen Ursprungs, einschließlich Fibrosarkom und Rhabdomyosarkom; Tumoren des zentralen und peripheren Nervensystems, einschließlich Astrozytom, Neuroblastom, Gliom und Schwannom; und weiteren Tumoren, einschließlich Melanom, Seminom, Teratokarzinom, Osteosarkom, Xeroderma pigmentosum, Keratoxanthom, follikulärem Thyroidkarzinom, Kaposi-Sarkom und Mesotheliom.

13. Verwendung eines Morpholinylanthracyclinderivats der Formel (I) oder eines pharmazeutisch verträglichen Salzes davon, wie in Anspruch 1 definiert, zur Herstellung von Konjugaten.

## Revendications

1. Dérivé de morpholinyl anthracycline de formule (I)
**Ant-L-W-Z-RM** **(I)**
dans lequel
RM est un radical sélectionné parmi les groupes : 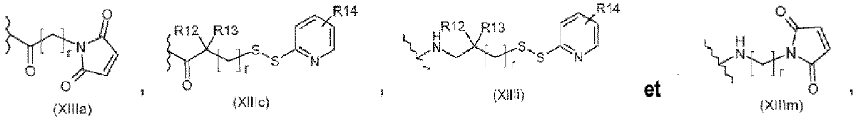 dans lequel
r est un nombre entier de 0 à 7 ;
R12 et R13 sont, chacun indépendamment, un atome d'hydrogène ou un groupe méthyle, et
R14 est un atome d'hydrogène ;
la ligne ondulée indique le point de fixation dans le dérivé de formule (I) ;
Z est nul ou un lieur peptidique, non peptidique, hydride peptidique et non peptidique sélectionné parmi : 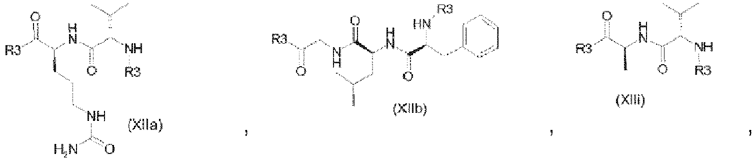 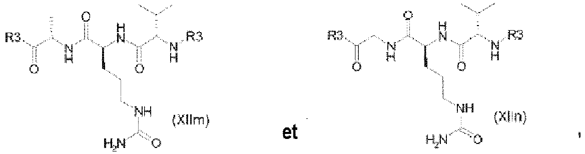 dans lequel un des deux substituants R3 est une liaison avec Ant, avec W, ou avec L, et l'autre est nul ;
W est nul ou un groupe sélectionné parmi : 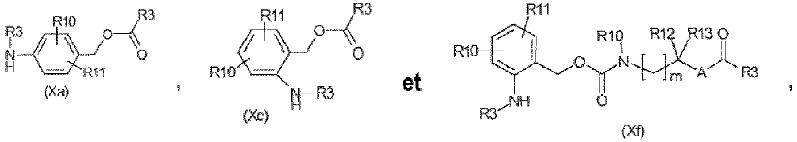 dans lequel
un des deux substituants R3 est une liaison avec L, ou avec Ant lorsque L est nul, et l'autre est nul ;
R10 et R11 sont un atome d'hydrogène ;
m est un nombre entier de 0 à 3 ;
A est N-CH₃ ; et
R12 et R13 sont un atome d'hydrogène ;
L est nul ou une fraction sélectionnée parmi : 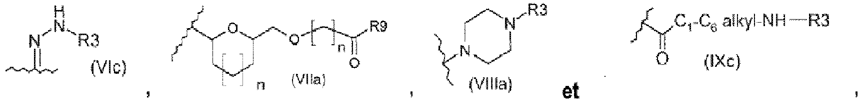 dans lequel
alkyle en C₁ à C₆ est une chaîne linéaire ou ramifiée ;
R3 est nul ou un atome d'hydrogène, et la ligne ondulée indique le point de fixation à Ant ;
R9 est nul ;
n est un nombre entier de 0 à 2 ;
Ant est une fraction anthracycline sélectionnée parmi les formules (IIa), (IIIa), (IVa) et (Va) : 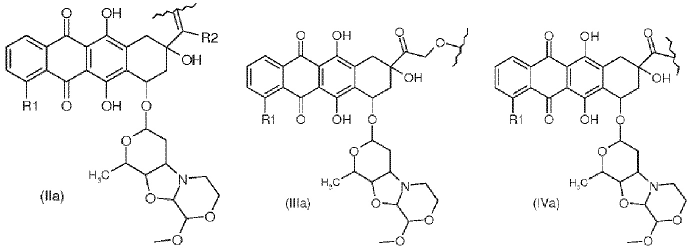 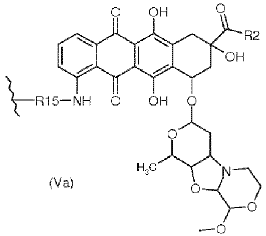
dans lequel la ligne ondulée indique la fixation à la fraction L, ou
au groupe W, ou
au lieur Z, ou
au radical RM ;
R1 est NH₂ ;
R2 est un groupe méthyle ou hydroxyméthyle ;
R15 est nul ;
ou un sel pharmaceutiquement acceptable de celui-ci.

2. Dérivé de morpholinyl anthracycline de formule (I), selon la revendication 1, dans lequel la fraction anthracycline (Ant) est de formule (IIa) et L est nul ou un groupe de formule (VIc) : 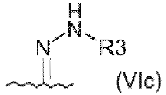 dans lequel :
R3 est nul, et
la ligne ondulée indique le point de fixation à Ant ;
ou un sel pharmaceutiquement acceptable de celui-ci.

3. Dérivé de morpholinyl anthracycline de formule (I), selon la revendication 1, dans lequel la fraction anthracycline (Ant) est de formule (IIIa) et L est nul ou un groupe de formule (VIIa) : 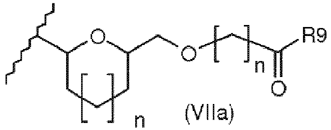 dans lequel :
R9 est nul ;
n est un nombre entier de 0 à 2, et
la ligne ondulée indique le point de fixation à Ant ;
ou un sel pharmaceutiquement acceptable de celui-ci.

4. Dérivé de morpholinyl anthracycline de formule (I), selon la revendication 1, dans lequel la fraction anthracycline (Ant) est de formule (IVa) et L est nul ou un groupe de formule (VIIIa) : 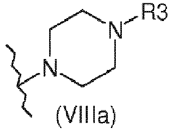 dans lequel :
R3 est nul, et
la ligne ondulée indique le point de fixation à Ant ;
ou un sel pharmaceutiquement acceptable de celui-ci.

5. Dérivé de morpholinyl anthracycline de formule (I), selon la revendication 1, dans lequel la fraction anthracycline (Ant) est de formule (Va) et L est nul ou un groupe de formule (IXc) : 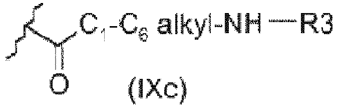 dans lequel :
alkyle en C₁ à C₆ est une chaîne linéaire ou ramifiée,
R3 est nul ou un atome d'hydrogène, et
la ligne ondulée indique le point de fixation à Ant ;
ou un sel pharmaceutiquement acceptable de celui-ci.

6. Dérivé de morpholinyl anthracycline de formule (I), selon la revendication 1, ou un sel pharmaceutiquement acceptable de celui-ci, qui est sélectionné dans le groupe constitué des composés (1)-(20), (23)-(27), (75)-(78) suivants :

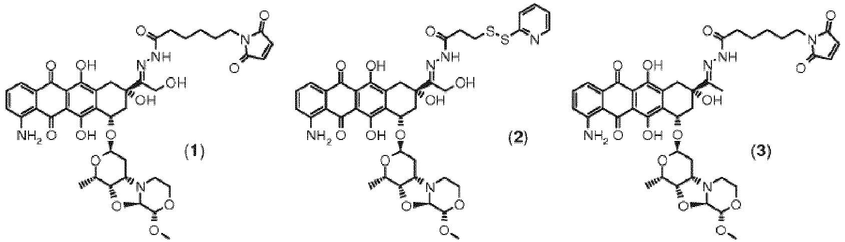

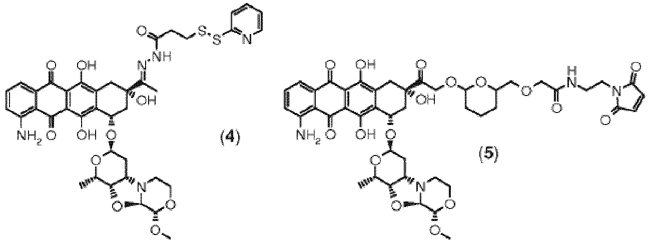

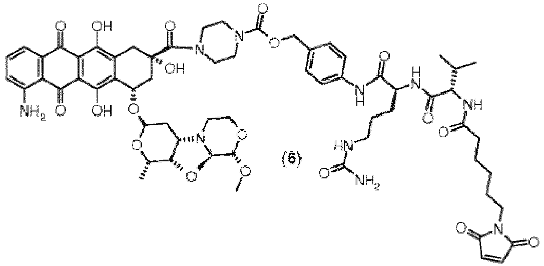

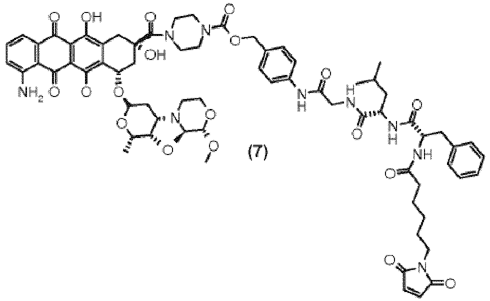

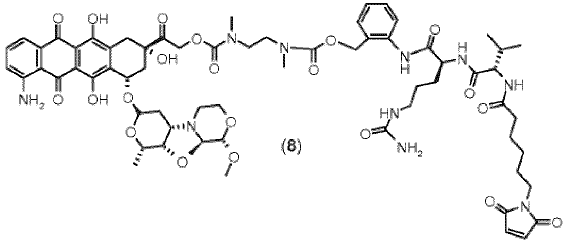

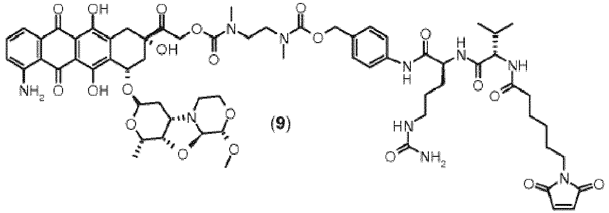

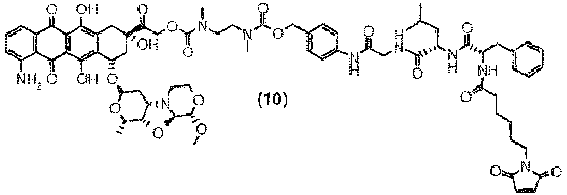

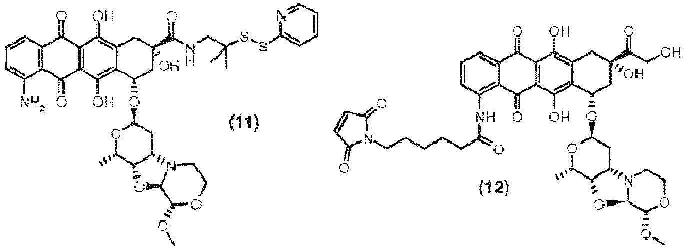

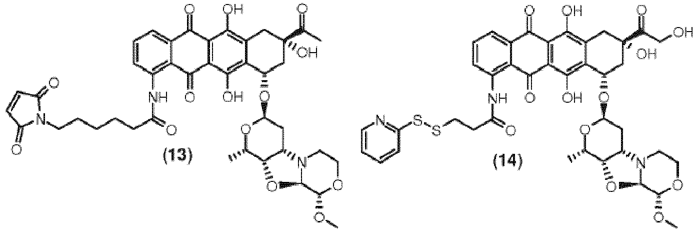

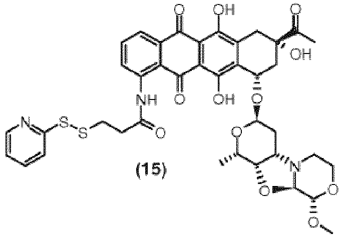

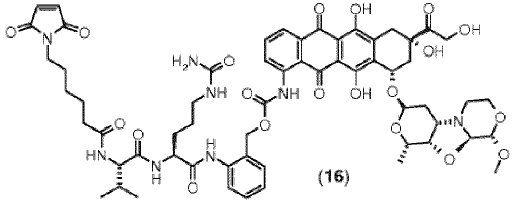

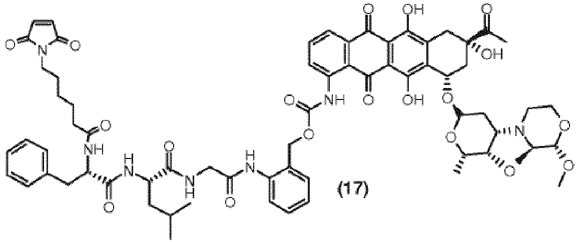

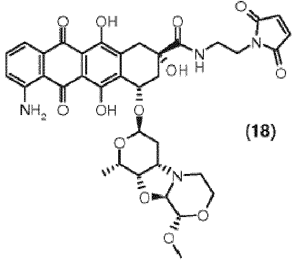

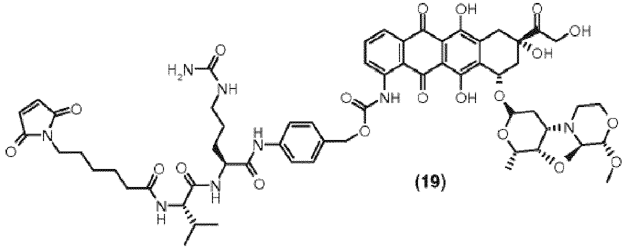

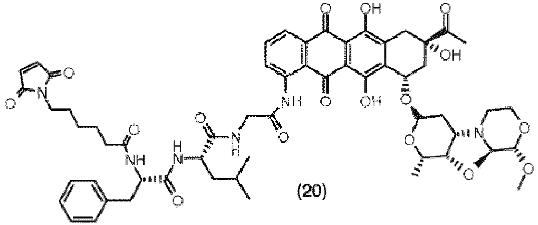

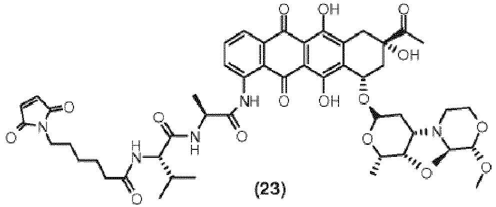

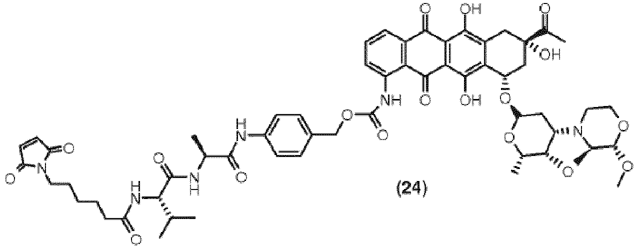

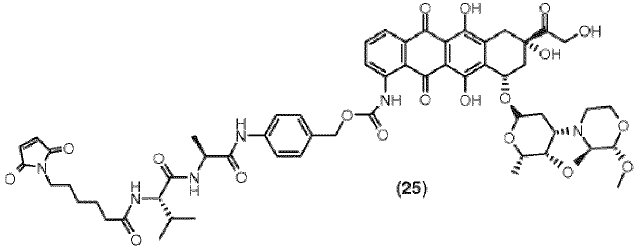

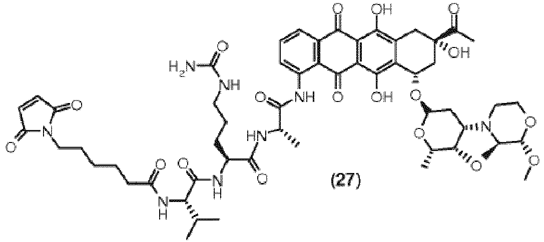

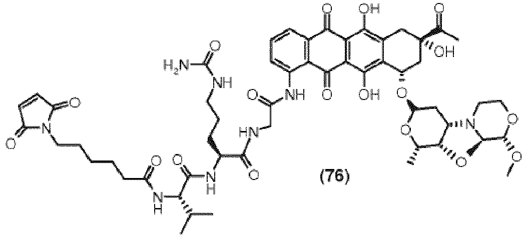

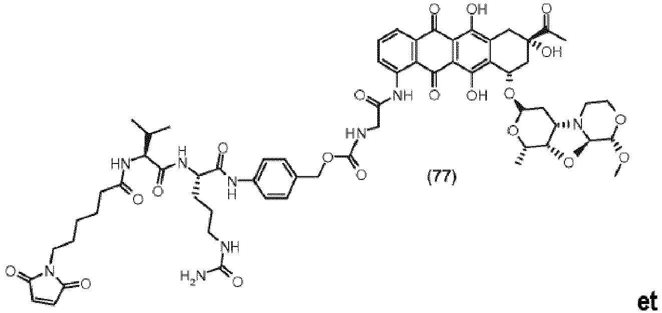

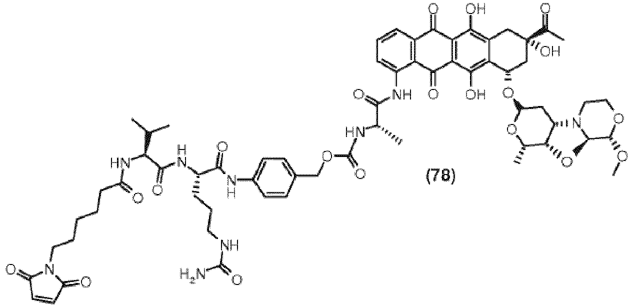

7. Composition pharmaceutique comprenant une quantité thérapeutiquement efficace d'un dérivé de morpholinyl anthracycline de formule (I) ou d'un sel pharmaceutiquement acceptable de celui-ci, selon la revendication 1, et au moins un excipient, un vecteur ou un diluant pharmaceutiquement acceptables.

8. Composition pharmaceutique selon la revendication 7 comprenant en outre un ou plusieurs agents chimiothérapeutiques.

9. Produit comprenant un dérivé de morpholinyl anthracycline de formule (I) ou un sel pharmaceutiquement acceptable de celui-ci, selon la revendication 1, et un ou plusieurs agents chimiothérapeutiques, sous la forme d'une préparation combinée pour une utilisation simultanée, séparée ou séquentielle dans une thérapie anticancéreuse.

10. Dérivé de morpholinyl anthracycline de formule (I) ou un sel pharmaceutiquement acceptable de celui-ci, selon la revendication 1, pour son utilisation en tant que médicament.

11. Dérivé de morpholinyl anthracycline de formule (I) ou un sel pharmaceutiquement acceptable de celui-ci, selon la revendication 1, pour son utilisation dans un procédé de traitement du cancer.

12. Dérivé de morpholinyl anthracycline pour son utilisation selon la revendication 11, **caractérisé en ce que** le cancer est sélectionné dans le groupe constitué des carcinomes, y compris le carcinome de la vessie, du sein, du côlon, du rein, du foie, du poumon, y compris le cancer du poumon à petites cellules, de l'oesophage, de la vésicule biliaire, de l'ovaire, du pancréas, de l'estomac, du col de l'utérus, de la thyroïde, de la prostate et de la peau, y compris le carcinome épidermoïde ; des tumeurs hématopoïétiques de la lignée lymphoïde, y compris la leucémie, la leucémie aiguë lymphoïde, la leucémie aiguë lymphoblastique, le lymphome B, le lymphome T, le lymphome hodgkinien, le lymphome non hodgkinien, le lymphome à tricholeucocytes et le lymphome de Burkitt ; des tumeurs hématopoïétiques de la lignée myéloïde, y compris les leucémies myélogènes aiguës et chroniques, le syndrome myélodysplasique et la leucémie aiguë promyélocytaire ; des tumeurs d'origine mésenchymateuse, y compris le fibrosarcome et le rhabdomyosarcome ; des tumeurs du système nerveux central et périphérique, y compris l'astrocytome, le neuroblastome, le gliome et les schwannomes ; des autres tumeurs, y compris le mélanome, le séminome, le tératocarcinome, l'ostéosarcome, la xérodermie pigmentaire, le kérato-acanthome, le cancer folliculaire de la thyroïde et le sarcome de Kaposi et le mésothéliome.

13. Utilisation d'un dérivé de morpholinyl anthracycline selon la formule (I) ou d'un sel pharmaceutiquement acceptable de celui-ci, selon la revendication 1, dans la préparation de conjugués.
